(19) Europäisches Patentamt
European Patent Office
Office européen des brevets

(11) EP 3 527 225 A1

(12) EUROPEAN PATENT APPLICATION
published in accordance with Art. 153(4) EPC

(43) Date of publication:
**21.08.2019 Bulletin 2019/34**

(21) Application number: **17860929.3**

(22) Date of filing: **11.10.2017**

(51) Int Cl.:
***A61K 39/395*** (2006.01) ***A61K 31/7088*** (2006.01)
***A61K 38/02*** (2006.01) ***A61K 45/00*** (2006.01)
***A61P 27/02*** (2006.01) ***A61P 27/06*** (2006.01)
***A61P 37/06*** (2006.01) ***A61P 43/00*** (2006.01)
***C07K 16/18*** (2006.01) ***C12N 15/09*** (2006.01)

(86) International application number:
**PCT/JP2017/036721**

(87) International publication number:
**WO 2018/070390 (19.04.2018 Gazette 2018/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **12.10.2016 JP 2016200551**

(71) Applicant: **Daiichi Sankyo Company, Limited**
**Tokyo 103-8426 (JP)**

(72) Inventor: **ISUMI, Yoshitaka**
**Tokyo 103-8426 (JP)**

(74) Representative: **Vossius & Partner**
**Patentanwälte Rechtsanwälte mbB**
**Siebertstrasse 3**
**81675 München (DE)**

# (54) COMPOSITION CONTAINING ANTI-ROBO4 ANTIBODY AND OTHER AGENTS

(57) Provided are a pharmaceutical composition and the like for use in treating or preventing angiogenic diseases. A combination of a monoclonal antibody or antigen-binding fragment thereof having features (i) to (v) below and a VEGF antagonist; or a pharmaceutical composition comprising the antibody or antigen-binding fragment thereof and a VEGF antagonist:
(i) binding to ROBO4 protein with a Kd value of $1 \times 10^{-8}$ M or less;
(ii) specifically binding to human ROBO4 protein shown in SEQ ID NO: 2 in the sequence listing;
(iii) suppressing or inhibiting migration of vascular endothelial cells *in vitro* in the absence of a crosslinking antibody;
(iv) suppressing or inhibiting angiogenesis *in vivo;* or
(v) being a chimeric antibody or a humanized antibody.

EP 3 527 225 A1

SEQUENCE LISTING

<110> DAIICHI SANKYO COMPANY, LIMITED

<120> COMBINATION OF ANTI-ROBO4 ANTIBODY AND OTHER AGENT

<130> FP1720

<150> JP2016-200551
<151> 2016-10-12

<160> 22

<170> PatentIn version 3.5

<210> 1
<211> 3024
<212> DNA
<213> Homo sapiens

<400> 1
atgggctctg gaggagacag cctcctgggg ggcaggggtt ccctgcctct gctgctcctg 60

ctcatcatgg gaggcatggc tcaggactcc ccgccccaga tcctagtcca cccccaggac 120

cagctgttcc agggccctgg ccctgccagg atgagctgcc aagcctcagg ccagccacct 180

cccaccatcc gctggttgct gaatgggcag cccctgagca tggtgccccc agacccacac 240

cacctcctgc ctgatgggac ccttctgctg ctacagcccc ctgcccgggg acatgcccac 300

gatggccagg ccctgtccac agacctgggt gtctacacat gtgaggccag caaccggctt 360

ggcacggcag tcagcagagg cgctcggctg tctgtggctg tcctccggga ggatttccag 420

atccagcctc gggacatggt ggctgtggtg ggtgagcagt ttactctgga atgtgggccg 480

ccctggggcc acccagagcc cacagtctca tggtggaaag atgggaaacc cctggccctc 540

cagcccggaa ggcacacagt gtccgggggg tccctgctga tggcaagagc agagaagagt 600

gacgaaggga cctacatgtg tgtggccacc aacagcgcag gacataggga gagccgcgca 660

gcccgggttt ccatccagga gccccaggac tacacggagc ctgtggagct tctggctgtg 720

cgaattcagc tggaaaatgt gacactgctg aacccggatc ctgcagaggg ccccaagcct 780

agaccggcgg tgtggctcag ctggaaggtc agtggccctg ctgcgcctgc ccaatcttac 840

acggccttgt tcaggaccca gactgccccg ggaggccagg gagctccgtg ggcagaggag 900

ctgctggccg gctggcagag cgcagagctt ggaggcctcc actggggcca agactacgag 960

ttcaaagtga gaccatcctc tggccgggct cgaggccctg acagcaacgt gctgctcctg 1020

aggctgccgg aaaaagtgcc cagtgcccca cctcaggaag tgactctaaa gcctggcaat 1080

ggcactgtct ttgtgagctg ggtcccacca cctgctgaaa accacaatgg catcatccgt 1140

ggctaccagg tctggagcct gggcaacaca tcactgccac cagccaactg gactgtagtt 1200

ggtgagcaga cccagctgga aatcgccacc catatgccag gctcctactg cgtgcaagtg 1260

```
gctgcagtca ctggtgctgg agctggggag cccagtagac ctgtctgcct ccttttagag     1320
caggccatgg agcgagccac ccaagaaccc agtgagcatg gtccctggac cctggagcag     1380
ctgagggcta ccttgaagcg gcctgaggtc attgccacct gcggtgttgc actctggctg     1440
ctgcttctgg gcaccgccgt gtgtatccac cgccggcgcc gagctagggt gcacctgggc     1500
ccaggtctgt acagatatac cagtgaggat gccatcctaa aacacaggat ggatcacagt     1560
gactcccagt ggttggcaga cacttggcgt tccacctctg gctctcggga cctgagcagc     1620
agcagcagcc tcagcagtcg gctgggggcg gatgcccggg acccactaga ctgtcgtcgc     1680
tccttgctct cctgggactc ccgaagcccc ggcgtgcccc tgcttccaga caccagcact     1740
ttttatggct ccctcatcgc tgagctgccc tccagtaccc cagccaggcc aagtccccag     1800
gtcccagctg tcaggcgcct cccaccccag ctggcccagc tctccagccc ctgttccagc     1860
tcagacagcc tctgcagccg caggggactc tcttctcccc gcttgtctct ggcccctgca     1920
gaggcttgga aggccaaaaa gaagcaggag ctgcagcatg ccaacagttc ccccactgctc    1980
cggggcagcc actccttgga gctccgggcc tgtgagttag gaaatagagg ttccaagaac     2040
ctttcccaaa gcccaggagc tgtgccccaa gctctggttg cctggcgggc cctgggaccg     2100
aaactcctca gctcctcaaa tgagctggtt actcgtcatc tccctccagc acccctcttt     2160
cctcatgaaa ctcccccaac tcagagtcaa cagacccagc ctccggtggc accacaggct     2220
ccctcctcca tcctgctgcc agcagccccc atccccatcc ttagcccctg cagtcccccct    2280
agcccccagg cctcttccct ctctggcccc agcccagctt ccagtcgcct gtccagctcc     2340
tcactgtcat ccctgggggga ggatcaagac agcgtgctga cccctgagga ggtagccctg    2400
tgcttggaac tcagtgaggg tgaggagact cccaggaaca gcgtctctcc catgccaagg     2460
gctccttcac cccccaccac ctatgggtac atcagcgtcc caacagcctc agagttcacg     2520
gacatgggca ggactggagg aggggtgggg cccaaggggg gagtcttgct gtgcccacct     2580
cggccctgcc tcacccccac ccccagcgag ggctccttag ccaatggttg gggctcagcc     2640
tctgaggaca atgccgccag cgccagagcc agccttgtca gctcctccga tggctccttc     2700
ctcgctgatg ctcactttgc ccgggccctg gcagtggctg tggatagctt tggtttcggt     2760
ctagagccca gggaggcaga ctgcgtcttc atagatgcct catcacctcc ctccccacgg     2820
gatgagatct tcctgacccc caacctctcc ctgcccctgt gggagtggag gccagactgg     2880
ttggaagaca tggaggtcag ccacacccag cggctgggaa gggggatgcc tccctggccc     2940
cctgactctc agatctcttc ccagagaagt cagctccact gtcgtatgcc caaggctggt     3000
gcttctcctg tagattactc ctga                                            3024
```

<210> 2
<211> 1007

<212> PRT
<213> Homo sapiens

<400> 2

```
Met Gly Ser Gly Gly Asp Ser Leu Leu Gly Gly Arg Gly Ser Leu Pro
1               5                   10                  15

Leu Leu Leu Leu Leu Ile Met Gly Gly Met Ala Gln Asp Ser Pro Pro
            20                  25                  30

Gln Ile Leu Val His Pro Gln Asp Gln Leu Phe Gln Gly Pro Gly Pro
        35                  40                  45

Ala Arg Met Ser Cys Gln Ala Ser Gly Gln Pro Pro Pro Thr Ile Arg
    50                  55                  60

Trp Leu Leu Asn Gly Gln Pro Leu Ser Met Val Pro Pro Asp Pro His
65                  70                  75                  80

His Leu Leu Pro Asp Gly Thr Leu Leu Leu Leu Gln Pro Pro Ala Arg
                85                  90                  95

Gly His Ala His Asp Gly Gln Ala Leu Ser Thr Asp Leu Gly Val Tyr
            100                 105                 110

Thr Cys Glu Ala Ser Asn Arg Leu Gly Thr Ala Val Ser Arg Gly Ala
        115                 120                 125

Arg Leu Ser Val Ala Val Leu Arg Glu Asp Phe Gln Ile Gln Pro Arg
    130                 135                 140

Asp Met Val Ala Val Val Gly Glu Gln Phe Thr Leu Glu Cys Gly Pro
145                 150                 155                 160

Pro Trp Gly His Pro Glu Pro Thr Val Ser Trp Trp Lys Asp Gly Lys
                165                 170                 175

Pro Leu Ala Leu Gln Pro Gly Arg His Thr Val Ser Gly Gly Ser Leu
            180                 185                 190

Leu Met Ala Arg Ala Glu Lys Ser Asp Glu Gly Thr Tyr Met Cys Val
        195                 200                 205

Ala Thr Asn Ser Ala Gly His Arg Glu Ser Arg Ala Ala Arg Val Ser
    210                 215                 220

Ile Gln Glu Pro Gln Asp Tyr Thr Glu Pro Val Glu Leu Leu Ala Val
225                 230                 235                 240
```

```
Arg Ile Gln Leu Glu Asn Val Thr Leu Leu Asn Pro Asp Pro Ala Glu
                245                 250                 255

Gly Pro Lys Pro Arg Pro Ala Val Trp Leu Ser Trp Lys Val Ser Gly
            260                 265                 270

Pro Ala Ala Pro Ala Gln Ser Tyr Thr Ala Leu Phe Arg Thr Gln Thr
        275                 280                 285

Ala Pro Gly Gly Gln Gly Ala Pro Trp Ala Glu Glu Leu Leu Ala Gly
    290                 295                 300

Trp Gln Ser Ala Glu Leu Gly Gly Leu His Trp Gly Gln Asp Tyr Glu
305                 310                 315                 320

Phe Lys Val Arg Pro Ser Ser Gly Arg Ala Arg Gly Pro Asp Ser Asn
                325                 330                 335

Val Leu Leu Leu Arg Leu Pro Glu Lys Val Pro Ser Ala Pro Pro Gln
            340                 345                 350

Glu Val Thr Leu Lys Pro Gly Asn Gly Thr Val Phe Val Ser Trp Val
        355                 360                 365

Pro Pro Pro Ala Glu Asn His Asn Gly Ile Ile Arg Gly Tyr Gln Val
    370                 375                 380

Trp Ser Leu Gly Asn Thr Ser Leu Pro Pro Ala Asn Trp Thr Val Val
385                 390                 395                 400

Gly Glu Gln Thr Gln Leu Glu Ile Ala Thr His Met Pro Gly Ser Tyr
                405                 410                 415

Cys Val Gln Val Ala Ala Val Thr Gly Ala Gly Ala Gly Glu Pro Ser
            420                 425                 430

Arg Pro Val Cys Leu Leu Leu Glu Gln Ala Met Glu Arg Ala Thr Gln
        435                 440                 445

Glu Pro Ser Glu His Gly Pro Trp Thr Leu Glu Gln Leu Arg Ala Thr
    450                 455                 460

Leu Lys Arg Pro Glu Val Ile Ala Thr Cys Gly Val Ala Leu Trp Leu
465                 470                 475                 480

Leu Leu Leu Gly Thr Ala Val Cys Ile His Arg Arg Arg Arg Ala Arg
```

485 490 495

Val His Leu Gly Pro Gly Leu Tyr Arg Tyr Thr Ser Glu Asp Ala Ile
500 505 510

Leu Lys His Arg Met Asp His Ser Asp Ser Gln Trp Leu Ala Asp Thr
515 520 525

Trp Arg Ser Thr Ser Gly Ser Arg Asp Leu Ser Ser Ser Ser Ser Leu
530 535 540

Ser Ser Arg Leu Gly Ala Asp Ala Arg Asp Pro Leu Asp Cys Arg Arg
545 550 555 560

Ser Leu Leu Ser Trp Asp Ser Arg Ser Pro Gly Val Pro Leu Leu Pro
565 570 575

Asp Thr Ser Thr Phe Tyr Gly Ser Leu Ile Ala Glu Leu Pro Ser Ser
580 585 590

Thr Pro Ala Arg Pro Ser Pro Gln Val Pro Ala Val Arg Arg Leu Pro
595 600 605

Pro Gln Leu Ala Gln Leu Ser Ser Pro Cys Ser Ser Ser Asp Ser Leu
610 615 620

Cys Ser Arg Arg Gly Leu Ser Ser Pro Arg Leu Ser Leu Ala Pro Ala
625 630 635 640

Glu Ala Trp Lys Ala Lys Lys Lys Gln Glu Leu Gln His Ala Asn Ser
645 650 655

Ser Pro Leu Leu Arg Gly Ser His Ser Leu Glu Leu Arg Ala Cys Glu
660 665 670

Leu Gly Asn Arg Gly Ser Lys Asn Leu Ser Gln Ser Pro Gly Ala Val
675 680 685

Pro Gln Ala Leu Val Ala Trp Arg Ala Leu Gly Pro Lys Leu Leu Ser
690 695 700

Ser Ser Asn Glu Leu Val Thr Arg His Leu Pro Pro Ala Pro Leu Phe
705 710 715 720

Pro His Glu Thr Pro Pro Thr Gln Ser Gln Gln Thr Gln Pro Pro Val
725 730 735

```
Ala Pro Gln Ala Pro Ser Ser Ile Leu Leu Pro Ala Ala Pro Ile Pro
            740                 745                 750

Ile Leu Ser Pro Cys Ser Pro Pro Ser Pro Gln Ala Ser Ser Leu Ser
        755                 760                 765

Gly Pro Ser Pro Ala Ser Ser Arg Leu Ser Ser Ser Ser Leu Ser Ser
    770                 775                 780

Leu Gly Glu Asp Gln Asp Ser Val Leu Thr Pro Glu Glu Val Ala Leu
785                 790                 795                 800

Cys Leu Glu Leu Ser Glu Gly Glu Glu Thr Pro Arg Asn Ser Val Ser
                805                 810                 815

Pro Met Pro Arg Ala Pro Ser Pro Pro Thr Thr Tyr Gly Tyr Ile Ser
            820                 825                 830

Val Pro Thr Ala Ser Glu Phe Thr Asp Met Gly Arg Thr Gly Gly Gly
        835                 840                 845

Val Gly Pro Lys Gly Gly Val Leu Leu Cys Pro Pro Arg Pro Cys Leu
    850                 855                 860

Thr Pro Thr Pro Ser Glu Gly Ser Leu Ala Asn Gly Trp Gly Ser Ala
865                 870                 875                 880

Ser Glu Asp Asn Ala Ala Ser Ala Arg Ala Ser Leu Val Ser Ser Ser
                885                 890                 895

Asp Gly Ser Phe Leu Ala Asp Ala His Phe Ala Arg Ala Leu Ala Val
            900                 905                 910

Ala Val Asp Ser Phe Gly Phe Gly Leu Glu Pro Arg Glu Ala Asp Cys
        915                 920                 925

Val Phe Ile Asp Ala Ser Ser Pro Pro Ser Pro Arg Asp Glu Ile Phe
    930                 935                 940

Leu Thr Pro Asn Leu Ser Leu Pro Leu Trp Glu Trp Arg Pro Asp Trp
945                 950                 955                 960

Leu Glu Asp Met Glu Val Ser His Thr Gln Arg Leu Gly Arg Gly Met
                965                 970                 975

Pro Pro Trp Pro Pro Asp Ser Gln Ile Ser Ser Gln Arg Ser Gln Leu
            980                 985                 990
```

His Cys Arg Met Pro Lys Ala Gly Ala Ser Pro Val Asp Tyr Ser
995 1000 1005

<210> 3
<211> 5
<212> PRT
<213> Mus musculus

<400> 3

Thr Tyr Ala Met Ser
1 5

<210> 4
<211> 17
<212> PRT
<213> Mus musculus

<400> 4

Thr Ile Ser Asn Gly Gly Thr Tyr Thr Tyr Tyr Pro Asp Ser Val Lys
1 5 10 15

Gly

<210> 5
<211> 17
<212> PRT
<213> Artificial Sequence

<220>
<223> CDRH2-2

<400> 5

Thr Ile Ser Gln Gly Gly Thr Tyr Thr Tyr Tyr Pro Asp Ser Val Lys
1 5 10 15

Gly

<210> 6
<211> 9
<212> PRT
<213> Mus musculus

<400> 6

Leu Ile Tyr Tyr Asp Tyr Leu Asp Tyr
1 5

<210> 7
<211> 16
<212> PRT

<213>  Mus musculus

<400>  7

Arg Ser Ser Gln Ser Leu Glu Asn Ser Asn Gly Asn Thr Tyr Leu Asn
1               5                   10                  15

<210>  8
<211>  16
<212>  PRT
<213>  Artificial Sequence

<220>
<223>  CDRL1-2

<400>  8

Arg Ser Ser Gln Ser Leu Glu Asn Glu Asn Lys Asn Leu Tyr Leu Asn
1               5                   10                  15

<210>  9
<211>  7
<212>  PRT
<213>  Mus musculus

<400>  9

Arg Val Ser Asn Arg Phe Ser
1               5

<210>  10
<211>  9
<212>  PRT
<213>  Mus musculus

<400>  10

Leu Gln Val Thr His Val Pro Trp Thr
1               5

<210>  11
<211>  463
<212>  PRT
<213>  Artificial Sequence

<220>
<223>  hMab1-H1 aa

<400>  11

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe

35 40 45

Ser Thr Tyr Ala Met Ser Trp Val Arg Gln Ala Pro Gly Lys Gly Leu
50 55 60

Glu Trp Val Ala Thr Ile Ser Asn Gly Gly Thr Tyr Thr Tyr Tyr Pro
65 70 75 80

Asp Ser Val Lys Gly Arg Phe Thr Ile Ser Arg Asp Asn Ala Lys Asn
85 90 95

Thr Leu Tyr Leu Gln Met Asn Ser Leu Arg Ala Glu Asp Thr Ala Val
100 105 110

Tyr Tyr Cys Ala Arg Leu Ile Tyr Tyr Asp Tyr Leu Asp Tyr Trp Gly
115 120 125

Gln Gly Thr Leu Val Thr Val Ser Ser Ala Ser Thr Lys Gly Pro Ser
130 135 140

Val Phe Pro Leu Ala Pro Cys Ser Arg Ser Thr Ser Glu Ser Thr Ala
145 150 155 160

Ala Leu Gly Cys Leu Val Lys Asp Tyr Phe Pro Glu Pro Val Thr Val
165 170 175

Ser Trp Asn Ser Gly Ala Leu Thr Ser Gly Val His Thr Phe Pro Ala
180 185 190

Val Leu Gln Ser Ser Gly Leu Tyr Ser Leu Ser Ser Val Val Thr Val
195 200 205

Pro Ser Ser Asn Phe Gly Thr Gln Thr Tyr Thr Cys Asn Val Asp His
210 215 220

Lys Pro Ser Asn Thr Lys Val Asp Lys Thr Val Glu Arg Lys Cys Cys
225 230 235 240

Val Glu Cys Pro Pro Cys Pro Ala Pro Pro Val Ala Gly Pro Ser Val
245 250 255

Phe Leu Phe Pro Pro Lys Pro Lys Asp Thr Leu Met Ile Ser Arg Thr
260 265 270

Pro Glu Val Thr Cys Val Val Val Asp Val Ser His Glu Asp Pro Glu
275 280 285

```
Val Gln Phe Asn Trp Tyr Val Asp Gly Val Glu Val His Asn Ala Lys
    290                 295                 300

Thr Lys Pro Arg Glu Glu Gln Phe Asn Ser Thr Phe Arg Val Val Ser
305                 310                 315                 320

Val Leu Thr Val Val His Gln Asp Trp Leu Asn Gly Lys Glu Tyr Lys
                325                 330                 335

Cys Lys Val Ser Asn Lys Gly Leu Pro Ala Pro Ile Glu Lys Thr Ile
            340                 345                 350

Ser Lys Thr Lys Gly Gln Pro Arg Glu Pro Gln Val Tyr Thr Leu Pro
        355                 360                 365

Pro Ser Arg Glu Glu Met Thr Lys Asn Gln Val Ser Leu Thr Cys Leu
    370                 375                 380

Val Lys Gly Phe Tyr Pro Ser Asp Ile Ala Val Glu Trp Glu Ser Asn
385                 390                 395                 400

Gly Gln Pro Glu Asn Asn Tyr Lys Thr Thr Pro Pro Met Leu Asp Ser
                405                 410                 415

Asp Gly Ser Phe Phe Leu Tyr Ser Lys Leu Thr Val Asp Lys Ser Arg
            420                 425                 430

Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys
    450                 455                 460


<210>  12
<211>  463
<212>  PRT
<213>  Artificial Sequence

<220>
<223>  hMab1-H2 aa

<400>  12

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe
```

```
        35                  40                  45

Ser Thr Tyr Ala Met Ser Trp Val Arg Gln Ala Pro Gly Lys Gly Leu
    50                  55                  60

Glu Trp Val Ala Thr Ile Ser Gln Gly Gly Thr Tyr Thr Tyr Tyr Pro
65                  70                  75                  80

Asp Ser Val Lys Gly Arg Phe Thr Ile Ser Arg Asp Asn Ala Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Leu Ile Tyr Tyr Asp Tyr Leu Asp Tyr Trp Gly
        115                 120                 125

Gln Gly Thr Leu Val Thr Val Ser Ser Ala Ser Thr Lys Gly Pro Ser
    130                 135                 140

Val Phe Pro Leu Ala Pro Cys Ser Arg Ser Thr Ser Glu Ser Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Asn Phe Gly Thr Gln Thr Tyr Thr Cys Asn Val Asp His
    210                 215                 220

Lys Pro Ser Asn Thr Lys Val Asp Lys Thr Val Glu Arg Lys Cys Cys
225                 230                 235                 240

Val Glu Cys Pro Pro Cys Pro Ala Pro Pro Val Ala Gly Pro Ser Val
                245                 250                 255

Phe Leu Phe Pro Pro Lys Pro Lys Asp Thr Leu Met Ile Ser Arg Thr
            260                 265                 270

Pro Glu Val Thr Cys Val Val Val Asp Val Ser His Glu Asp Pro Glu
        275                 280                 285
```

Val Gln Phe Asn Trp Tyr Val Asp Gly Val Glu Val His Asn Ala Lys
    290                 295                 300

Thr Lys Pro Arg Glu Glu Gln Phe Asn Ser Thr Phe Arg Val Val Ser
305                 310                 315                 320

Val Leu Thr Val Val His Gln Asp Trp Leu Asn Gly Lys Glu Tyr Lys
                325                 330                 335

Cys Lys Val Ser Asn Lys Gly Leu Pro Ala Pro Ile Glu Lys Thr Ile
            340                 345                 350

Ser Lys Thr Lys Gly Gln Pro Arg Glu Pro Gln Val Tyr Thr Leu Pro
        355                 360                 365

Pro Ser Arg Glu Glu Met Thr Lys Asn Gln Val Ser Leu Thr Cys Leu
    370                 375                 380

Val Lys Gly Phe Tyr Pro Ser Asp Ile Ala Val Glu Trp Glu Ser Asn
385                 390                 395                 400

Gly Gln Pro Glu Asn Asn Tyr Lys Thr Thr Pro Pro Met Leu Asp Ser
                405                 410                 415

Asp Gly Ser Phe Phe Leu Tyr Ser Lys Leu Thr Val Asp Lys Ser Arg
            420                 425                 430

Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys
    450                 455                 460

<210> 13
<211> 463
<212> PRT
<213> Artificial Sequence

<220>
<223> hMAb1-H3 aa

<400> 13

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe

```
        35                  40                  45

Ser Thr Tyr Ala Met Ser Trp Val Arg Gln Ala Pro Gly Lys Arg Leu
    50                  55                  60

Glu Trp Val Ala Thr Ile Ser Asn Gly Gly Thr Tyr Thr Tyr Tyr Pro
65                  70                  75                  80

Asp Ser Val Lys Gly Arg Phe Thr Ile Ser Arg Asp Asn Ala Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Leu Ile Tyr Tyr Asp Tyr Leu Asp Tyr Trp Gly
        115                 120                 125

Gln Gly Thr Leu Val Thr Val Ser Ser Ala Ser Thr Lys Gly Pro Ser
    130                 135                 140

Val Phe Pro Leu Ala Pro Cys Ser Arg Ser Thr Ser Glu Ser Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Asn Phe Gly Thr Gln Thr Tyr Thr Cys Asn Val Asp His
    210                 215                 220

Lys Pro Ser Asn Thr Lys Val Asp Lys Thr Val Glu Arg Lys Cys Cys
225                 230                 235                 240

Val Glu Cys Pro Pro Cys Pro Ala Pro Pro Val Ala Gly Pro Ser Val
                245                 250                 255

Phe Leu Phe Pro Pro Lys Pro Lys Asp Thr Leu Met Ile Ser Arg Thr
            260                 265                 270

Pro Glu Val Thr Cys Val Val Val Asp Val Ser His Glu Asp Pro Glu
        275                 280                 285
```

Val Gln Phe Asn Trp Tyr Val Asp Gly Val Glu Val His Asn Ala Lys
    290                 295                 300

Thr Lys Pro Arg Glu Glu Gln Phe Asn Ser Thr Phe Arg Val Val Ser
305                 310                 315                 320

Val Leu Thr Val Val His Gln Asp Trp Leu Asn Gly Lys Glu Tyr Lys
                325                 330                 335

Cys Lys Val Ser Asn Lys Gly Leu Pro Ala Pro Ile Glu Lys Thr Ile
            340                 345                 350

Ser Lys Thr Lys Gly Gln Pro Arg Glu Pro Gln Val Tyr Thr Leu Pro
        355                 360                 365

Pro Ser Arg Glu Glu Met Thr Lys Asn Gln Val Ser Leu Thr Cys Leu
    370                 375                 380

Val Lys Gly Phe Tyr Pro Ser Asp Ile Ala Val Glu Trp Glu Ser Asn
385                 390                 395                 400

Gly Gln Pro Glu Asn Asn Tyr Lys Thr Thr Pro Pro Met Leu Asp Ser
                405                 410                 415

Asp Gly Ser Phe Phe Leu Tyr Ser Lys Leu Thr Val Asp Lys Ser Arg
            420                 425                 430

Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys
    450                 455                 460

<210> 14
<211> 463
<212> PRT
<213> Artificial Sequence

<220>
<223> hMAb1-H4 aa

<400> 14

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe

```
        35                  40                  45

Ser Thr Tyr Ala Met Ser Trp Val Arg Gln Ala Pro Gly Lys Arg Leu
    50                  55                  60

Glu Trp Val Ala Thr Ile Ser Gln Gly Gly Thr Tyr Thr Tyr Tyr Pro
65                  70                  75                  80

Asp Ser Val Lys Gly Arg Phe Thr Ile Ser Arg Asp Asn Ala Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Leu Ile Tyr Tyr Asp Tyr Leu Asp Tyr Trp Gly
        115                 120                 125

Gln Gly Thr Leu Val Thr Val Ser Ser Ala Ser Thr Lys Gly Pro Ser
    130                 135                 140

Val Phe Pro Leu Ala Pro Cys Ser Arg Ser Thr Ser Glu Ser Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Asn Phe Gly Thr Gln Thr Tyr Thr Cys Asn Val Asp His
    210                 215                 220

Lys Pro Ser Asn Thr Lys Val Asp Lys Thr Val Glu Arg Lys Cys Cys
225                 230                 235                 240

Val Glu Cys Pro Pro Cys Pro Ala Pro Pro Val Ala Gly Pro Ser Val
                245                 250                 255

Phe Leu Phe Pro Pro Lys Pro Lys Asp Thr Leu Met Ile Ser Arg Thr
            260                 265                 270

Pro Glu Val Thr Cys Val Val Val Asp Val Ser His Glu Asp Pro Glu
        275                 280                 285
```

```
Val Gln Phe Asn Trp Tyr Val Asp Gly Val Glu Val His Asn Ala Lys
    290                 295                 300

Thr Lys Pro Arg Glu Glu Gln Phe Asn Ser Thr Phe Arg Val Val Ser
305                 310                 315                 320

Val Leu Thr Val Val His Gln Asp Trp Leu Asn Gly Lys Glu Tyr Lys
                325                 330                 335

Cys Lys Val Ser Asn Lys Gly Leu Pro Ala Pro Ile Glu Lys Thr Ile
            340                 345                 350

Ser Lys Thr Lys Gly Gln Pro Arg Glu Pro Gln Val Tyr Thr Leu Pro
        355                 360                 365

Pro Ser Arg Glu Glu Met Thr Lys Asn Gln Val Ser Leu Thr Cys Leu
    370                 375                 380

Val Lys Gly Phe Tyr Pro Ser Asp Ile Ala Val Glu Trp Glu Ser Asn
385                 390                 395                 400

Gly Gln Pro Glu Asn Asn Tyr Lys Thr Thr Pro Pro Met Leu Asp Ser
                405                 410                 415

Asp Gly Ser Phe Phe Leu Tyr Ser Lys Leu Thr Val Asp Lys Ser Arg
            420                 425                 430

Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys
    450                 455                 460


<210>  15
<211>  239
<212>  PRT
<213>  Artificial Sequence

<220>
<223>  hMAb1-L1 aa

<400>  15

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Leu Ser Leu Pro
            20                  25                  30

Val Thr Leu Gly Glu Pro Ala Ser Ile Ser Cys Arg Ser Ser Gln Ser
```

35 40 45

Leu Glu Asn Ser Asn Gly Asn Thr Tyr Leu Asn Trp Tyr Leu Gln Lys
50 55 60

Pro Gly Gln Ser Pro Gln Leu Leu Ile Tyr Arg Val Ser Asn Arg Phe
65 70 75 80

Ser Gly Val Pro Asp Arg Phe Ser Gly Ser Gly Ser Gly Thr Asp Phe
85 90 95

Thr Leu Lys Ile Ser Arg Val Glu Ala Glu Asp Val Gly Val Tyr Tyr
100 105 110

Cys Leu Gln Val Thr His Val Pro Trp Thr Phe Gly Pro Gly Thr Lys
115 120 125

Val Asp Ile Lys Arg Thr Val Ala Ala Pro Ser Val Phe Ile Phe Pro
130 135 140

Pro Ser Asp Glu Gln Leu Lys Ser Gly Thr Ala Ser Val Val Cys Leu
145 150 155 160

Leu Asn Asn Phe Tyr Pro Arg Glu Ala Lys Val Gln Trp Lys Val Asp
165 170 175

Asn Ala Leu Gln Ser Gly Asn Ser Gln Glu Ser Val Thr Glu Gln Asp
180 185 190

Ser Lys Asp Ser Thr Tyr Ser Leu Ser Ser Thr Leu Thr Leu Ser Lys
195 200 205

Ala Asp Tyr Glu Lys His Lys Val Tyr Ala Cys Glu Val Thr His Gln
210 215 220

Gly Leu Ser Ser Pro Val Thr Lys Ser Phe Asn Arg Gly Glu Cys
225 230 235

<210> 16
<211> 239
<212> PRT
<213> Artificial Sequence

<220>
<223> hMAb1-L2 aa

<400> 16

Met Val Leu Gln Thr Gln Val Phe Ile Ser Leu Leu Leu Trp Ile Ser
1 5 10 15

Gly Ala Tyr Gly Asp Ile Val Met Thr Gln Ser Pro Leu Ser Leu Pro
20 25 30

Val Thr Leu Gly Glu Pro Ala Ser Ile Ser Cys Arg Ser Ser Gln Ser
35 40 45

Leu Glu Asn Glu Asn Lys Asn Leu Tyr Leu Asn Trp Tyr Leu Gln Lys
50 55 60

Pro Gly Gln Ser Pro Gln Leu Leu Ile Tyr Arg Val Ser Asn Arg Phe
65 70 75 80

Ser Gly Val Pro Asp Arg Phe Ser Gly Ser Gly Ser Gly Thr Asp Phe
85 90 95

Thr Leu Lys Ile Ser Arg Val Glu Ala Glu Asp Val Gly Val Tyr Tyr
100 105 110

Cys Leu Gln Val Thr His Val Pro Trp Thr Phe Gly Pro Gly Thr Lys
115 120 125

Val Asp Ile Lys Arg Thr Val Ala Ala Pro Ser Val Phe Ile Phe Pro
130 135 140

Pro Ser Asp Glu Gln Leu Lys Ser Gly Thr Ala Ser Val Val Cys Leu
145 150 155 160

Leu Asn Asn Phe Tyr Pro Arg Glu Ala Lys Val Gln Trp Lys Val Asp
165 170 175

Asn Ala Leu Gln Ser Gly Asn Ser Gln Glu Ser Val Thr Glu Gln Asp
180 185 190

Ser Lys Asp Ser Thr Tyr Ser Leu Ser Ser Thr Leu Thr Leu Ser Lys
195 200 205

Ala Asp Tyr Glu Lys His Lys Val Tyr Ala Cys Glu Val Thr His Gln
210 215 220

Gly Leu Ser Ser Pro Val Thr Lys Ser Phe Asn Arg Gly Glu Cys
225 230 235

<210> 17
<211> 1389
<212> DNA
<213> Artificial Sequence

<220>
<223> hMAb1-H1

<400> 17
atgaaacacc tgtggttctt cctcctgctg gtggcagctc ccagatgggt gctgagcgag 60

gtgcagctgg tggaaagcgg cggaggcctg gtgcagcctg gcggctctct gagactgagc 120

tgtgccgcca gcggcttcac cttcagcacc tacgccatga gctgggtccg acaggcccct 180

ggcaagggac tggaatgggt ggcaaccatc agcaacggcg gcacctacac ctactacccc 240

gacagcgtga agggccggtt caccatcagc cgggacaacg ccaagaacac cctgtacctg 300

cagatgaaca gcctgcgggc cgaggacacc gccgtgtact actgcgccag actgatctac 360

tacgactacc tggactactg gggccagggc accctggtca ccgtcagctc agccagcacc 420

aagggccctt ccgtgttccc tctggcccct tgtagccgtt ccaccagcga gtccaccgcc 480

gcccttggct gtctggtgaa ggactacttc cctgagcctg tgaccgtgag ctggaactcc 540

ggagccctta ccagcggcgt gcacaccttc cctgccgtgc tgcagtccag cggcctttac 600

tccctgagct ccgtggtgac cgtgcctagc tccaacttcg gcacccaaac ctacacctgt 660

aacgtggacc acaagcctag caacaccaag gtggacaaga ccgtggagcg taagtgttgt 720

gtggagtgtc ctccttgtcc tgcccctcct gtggccggac cttccgtgtt ccttttccct 780

cctaagccta aggacaccct gatgatcagc cgtacccctg aggtgacctg tgtggtggtg 840

gacgtgtccc acgaggaccc tgaggtgcag ttcaactggt acgtggacgg cgtggaggtg 900

cacaacgcca agaccaagcc tcgtgaggag caattcaaca gcaccttccg tgtggtgtcc 960

gtgcttaccg tggtgcacca agactggctg aacggcaagg agtacaagtg taaggtgagc 1020

aacaagggac ttcctgcccc tatcgagaag accatctcca agaccaaggg ccaacctcgt 1080

gagcctcaag tgtacaccct tcctcctagc cgtgaggaga tgaccaagaa ccaagtgtcc 1140

cttacctgtc tggtgaaggg cttctaccct agcgacatcg ccgtggagtg ggagtccaac 1200

ggacaacctg agaacaacta caagaccacc cctcctatgc ttgacagcga cggctccttc 1260

ttcctgtaca gcaagctgac cgtggacaag tcccgttggc aacaaggcaa cgtgttcagc 1320

tgttccgtga tgcacgaggc cctgcacaac cactacaccc aaaagagcct ttccctgagc 1380

cctggaaag 1389

<210> 18
<211> 1389
<212> DNA
<213> Artificial Sequence

<220>
<223> hMAb1-H2

<400> 18
atgaaacacc tgtggttctt cctcctgctg gtggcagctc ccagatgggt gctgagcgag 60

gtgcagctgg tggaaagcgg cggaggcctg gtgcagcctg gcggctctct gagactgagc 120

tgtgccgcca gcggcttcac cttcagcacc tacgccatga gctgggtccg acaggcccct 180

ggcaagggac tggaatgggt ggcaaccatc agccaaggcg gcacctacac ctactacccc 240

gacagcgtga agggccggtt caccatcagc cgggacaacg ccaagaacac cctgtacctg 300

cagatgaaca gcctgcgggc cgaggacacc gccgtgtact actgcgccag actgatctac 360

tacgactacc tggactactg gggccagggc accctggtca ccgtcagctc agccagcacc 420

aagggccctt ccgtgttccc tctggcccct tgtagccgtt ccaccagcga gtccaccgcc 480

gcccttggct gtctggtgaa ggactacttc cctgagcctg tgaccgtgag ctggaactcc 540

ggagccctta ccagcggcgt gcacaccttc cctgccgtgc tgcagtccag cggcctttac 600

tccctgagct ccgtggtgac cgtgcctagc tccaacttcg gcacccaaac ctacacctgt 660

aacgtggacc acaagcctag caacaccaag gtggacaaga ccgtggagcg taagtgttgt 720

gtggagtgtc ctccttgtcc tgcccctcct gtggccggac cttccgtgtt ccttttccct 780

cctaagccta aggacaccct gatgatcagc cgtacccctg aggtgacctg tgtggtggtg 840

gacgtgtccc acgaggaccc tgaggtgcag ttcaactggt acgtggacgg cgtggaggtg 900

cacaacgcca agaccaagcc tcgtgaggag caattcaaca gcaccttccg tgtggtgtcc 960

gtgcttaccg tggtgcacca agactggctg aacggcaagg agtacaagtg taaggtgagc 1020

aacaagggac ttcctgcccc tatcgagaag accatctcca agaccaaggg ccaacctcgt 1080

gagcctcaag tgtacaccct tcctcctagc cgtgaggaga tgaccaagaa ccaagtgtcc 1140

cttacctgtc tggtgaaggg cttctaccct agcgacatcg ccgtggagtg ggagtccaac 1200

ggacaacctg agaacaacta caagaccacc cctcctatgc ttgacagcga cggctccttc 1260

ttcctgtaca gcaagctgac cgtggacaag tcccgttggc aacaaggcaa cgtgttcagc 1320

tgttccgtga tgcacgaggc cctgcacaac cactacaccc aaaagagcct ttccctgagc 1380

cctggaaag 1389

<210> 19
<211> 1389
<212> DNA
<213> Artificial Sequence

<220>
<223> hMAb1-H3

<400> 19
atgaaacacc tgtggttctt cctcctgctg gtggcagctc ccagatgggt gctgagcgag 60

gtgcagctgg tggaaagcgg cggaggcctg gtgcagcctg gcggctctct gagactgagc 120

tgtgccgcca gcggcttcac cttcagcacc tacgccatga gctgggtccg acaggcccct 180

ggcaagcggc tggaatgggt ggcaaccatc agcaacggcg gcacctacac ctactacccc 240

gacagcgtga agggccggtt caccatcagc cgggacaacg ccaagaacac cctgtacctg 300

cagatgaaca gcctgcgggc cgaggacacc gccgtgtact actgcgccag actgatctac 360

tacgactacc tggactactg gggccagggc accctggtca ccgtcagctc agccagcacc 420

aagggccctt ccgtgttccc tctggcccct tgtagccgtt ccaccagcga gtccaccgcc 480

gcccttggct gtctggtgaa ggactacttc cctgagcctg tgaccgtgag ctggaactcc 540

ggagccctta ccagcggcgt gcacaccttc cctgccgtgc tgcagtccag cggcctttac 600

tccctgagct ccgtggtgac cgtgcctagc tccaacttcg gcacccaaac ctacacctgt 660

aacgtggacc acaagcctag caacaccaag gtggacaaga ccgtggagcg taagtgttgt 720

gtggagtgtc ctccttgtcc tgcccctcct gtggccggac cttccgtgtt ccttttccct 780

cctaagccta aggacaccct gatgatcagc cgtacccctg aggtgacctg tgtggtggtg 840

gacgtgtccc acgaggaccc tgaggtgcag ttcaactggt acgtggacgg cgtggaggtg 900

cacaacgcca agaccaagcc tcgtgaggag caattcaaca gcaccttccg tgtggtgtcc 960

gtgcttaccg tggtgcacca agactggctg aacggcaagg agtacaagtg taaggtgagc 1020

aacaagggac ttcctgcccc tatcgagaag accatctcca agaccaaggg ccaacctcgt 1080

gagcctcaag tgtacaccct tcctcctagc cgtgaggaga tgaccaagaa ccaagtgtcc 1140

cttacctgtc tggtgaaggg cttctaccct agcgacatcg ccgtggagtg ggagtccaac 1200

ggacaacctg agaacaacta caagaccacc cctcctatgc ttgacagcga cggctccttc 1260

ttcctgtaca gcaagctgac cgtggacaag tcccgttggc aacaaggcaa cgtgttcagc 1320

tgttccgtga tgcacgaggc cctgcacaac cactacaccc aaaagagcct ttccctgagc 1380

cctggaaag 1389

<210> 20
<211> 1389
<212> DNA
<213> Artificial Sequence

<220>
<223> hMAb1-H4

<400> 20
atgaaacacc tgtggttctt cctcctgctg gtggcagctc ccagatgggt gctgagcgag 60

gtgcagctgg tggaaagcgg cggaggcctg gtgcagcctg gcggctctct gagactgagc 120

tgtgccgcca gcggcttcac cttcagcacc tacgccatga gctgggtccg acaggcccct 180

ggcaagcggc tggaatgggt ggcaaccatc agccaaggcg gcacctacac ctactacccc 240

gacagcgtga agggccggtt caccatcagc cgggacaacg ccaagaacac cctgtacctg 300

cagatgaaca gcctgcgggc cgaggacacc gccgtgtact actgcgccag actgatctac 360

tacgactacc tggactactg gggccagggc accctggtca ccgtcagctc agccagcacc 420

aagggccctt ccgtgttccc tctggcccct tgtagccgtt ccaccagcga gtccaccgcc 480

gcccttggct gtctggtgaa ggactacttc cctgagcctg tgaccgtgag ctggaactcc 540

ggagccctta ccagcggcgt gcacaccttc cctgccgtgc tgcagtccag cggcctttac 600

tccctgagct ccgtggtgac cgtgcctagc tccaacttcg gcacccaaac ctacacctgt 660

aacgtggacc acaagcctag caacaccaag gtggacaaga ccgtggagcg taagtgttgt 720

gtggagtgtc ctccttgtcc tgcccctcct gtggccggac cttccgtgtt cctttttccct 780

cctaagccta aggacaccct gatgatcagc cgtacccctg aggtgacctg tgtggtggtg 840

gacgtgtccc acgaggaccc tgaggtgcag ttcaactggt acgtggacgg cgtggaggtg 900

cacaacgcca agaccaagcc tcgtgaggag caattcaaca gcaccttccg tgtggtgtcc 960

gtgcttaccg tggtgcacca agactggctg aacggcaagg agtacaagtg taaggtgagc 1020

aacaagggac ttcctgcccc tatcgagaag accatctcca agaccaaggg ccaacctcgt 1080

gagcctcaag tgtacaccct tcctcctagc cgtgaggaga tgaccaagaa ccaagtgtcc 1140

cttacctgtc tggtgaaggg cttctaccct agcgacatcg ccgtggagtg ggagtccaac 1200

ggacaacctg agaacaacta caagaccacc cctcctatgc ttgacagcga cggctccttc 1260

ttcctgtaca gcaagctgac cgtggacaag tcccgttggc aacaaggcaa cgtgttcagc 1320

tgttccgtga tgcacgaggc cctgcacaac cactacaccc aaaagagcct ttccctgagc 1380

cctggaaag 1389

<210> 21
<211> 717
<212> DNA
<213> Artificial Sequence

<220>
<223> hMAb1-L1

<400> 21
atggtgctgc agacccaggt gttcatctcc ctgctgctgt ggatctccgg cgcgtacggc 60

gacatcgtga tgacccagag ccccctgagc ctgcccgtga cactgggcga gcctgccagc 120

atcagctgca gaagcagcca gagcctggaa aacagcaacg gcaacaccta cctgaactgg 180

tatctgcaga agcccggcca gtcccccccag ctgctgatct accgggtgtc caaccggttc 240

agcggcgtgc ccgacagatt cagcggcagc ggctccggca ccgacttcac cctgaagatc 300

agccgggtgg aagccgagga cgtgggcgtg tactactgtc tgcaggtcac acacgtgccc 360

tggaccttcg gccctggcac caaggtggac atcaagcgta cggtggccgc cccctccgtg 420

ttcatcttcc ccccctccga cgagcagctg aagtccggca ccgcctccgt ggtgtgcctg 480

```
ctgaataact tctaccccag agaggccaag gtgcagtgga aggtggacaa cgccctgcag      540

tccgggaact cccaggagag cgtgaccgag caggacagca aggacagcac ctacagcctg      600

agcagcaccc tgaccctgag caaagccgac tacgagaagc acaaggtgta cgcctgcgag      660

gtgacccacc agggcctgag ctcccccgtc accaagagct tcaacagggg ggagtgt         717


<210>  22
<211>  717
<212>  DNA
<213>  Artificial Sequence

<220>
<223>  hMAb1-L2

<400>  22
atggtgctgc agacccaggt gttcatctcc ctgctgctgt ggatctccgg cgcgtacggc       60

gacatcgtga tgacccagag cccccctgagc ctgcccgtga cactgggcga gcctgccagc      120

atcagctgca gaagcagcca gagcctggaa aacgagaaca agaacctgta cctgaactgg      180

tatctgcaga agcccggcca gtccccccag ctgctgatct accgggtgtc caaccggttc      240

agcggcgtgc ccgacagatt cagcggcagc ggctccggca ccgacttcac cctgaagatc      300

agccgggtgg aagccgagga cgtgggcgtg tactactgtc tgcaggtcac acacgtgccc      360

tggaccttcg gccctggcac caaggtggac atcaagcgta cggtggccgc cccctccgtg      420

ttcatcttcc cccctccga cgagcagctg aagtccggca ccgcctccgt ggtgtgcctg      480

ctgaataact tctaccccag agaggccaag gtgcagtgga aggtggacaa cgccctgcag      540

tccgggaact cccaggagag cgtgaccgag caggacagca aggacagcac ctacagcctg      600

agcagcaccc tgaccctgag caaagccgac tacgagaagc acaaggtgta cgcctgcgag      660

gtgacccacc agggcctgag ctcccccgtc accaagagct tcaacagggg ggagtgt         717
```

**Description**

Technical Field

The present invention relates to: a combination of a novel antibody or functional fragment thereof or a modified form of the antibody or functional fragment thereof and an additional active ingredient; a pharmaceutical composition comprising the antibody or functional fragment thereof or a modified form of the antibody or functional fragment thereof and an additional active ingredient; and the like.

Background Art

The number of patients with ophthalmic angiogenic diseases such as age-related macular degeneration, diabetic retinopathy, macular edema, and central retinal vein occlusion are increasing as the aging of the society proceeds.

Age-related macular degeneration is a primary cause of blindness in adults in developed countries including Japan, United States, and Europe. There are two types of age-related macular degenerations, namely, exudative and atrophic age-related macular degenerations, and they present as the same symptoms but differ in the onset mechanism. Cells called retinal pigment epithelial cells (RPE cells) are present as a monolayer in a lower part of the retina with the macula, and tissue with numerous blood vessels is present beneath the layer, the tissue called choroid. In exudative age-related macular degeneration, RPE cells are damaged because of various factors associated with aging, which causes the choroid to form abnormal neovessels beneath the RPE cells or between the RPE cells and visual cells, and haemorrhage, leakage of blood components, or the like therefrom damages the function of visual cells to rapidly deteriorate the visual acuity.

In treatment of exudative age-related macular degeneration, drug therapy with intravitreal administration of an anti-VEGF drug of ranibizumab (Patent Literature 1), aflibercept (Patent Literature 2), or bevacizumab (Patent Literature 1, Non Patent Literature 1), photodynamic therapy with drip injection of a photosensitive substance followed by laser irradiation to lesions, or combination of them is currently carried out to cause regression of abnormal neovessels generated from the choroid which cause visual acuity. Although some patients show improvement in visual acuity by these therapies, the improvement of visual acuity is insufficient. In addition, other patients are insensitive to these therapies.

Roundabout homolog 4 (ROB04) is a protein having a molecular weight of 110 kDa and a single-pass transmembrane structure (Non Patent Literature 2), and reported to be involved in angiogenesis-suppressing action (Patent Literature 3, Non Patent Literatures 3, 4). An anti-ROBO4 antibody is known to exhibit angiogenesis-suppressing action in a laser-induced choroidal neovascularization model of a monkey, as an animal disease model for exudative age-related macular degeneration and diabetic retinopathy (Patent Literature 4).

However, a drug such that the angiogenesis-suppressing action is enhanced when the drug is used in combination with an anti-ROBO4 antibody is still unknown.

Citation List

Patent Literature

Patent Literature 1: WO98/45331
Patent Literature 2: WO00/075319
Patent Literature 3: WO2008/073441
Patent Literature 4: WO2013/160879

Non Patent Literature

Non Patent Literature 1: Cancer Res, 1997 57(20): 4593-4599
Non Patent Literature 2: Genomics, 2002 79(4), p. 547-552 Non Patent Literature 3: Nature Medicine, 2008(14), p. 448-453
Non Patent Literature 4: Molecular Vision, 2009, Vol. 15, p. 1057-1069

## Summary of the Invention

### Technical Problem

It is an object of the present invention to provide a combination of an anti-ROBO4 antibody with an additional agent, a pharmaceutical composition comprising the antibody and an additional agent, and the like. It is another object of the present invention to provide a combination of an anti-ROBO4 antibody with an additional agent for use in treating or preventing ophthalmic angiogenic diseases (ocular angiogenic diseases), and a pharmaceutical composition comprising the antibody and an additional agent for use in treating or preventing ophthalmic angiogenic diseases.

It is still another object of the present invention to provide a method for treating or preventing an ophthalmic angiogenic disease by administering an anti-ROBO4 antibody and an additional agent in combination, or by administering a pharmaceutical composition comprising the antibody and an additional agent.

### Solution to the Problem

The present inventors have conducted studies directed towards achieving the aforementioned objects. As a result, the present inventors have found that use of an anti-ROBO4 antibody having angiogenesis-suppressing or -inhibiting activity in combination with any of various VEGF antagonists (e.g., aflibercept, ranibizumab, or bevacizumab) shows superior angiogenesis-suppressing effect, thereby completing the present invention.

The present invention relates to the following.

(1) A combination of a monoclonal antibody or antigen-binding fragment thereof having features (i) to (v) below with a VEGF antagonist; or a pharmaceutical composition comprising the antibody or antigen-binding fragment thereof and a VEGF antagonist:

- (i) binding to ROB04 protein with a Kd value of $1 \times 10^{-8}$ M or less;
- (ii) specifically binding to human ROBO4 protein shown in SEQ ID NO: 2 in the sequence listing;
- (iii) suppressing or inhibiting migration of vascular endothelial cells *in vitro* in the absence of a crosslinking antibody;
- (iv) suppressing or inhibiting angiogenesis *in vivo;* or
- (v) being a chimeric antibody or a humanized antibody.

(2) The combination or pharmaceutical composition according to (1), wherein the antibody comprises: a heavy chain comprising CDRH1 consisting of the amino acid sequence shown in SEQ ID NO: 3 (Figure 6) in the sequence listing, CDRH2 consisting of the amino acid sequence shown in SEQ ID NO: 4 (Figure 7) in the sequence listing or the amino acid sequence shown in SEQ ID NO: 5 (Figure 8) in the sequence listing, and CDRH3 consisting of the amino acid sequence shown in SEQ ID NO: 6 (Figure 9) in the sequence listing; and a light chain comprising CDRL1 consisting of the amino acid sequence shown in SEQ ID NO: 7 (Figure 10) in the sequence listing or the amino acid sequence shown in SEQ ID NO: 8 (Figure 11) in the sequence listing, CDRL2 consisting of the amino acid sequence shown in SEQ ID NO: 9 (Figure 12) in the sequence listing, and CDRL3 consisting of the amino acid sequence shown in SEQ ID NO: 10 (Figure 13) in the sequence listing.

(3) The combination or pharmaceutical composition according to (1) or (2), wherein the antibody comprises any one of the following combinations (i) to (vi) of a heavy chain variable region and a light chain variable region:

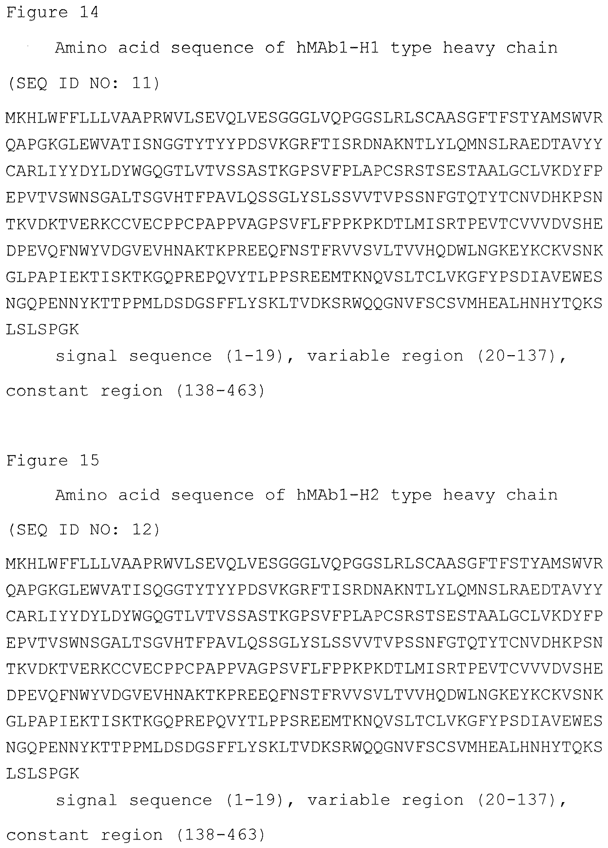

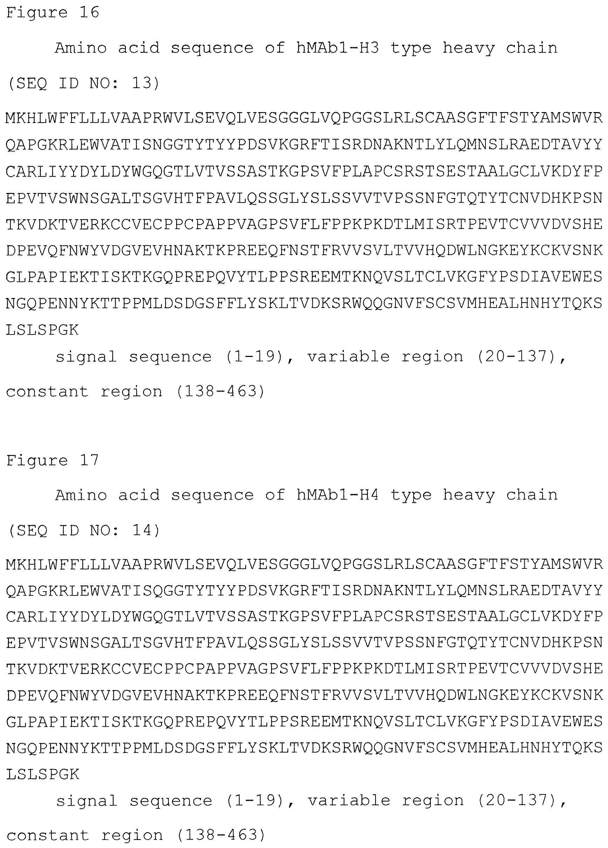

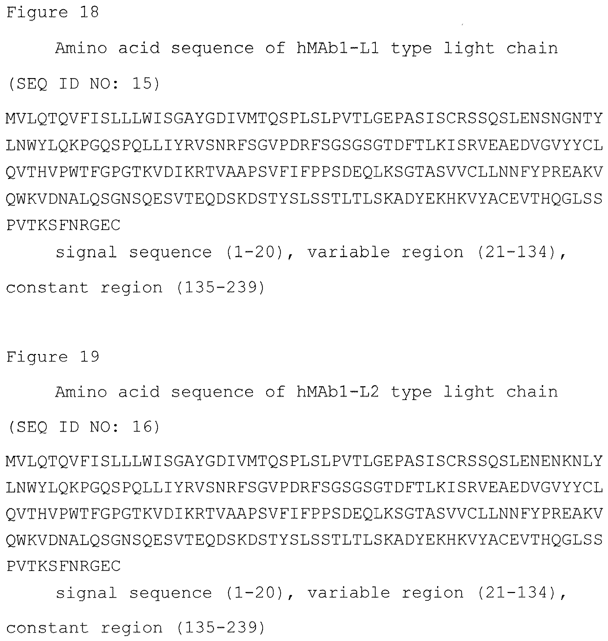

- (i) a heavy chain variable region consisting of the amino acid sequence at amino acid positions 20 to 137 in SEQ ID NO: 12 (Figure 15) in the sequence listing, and a light chain variable region consisting of the amino acid sequence at amino acid positions 21 to 134 in SEQ ID NO: 15 (Figure 18) in the sequence listing;
- (ii) a heavy chain variable region consisting of the amino acid sequence at amino acid positions 20 to 137 in SEQ ID NO: 12 (Figure 15) in the sequence listing, and a light chain variable region consisting of the amino acid sequence at amino acid positions 21 to 134 in SEQ ID NO: 16 (Figure 19) in the sequence listing;
- (iii) a heavy chain variable region consisting of the amino acid sequence at amino acid positions 20 to 137 in SEQ ID NO: 14 (Figure 17) in the sequence listing, and a light chain variable region consisting of the amino acid sequence at amino acid positions 21 to 134 in SEQ ID NO: 16 (Figure 19) in the sequence listing;
- (iv) a heavy chain variable region consisting of the amino acid sequence at amino acid positions 20 to 137 in SEQ ID NO: 14 (Figure 17) in the sequence listing, and a light chain variable region consisting of the amino acid sequence at amino acid positions 21 to 134 in SEQ ID NO: 15 (Figure 18) in the sequence listing;
- (v) a heavy chain variable region consisting of the amino acid sequence at amino acid positions 20 to 137 in SEQ ID NO: 11 (Figure 14) in the sequence listing, and a light chain variable region consisting of the amino acid sequence at amino acid positions 21 to 134 in SEQ ID NO: 15 (Figure 18) in the sequence listing; and
(vi) a heavy chain variable region consisting of the amino acid sequence at amino acid positions 20 to 137 in SEQ ID NO: 13 (Figure 16) in the sequence listing, and a light chain variable region consisting of the amino acid sequence at amino acid positions 21 to 134 in SEQ ID NO: 15 (Figure 18) in the sequence listing.

(4) The combination or pharmaceutical composition according to any one of (1) to (3), wherein the antibody comprises any one of the following combinations (i) to (vi) of a heavy chain and a light chain:

(i) a heavy chain consisting of the amino acid sequence at amino acid positions 20 to 463 in SEQ ID NO: 12 (Figure 15) in the sequence listing, and a light chain consisting of the amino acid sequence at amino acid positions 21 to 239 in SEQ ID NO: 15 (Figure 18) in the sequence listing (H-1140);
(ii) a heavy chain consisting of the amino acid sequence at amino acid positions 20 to 463 in SEQ ID NO: 12 (Figure 15) in the sequence listing, and a light chain consisting of the amino acid sequence at amino acid positions 21 to 239 in SEQ ID NO: 16 (Figure 19) in the sequence listing (H-1143);
(iii) a heavy chain consisting of the amino acid sequence at amino acid positions 20 to 463 in SEQ ID NO: 14 (Figure 17) in the sequence listing, and a light chain consisting of the amino acid sequence at amino acid positions 21 to 239 in SEQ ID NO: 16 (Figure 19) in the sequence listing (H-2143);
(iv) a heavy chain consisting of the amino acid sequence at amino acid positions 20 to 463 in SEQ ID NO: 14 (Figure 17) in the sequence listing, and a light chain consisting of the amino acid sequence at amino acid positions 21 to 239 in SEQ ID NO: 15 (Figure 18) in the sequence listing (H-2140);
(v) a heavy chain consisting of the amino acid sequence at amino acid positions 20 to 463 in SEQ ID NO: 11 (Figure 14) in the sequence listing, and a light chain consisting of the amino acid sequence at amino acid positions 21 to 239 in SEQ ID NO: 15 (Figure 18) in the sequence listing (H-1040); and
(vi) a heavy chain consisting of the amino acid sequence at amino acid positions 20 to 463 in SEQ ID NO: 13 (Figure 16) in the sequence listing, and a light chain consisting of the amino acid sequence at amino acid positions 21 to 239 in SEQ ID NO: 15 (Figure 18) in the sequence listing (H-2040).

(5) The combination or pharmaceutical composition according to (1), comprising a heavy chain and a light chain having an identity of 95% or more to the amino acid sequences of a heavy chain and light chain, respectively, of the antibody according to any one described in (4), and binds to human ROBO4.
(6) The combination or pharmaceutical composition according to (1), wherein the antibody or antigen-binding fragment thereof binds to a site on an antigen recognized by the antibody according to any one of (2) to (4).
(7) The combination or pharmaceutical composition according to (1), wherein the antibody or antigen-binding fragment thereof competes with the antibody according to any one of (2) to (4) for binding to human ROBO4.
(8) A combination of an antibody or antigen-binding fragment thereof obtained by a method comprising steps (i) and (ii) below and a VEGF antagonist; or a pharmaceutical composition comprising the antibody or antigen-binding fragment thereof and a VEGF antagonist:

(i) a step of culturing cells according to (A) and (B) below;

(A) recombinant cells into which a recombinant vector comprising an insert of nucleotide according to any one of (a) to (c) below or the nucleotide has been introduced; or
and the nucleotide is any one of the following nucleotides (a) to (c) :

(a) a nucleotide comprising a nucleotide sequence encoding a partial and/or whole amino acid sequence of a heavy chain and/or light chain of the antibody according to any one of claims (1) to (7);
(b) a nucleotide consisting of a nucleotide sequence comprising a nucleotide sequence encoding a partial and/or whole amino acid sequence of a heavy chain and/or light chain of the antibody according to any one of claims (1) to (7); and
(c) a nucleotide consisting of a nucleotide sequence encoding a partial and/or whole amino acid sequence of a heavy chain and/or light chain of the antibody according to any one of claims (1) to (7),

(B) cells producing the antibody or antigen-binding fragment thereof according to any one of (1) to (7),

(ii) a step of collecting the antibody or antigen-binding fragment thereof according to any one of (1) to (7) from a culture obtained by the step (i).

(9) The combination or pharmaceutical composition according to any one of (1) to (8), wherein one to several amino acids are deleted at the carboxyl terminus of a heavy chain or light chain of the antibody.

(10) A combination of a modified form of the antibody or antigen-binding fragment thereof comprised in the combination or composition according to any one of (1) to (9) and a VEGF antagonist; or a pharmaceutical composition comprising the modified form and a VEGF antagonist.

(11) The combination or pharmaceutical composition according to any one of (1) to (10), which is for use in treating or preventing an angiogenic disease.

(12) The combination or pharmaceutical composition according to (11), wherein the disease is an ocular angiogenic disease.

(13) The combination or pharmaceutical composition according to (12), wherein the ocular angiogenic disease is exudative age-related macular degeneration, diabetic retinopathy, macular edema, intraocular neovascular disease, central retinal vein occlusion, retrolental fibroplasia, proliferative retinopathy, neovascular glaucoma, or immune rejection of transplanted corneal tissue.

(14) A pharmaceutical composition for use in combination with a VEGF antagonist, the pharmaceutical composition comprising the antibody or antigen-binding fragment thereof comprised in the combination or composition according to any one of (1) to (9) or the modified form comprised in the combination or composition according to (10).

(15) A pharmaceutical composition comprising a VEGF antagonist, wherein when used in combination with the antibody or antigen-binding fragment thereof comprised in the combination or composition according to any one of (1) to (9) or the modification comprised in the combination or composition according to (10), the pharmaceutical composition increases effect of the antibody or antigen-binding fragment thereof or the modified form.

(16) A pharmaceutical composition comprising the antibody or antigen-binding fragment thereof comprised in the combination or composition according to any one of (1) to (9) or the modified form comprised in the combination or composition according to (10), wherein when used in combination with a VEGF antagonist, the pharmaceutical composition increases effect of the VEGF antagonist.

(17) A pharmaceutical composition, wherein the antibody or antigen-binding fragment thereof comprised in the combination or composition according to any one of (1) to (9) or the modified form comprised in the combination or composition according to (10) are administered in combination with a VEGF antagonist.

(18) The pharmaceutical composition according to (17), wherein the antibody or antigen-binding fragment thereof comprised in the combination or composition according to any one of (1) to (9) or the modified form comprised in the combination or composition according to (10) and the VEGF antagonist are separately contained in different formulations each as an active ingredient, and administered simultaneously or separately at different times.

(19) The pharmaceutical composition according to (17), wherein the antibody or antigen-binding fragment thereof comprised in the combination or composition according to any one of (1) to (9) or the modified form comprised in the combination or composition according to (10) and the VEGF antagonist are contained in a single formulation as active ingredients.

(20) The pharmaceutical composition according to any one of (1) to (19), wherein the VEGF antagonist is one or two or more selected from the group consisting of an antibody or antigen-binding fragment, a fusion protein, an aptamer, and a low-molecular-weight compound.

(21) The pharmaceutical composition according to any one of (1) to (20), wherein the VEGF antagonist is one or two or more selected from the group consisting of ranibizumab, aflibercept, bevacizumab, brolucizumab, LMG324, RG7716, pegaptanib sodium, abicipar pegol, DE-120, and OPT-302.

(22) The pharmaceutical composition according to (21), wherein the VEGF antagonist is aflibercept.

(23) The pharmaceutical composition according to (21), wherein the VEGF antagonist is ranibizumab.

(24) The pharmaceutical composition according to (21), wherein the VEGF antagonist is bevacizumab.

(25) A method for treating an ocular angiogenic disease, wherein the antibody or antigen-binding fragment thereof comprised in the combination or pharmaceutical composition according to any one of (1) to (9) or the modified form comprised in the combination or pharmaceutical composition according to (10) is administered in combination with a VEGF antagonist.

(26) The method according to (22), wherein the ocular angiogenic disease is exudative age-related macular degeneration, diabetic retinopathy, macular edema, intraocular neovascular disease, central retinal vein occlusion, retrolental fibroplasia, proliferative retinopathy, neovascular glaucoma, or immune rejection of transplanted corneal tissue.

(27) The method according to (22) or (23), wherein the VEGF antagonist is one or two or more selected from the group consisting of an antibody or antigen-binding fragment, a fusion protein, an aptamer, and a low-molecular-weight compound.

(28) The method according to any one of (22) to (24), wherein the VEGF antagonist is one or two or more selected from the group consisting of ranibizumab, aflibercept, bevacizumab, brolucizumab, LMG324, RG7716, pegaptanib sodium, abicipar pegol, DE-120, and OPT-302.

(29) The method according to (28), wherein the VEGF antagonist is aflibercept.

(30) The method according to (28), wherein the VEGF antagonist is ranibizumab.

(31) The method according to (28), wherein the VEGF antagonist is bevacizumab.

Advantageous Effects of the Invention

Use of the combination of an antibody and a VEGF antagonist provided by the present invention enables treatment or prevention of various ocular angiogenic diseases.

Brief Description of the Drawings

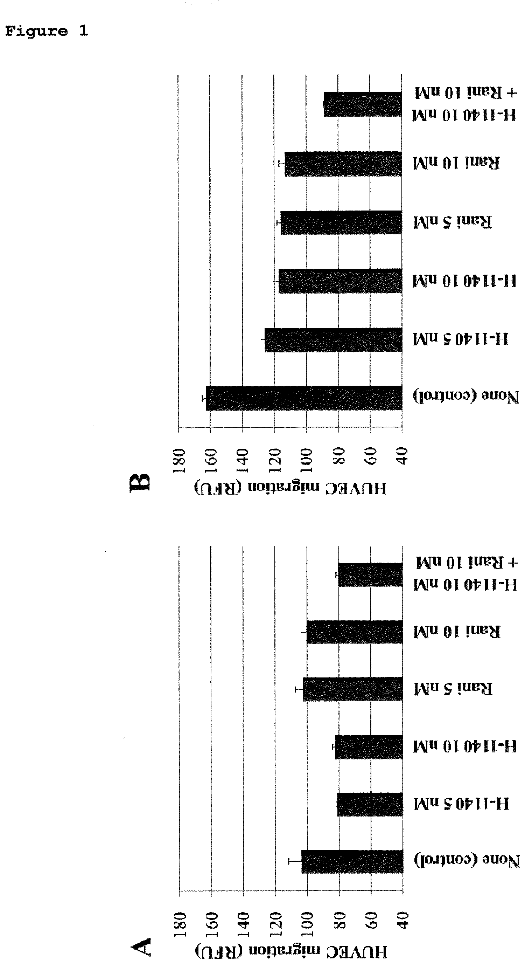

Figure 1A shows the effects of anti-ROBO4 antibody (H-1140) alone, ranibizumab alone, and their combination on HUVEC migration under conditions without stimulation. Each error bar indicates a standard error (n = 3). Figure 1B shows the effects of anti-ROBO4 antibody (H-1140) alone, ranibizumab alone, and their combination on HUVEC migration under conditions with stimulation by H-RPE cell culture supernatant Each error bar indicates a standard error (n = 3).

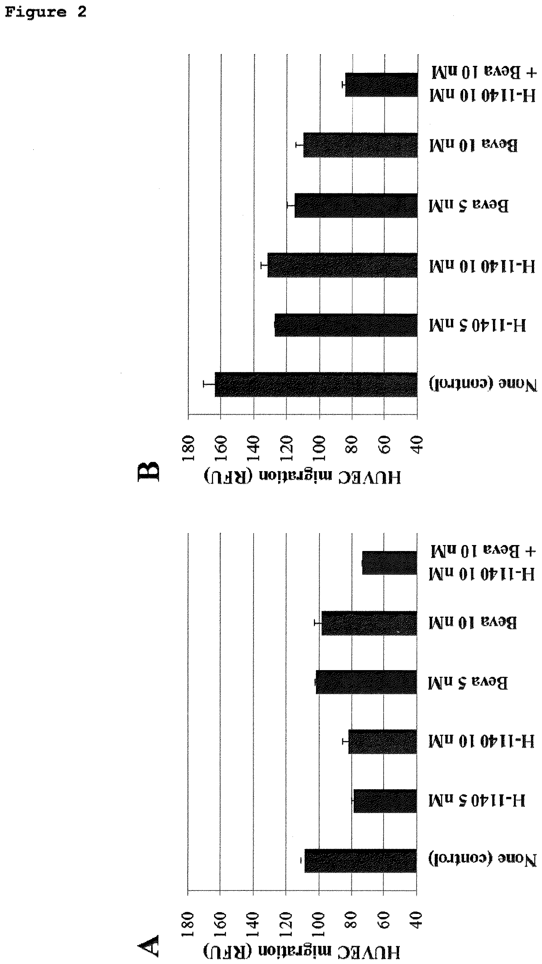

Figure 2A shows the effects of anti-ROBO4 antibody (H-1140) alone, bevacizumab alone, and their combination on HUVEC migration under conditions without stimulation Each error bar indicates a standard error (n = 3). Figure 2B shows the effects of anti-ROBO4 antibody (H-1140) alone, bevacizumab alone, and their combination on HUVEC migration under conditions with stimulation by H-RPE cell culture supernatant Each error bar indicates a standard error (n = 3).

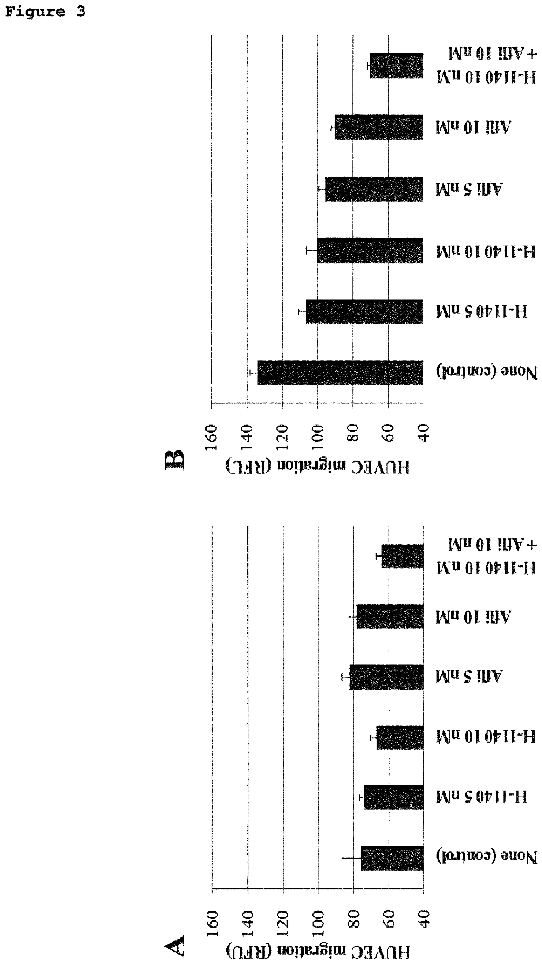

Figure 3A shows the effects of anti-ROBO4 antibody (H-1140) alone, aflibercept alone, and their combination on HUVEC migration under conditions without stimulation. Each error bar indicates a standard error (n = 3). Figure 3B shows the effects of anti-ROB04 antibody (H-1140) alone, aflibercept alone, and their combination on HUVEC migration under conditions with stimulation by H-RPE cell culture supernatant. Each error bar indicates a standard error (n = 3).

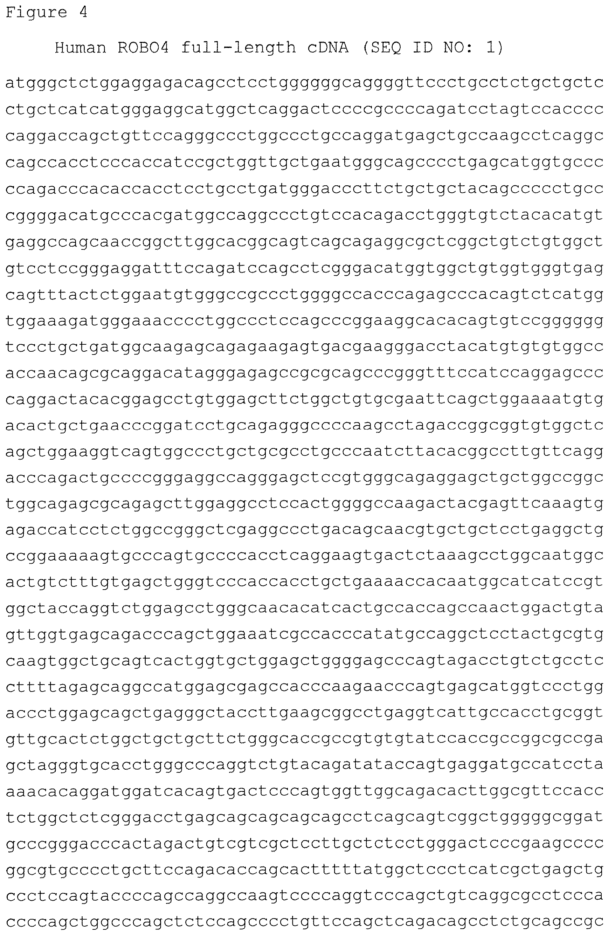

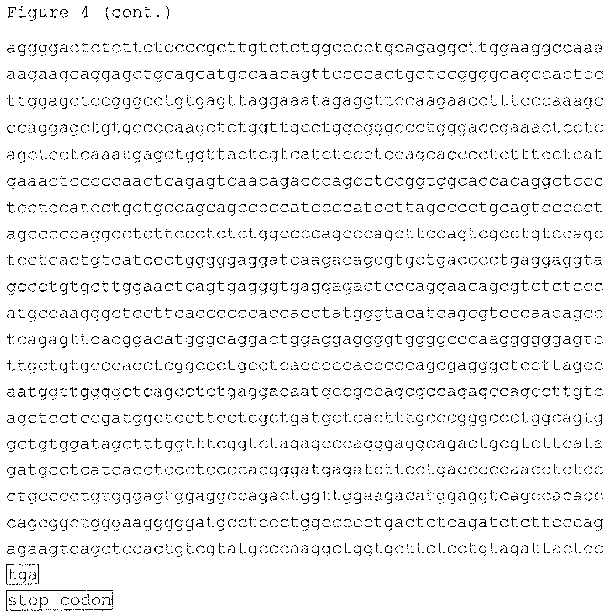

Figure 4 shows human ROB04 full-length cDNA (SEQ ID NO: 1).

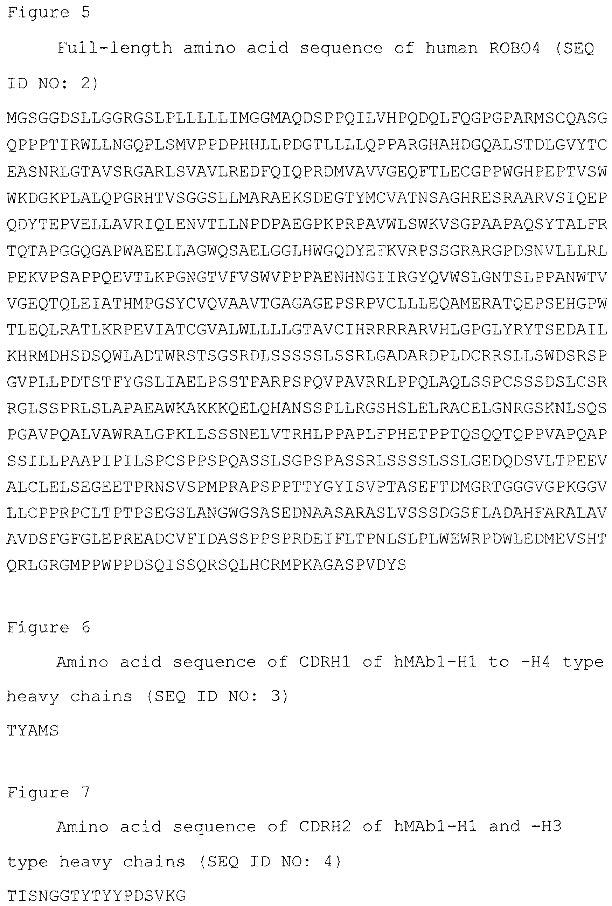

Figure 5 shows the full-length amino acid sequence of human ROBO4 (SEQ ID NO: 2).

Figure 6 shows the amino acid sequence of CDRH1 of hMAb1-H1 to -H4 type heavy chains (SEQ ID NO: 3).

Figure 7 shows the amino acid sequence of CDRH2 of an hMAb1-H1 or -H3 type heavy chain (SEQ ID NO: 4).

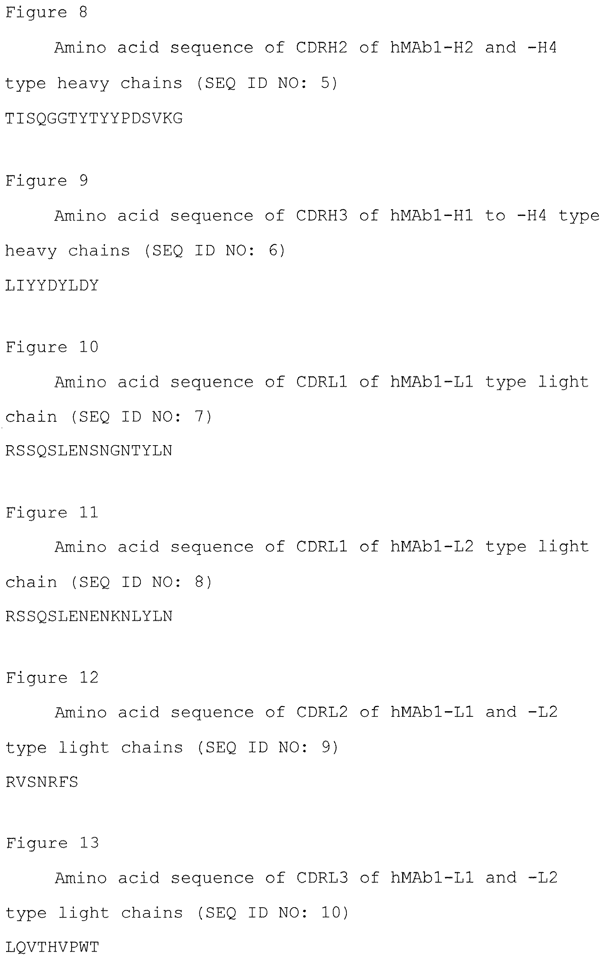

Figure 8 shows the amino acid sequence of CDRH2 of an hMAb1-H2 or -H4 type heavy chain (SEQ ID NO: 5).

Figure 9 shows the amino acid sequence of CDRH3 of hMAb1-H1 to -H4 type heavy chains (SEQ ID NO: 6).

Figure 10 shows the amino acid sequence of CDRL1 of an hMAb1-L1 type light chain (SEQ ID NO: 7).

Figure 11 shows the amino acid sequence of CDRL1 of an hMAb1-L2 type light chain (SEQ ID NO: 8).

Figure 12 shows the amino acid sequence of CDRL2 of hMAb1-L1 and -L2 type light chains (SEQ ID NO: 9).

Figure 13 shows the amino acid sequence of CDRL3 of hMAb1-L1 and -L2 type light chains (SEQ ID NO: 10).

Figure 14 shows the amino acid sequence of an hMAb1-H1 type heavy chain (SEQ ID NO: 11). A signal sequence is at amino acid positions 1 to 19, a variable region is at amino acid positions 20 to 137, and a constant region is at amino acid positions 138 to 463.

Figure 15 shows the amino acid sequence of an hMAb1-H2 type heavy chain (SEQ ID NO: 12). A signal sequence is at amino acid positions 1 to 19, a variable region is at amino acid positions 20 to 137, and a constant region is at amino acid positions 138 to 463.

Figure 16 shows the amino acid sequence of an hMAb1-H3 type heavy chain (SEQ ID NO: 13). A signal sequence is at amino acid positions 1 to 19, a variable region is at amino acid positions 20 to 137, and a constant region is at amino acid positions 138 to 463.

Figure 17 shows the amino acid sequence of an hMAb1-H4 type heavy chain (SEQ ID NO: 14). A signal sequence is at amino acid positions 1 to 19, a variable region is at amino acid positions 20 to 137, and a constant region is at amino acid positions 138 to 463.

Figure 18 shows the amino acid sequence of an hMAb1-L1 type light chain (SEQ ID NO: 15). A signal sequence is at amino acid positions 1 to 20, a variable region is at amino acid positions 21 to 134, and a constant region is at amino acid positions 135 to 239.

Figure 19 shows the amino acid sequence of an hMAb1-L2 type light chain (SEQ ID NO: 16). A signal sequence is at amino acid positions 1 to 20, a variable region is at amino acid positions 21 to 134, and a constant region is at amino acid positions 135 to 239.

Description of Embodiments

1. Definitions

In the present invention, the term "gene" is used to mean a nucleotide including a nucleotide sequence encoding amino acids of protein or a complementary strand thereof, and the term "gene" also includes a polynucleotide, oligonucleotide, DNA, mRNA, cDNA, and cRNA as a nucleotide including a nucleotide sequence encoding amino acids of protein or a complementary strand thereof. Such a gene is a single-stranded, double-stranded, or triple- or more stranded nucleotide, and the term "gene" also includes an associate of a DNA strand and an RNA strand, a mixture of ribonucleotide (RNA) and deoxyribonucleotide (DNA) on a nucleotide strand, and a double-stranded or triple- or more stranded nucleotide including such a nucleotide strand. Examples of the "ROBO4 gene" in the present invention include DNA, mRNA, cDNA, and cRNA including a nucleotide sequence encoding the amino acid sequence of ROBO4 protein.

In the present invention, the terms "nucleotide", "nucleic acid", and "nucleic acid molecule" have the same meaning, and, for example, DNA, RNA, a probe, an oligonucleotide, a polynucleotide, and a primer are also included in the term "nucleotide". Such a nucleotide is a single-stranded, double-stranded, or triple- or more stranded nucleotide, and the term "nucleotide" also includes an associate of a DNA strand and an RNA strand, a mixture of ribonucleotide (RNA) and deoxyribonucleotide (DNA) on a nucleotide strand, and a double-stranded or triple- or more stranded associate including such a nucleotide strand.

In the present invention, the terms "polypeptide", "peptide", and "protein" have the same meaning.

In the present invention, the term "antigen" is occasionally used to mean the term "immunogen".

In the present invention, the term "cell" includes various cells derived from animal individuals, subcultured cells, primary cultured cells, cell lines, recombinant cells, and microorganisms.

In the present invention, an antibody to recognize ROBO4 is occasionally referred to as "anti-ROBO4 antibody". This antibody includes a chimerized antibody, a humanized antibody, and a human antibody.

The term "functional fragment of an antibody" in the present invention is also called "antigen-binding fragment of an antibody", and used to mean an antibody fragment to exhibit at least part of functions exhibited by the original antibody. Examples of such a "functional fragment of an antibody" include antigen-binding fragments such as Fab, $F(ab')_2$, scFv, Fab', and single chain immunoglobulin, but are not limited thereto. Such a functional fragment of an antibody may be a product obtained by treating the full-length molecule of antibody protein with an enzyme such as papain and pepsin, or a recombinant protein produced in appropriate host cells with a recombinant gene.

In the present invention, the term "site" to which an antibody binds, in other words, "site" which an antibody recognizes means a partial peptide or partial higher-order structure on an antigen which an antibody binds to or recognizes. In the present invention, such a site is also called an epitope or a binding site for an antibody. Examples of sites on ROBO4 protein which the anti-ROBO4 antibody of the present invention binds to or recognizes include partial peptides or partial higher-order structures on ROBO4 protein.

It is known that the heavy chain and light chain of an antibody molecule each have three complementarity determining regions (CDRs). Such a complementarity determining region is also referred to as a hypervariable domain, and is located in the variable region of the heavy chain and light chain of an antibody. These regions have a particularly highly variable primary structure and are typically separated into three sites on the primary structure of the polypeptide chain in each of the heavy chain and light chain. In the present invention, with regard to the complementarity determining region of an antibody, the complementarity determining regions of a heavy chain are referred to as CDRH1, CDRH2, and CDRH3, respectively, from the amino-terminal side of the amino acid sequence of the heavy chain, whereas the complementarity determining regions of a light chain are referred to as CDRL1, CDRL2, and CDRL3, respectively, from the amino-terminal side of the amino acid sequence of the light chain. These sites are located close to one another on the three-dimensional structure, and determine the specificity of the antibody to an antigen, to which the antibody binds.

In the present invention, the term "antibody mutant" is used to mean a polypeptide which has an amino acid sequence obtained by substitution, deletion, addition, and/or insertion (hereinafter, collectively referred to as "mutation") of an amino acid of the amino acid sequence possessed by the original antibody, and binds to the ROB04 protein of the present invention. The number of mutated amino acids in such an antibody mutant is 1, 2, 3, 4, 5, 6, 7, 8, 9, 10, 12, 15, 20, 25, 30, 40, or 50. Such antibody mutants are also included in the "antibody" of the present invention.

In the present invention, "several" in the phrase "one to several" refers to 3 to 10.

Examples of the activities/characteristics exhibited by the antibody of the present invention include biological activity and physicochemical characteristics, and specific examples thereof include various biological activities, binding activity to an antigen or epitope, stability in production or storage, and thermal stability.

In the present invention, the phrase "to hybridize under stringent conditions" is used to mean that hybridization is carried out under conditions in which hybridization is carried out in a solution containing 5 × SSC at 65°C, then the resultant is subjected to washing with an aqueous solution containing 2 × SSC-0.1% SDS at 65°C for 20 minutes, washing with an aqueous solution containing 0.5 × SSC-0.1% SDS at 65°C for 20 minutes, and washing with an aqueous solution containing 0.2 × SSC-0.1% SDS at 65°C for 20 minutes, or conditions equivalent thereto. SSC is an aqueous solution of 150 mM NaCl-15 mM sodium citrate, and n × SSC means an n-fold concentration of SSC.

In the present invention, the term "additional active ingredient" or "additional drug" is used to mean a component or drug which has therapeutic or prophylactic activity to diseases and is used in combination with an anti-ROBO4 antibody or comprised in the pharmaceutical composition of the present invention together with an anti-ROBO4 antibody. In a preferred mode, such a combination or pharmaceutical composition is effective for treatment or prevention of angiogenic diseases (preferably, ocular angiogenic diseases).

2. Antigen protein

In the present description, "ROBO4" and "ROBO4 protein" are used to mean the same meaning.

(2-1) Properties

The ROBO4 protein of the present invention has the following characteristics.

(i) ROBO4, a receptor protein for SLIT2 protein, has a molecular weight of 110 kDa and a single-pass transmembrane structure, and is involved in angiogenesis. Although the ROBO4 protein of the present invention can exist as a free form liberated from a membrane such as a cell membrane, the ROBO4 protein of the present invention may be in a form binding to a membrane such as a cell membrane. Here, molecular weight refers to apparent molecular weight in SDS-PAGE under non-reducing condition. In an extracellular region in the N-terminal side of ROBO4, two immunoglobulin-like domains (hereinafter, referred to as "Ig-like domain") and two fibronectin type III domains are present, and a proline-rich region is present in an extracellular region in the C-terminal side. In the present description, the two immunoglobulin-like domains are referred to as an Ig-like domain 1 and an Ig-like domain 2, respectively, from the amino-terminal side. Human ROBO4 protein consists of the amino acid sequence at amino acid positions 28 to 1007 in SEQ ID NO: 2 in the sequence listing. In SEQ ID NO: 2 in the sequence listing, amino acid positions 1 to 27 correspond to a secretion signal, amino acid positions 28 to 467 correspond to an extracellular region, amino acid positions 46 to 131 correspond to an Ig-like domain 1, amino acid positions 137 to 224 correspond to an Ig-like domain 2, amino acid positions 252 to 340 correspond to a fibronectin type III domain 1, amino acid positions 347 to 438 correspond to a fibronectin type III domain 2, amino acid positions 468 to 490 correspond to an intra-cell membrane region, and amino acid positions 491 to 1007 correspond to an intracellular region.

(ii) The ROBO4 protein of the present invention has angiogenesis-suppressing effect. In the present invention, "suppressing angiogenesis" is used to mean directly or indirectly suppressing and/or inhibiting angiogenesis singly, in collaboration with another factor, or as an associate with another factor. Angiogenesis-suppressing effect can be evaluated, for example, on the basis of suppressing effect for vascular permeability, cell migration promoting activity, or lumen formation activity by VEGF, as an index.

(iii) The ROBO4 protein of the present invention comprises any one of the following amino acid sequences (a) to (e) (hereinafter, referred to as "the ROBO4 amino acid sequence"), or consists of an amino acid sequence including the ROBO4 amino acid sequence, or consists of the ROBO4 amino acid sequence:

(a) an amino acid sequence shown in SEQ ID NO: 2 in the sequence listing;
(b) an amino acid sequence having a sequence identity of 80% or more, 82% or more, 84% or more, 86% or more, 88% or more, 90% or more, 92% or more, 94% or more, 96% or more, 98% or more, or 99% or more to the amino acid sequence shown in SEQ ID NO: 2 in the sequence listing, and being the amino acid sequence of a polypeptide which exhibits angiogenesis-suppressing effect;
(c) an amino acid sequence obtained by substituting, deleting, adding, or inserting 1 to 50, 1 to 45, 1 to 40, 1 to 35, 1 to 30, 1 to 25, 1 to 20, 1 to 15, 1 to 10, 1 to 8, 1 to 6, 1 to 5, 1 to 4, 1 to 3, 1 or 2, or 1 amino acid(s) in the amino acid sequence shown in SEQ ID NO: 2 in the sequence listing, and being the amino acid sequence of a polypeptide which suppresses angiogenesis;
(d) an amino acid sequence obtained by deleting at amino acid positions 1 to 45 or 1 to 131 in the amino acid sequence shown in SEQ ID NO: 2 in the sequence listing, and being the amino acid sequence of a polypeptide which suppresses angiogenesis; and
(e) an amino acid sequence encoded by the nucleotide sequence of nucleotides hybridizable under stringent conditions with nucleotides having a nucleotide sequence complementary to a nucleotide sequence encoding the amino acid sequence shown in SEQ ID NO: 2 in the sequence listing, and being the amino acid sequence of a polypeptide which suppresses angiogenesis.

ROBO4 protein can exist as the whole or part of a homo- or heterooligomeric associate composed of two or more subunits.

The amino acid sequence and/or other characteristics of ROBO4 protein are/is not required to be identical or uniform in an individual, tissue, body fluid, cell, ROBO4 protein-containing fraction, or purified or partially purified ROBO4 protein authentic preparation, etc., or among a plurality of individuals, tissues, cells, ROBO4 protein-containing fractions, or ROBO4 protein authentic preparations. ROBO4 proteins having different amino acid sequences and/or different characteristics may be contained in one individual, tissue, body fluid, cell, ROBO4 protein-containing fraction, or purified or partially purified ROBO4 protein authentic preparation, etc. The amino acid sequence and/or other characteristics of ROBO4 protein may be different among a plurality of individuals, tissues, cells, ROBO4 protein-containing fractions, or ROBO4 protein authentic preparations. Even proteins having different amino acid sequences and/or characteristics in this manner are all included in the "ROBO4 protein" of the present invention, if having the characteristics described in (i) to (iii) above.

(iv) The ROBO4 protein of the present invention can be obtained from tissue of a vertebrate, preferably of a mammal, more preferably of a rodent such as a mouse and a rat or a human, even more preferably of a human or a mouse, or cells derived from such tissue, or a culture or the like of such cells. The tissue and cells may be any tissue and cells as long as ROBO4 protein is present therein, and examples thereof include the joint tissue, blood, lymph, thymus, spleen, and cells derived from any of them. Preferred tissue and cells are tissue and cells derived from an animal or patient with angiogenesis. However, the origin of the ROBO4 protein of the present invention is not limited to the aforementioned origins, and ROBO4 proteins even derived from other animal species, other tissue, other cells, etc., are included in the ROBO4 protein of the present invention, if having the characteristics described in (i) to (iii) above.

The ROBO4 protein of the present invention may be natural or recombinant. Fusion products with another peptide or protein such as a carrier and a tag are also included in the ROBO4 protein. Further, the ROBO4 protein includes ROBO4 protein with chemical modification such as addition of polymer such as PEG and/or biological modification including glycosylation. Furthermore, the ROBO4 protein of the present invention includes ROBO4 protein fragments. ROBO4 protein fragments having the characteristics described in (ii) above are called ROBO4 protein functional fragments.

(2-2) ROBO4 gene

The ROBO4 gene of the present invention includes a nucleotide sequence of any one of the following (a) to (c) (hereinafter, referred to as "the ROBO4 gene sequence"), or consists of a nucleotide sequence including the ROBO4 gene sequence, or consists of the ROBO4 gene sequence:

(a) a nucleotide sequence shown in SEQ ID NO: 1 in the sequence listing;

(b) a nucleotide sequence being hybridizable under stringent conditions with a nucleotide consisting of a nucleotide sequence complementary to the nucleotide sequence shown in SEQ ID NO: 1 in the sequence listing, and encoding the amino acid sequence of a polypeptide which suppresses angiogenesis; and

(c) a nucleotide sequence obtained by substituting, deleting, adding, or inserting 1 to 150, 1 to 140, 1 to 130, 1 to 120, 1 to 110, 1 to 100, 1 to 90, 1 to 80, 1 to 70, 1 to 60, 1 to 50, 1 to 45, 1 to 40, 1 to 30, 1 to 25, 1 to 20, 1 to 15, 1 to 10, 1 to 8, 1 to 6, 1 to 5, 1 to 4, 1 to 3, 1 or 2, or 1 nucleotide(s) in the nucleotide sequence shown in SEQ ID NO: 1 in the sequence listing, and encoding the amino acid sequence of a polypeptide which suppresses angiogenesis.

The ROBO4 gene is overexpressed in blood vessels in a fibrovascular membrane or intratumoral blood vessels in a patient with a disease involving angiogenesis such as proliferative diabetic retinopathy. In addition, the ROBO4 gene seems to be overexpressed in tissue or blood fractions derived from a patient affected by a disease presumably associated with angiogenesis such as exudative age-related macular degeneration, macular edema, central retinal vein occlusion, intraocular neovascular disease, atherosclerosis, retrolental fibroplasia, haemangioma, chronic inflammation , proliferative retinopathy, neovascular glaucoma, immune rejection of transplanted corneal tissue or other tissue, rheumatoid arthritis, psoriasis, acute inflammation, sepsis, and adiposity, or derived from a model animal for the disease.

Expression and the expression level of the ROBO4 gene may be determined by using a ROBO4 gene transcript or ROBO4 protein as an index, and the former can be measured, for example, by using an RT-PCR method or a Northern blotting/hybridization method, and the latter can be measured by using immunoassay such as Enzyme-linked immunosorbent assay (hereinafter, referred to as "ELISA") method.

(2-3) Preparation of protein

The ROBO4 protein of the present invention can be prepared, for example, by purification and isolation from animal tissue (including body fluid), cells derived from the tissue, or a culture of the cells, gene recombination, *in vitro* translation, or chemical synthesis.

3. Antibody

(3-1) Classification of antibodies

The antibody of the present invention may be a monoclonal antibody or a polyclonal antibody. Examples of the monoclonal antibody of the present invention include antibodies derived from non-human animals (non-human animal antibodies), antibodies derived from a human (human antibodies), chimerized antibodies (also referred to as "chimeric antibodies"), and humanized antibodies.
Examples of non-human animal antibodies include antibodies derived from vertebrates such as mammals and avians. Examples of antibodies derived from mammals include antibodies derived from rodents such as mouse antibodies and rat antibodies. Examples of antibodies derived from avians include chicken antibodies.
Examples of chimerized antibodies include, but are not limited to, antibodies obtained by binding together a variable region derived from a non-human animal antibody and the human antibody (human immunoglobulin) constant region. Examples of the variable region derived from a non-human animal antibody include heavy chain and light chain variable regions derived from MAb1 (WO2013/160879).
Examples of humanized antibodies include, but are not limited to, those obtained by transplanting CDRs in the variable region of a non-human animal antibody into a human antibody (the variable region of human immunoglobulin), those obtained by transplanting a part of the sequence of the framework regions, in addition to CDRs, of a non-human animal antibody into a human antibody, and those obtained by substituting one or two or more amino acids derived from a non-human animal antibody in any of them with a human-type amino acid(s). Examples of CDRs in the variable region of a non-human animal antibody include CDRH1 to CDRH3 in the above-mentioned heavy chain variable region derived from MAb1 (SEQ ID NOS: 3, 4, 6) and CDRL1 to CDRL3 in the above-mentioned light chain variable region derived from MAb1 (SEQ ID NOS: 7, 9, 10).
Any human antibody which recognizes the antigen of the present invention may be used, and examples thereof include human antibodies which bind to a site to which an antibody including CDRs of the antibody of the present invention binds, and human antibodies which bind to a site on ROBO4 to which the above-mentioned MAb1 binds.
The antibody of the present invention may be any antibody composed of moieties derived from a plurality of different antibodies with activity to bind to ROBO4, and examples thereof include antibodies obtained by exchanging a heavy chain and/or light chain between a plurality of different antibodies, antibodies obtained by exchanging a full-length heavy chain and/or light chain therebetween, antibodies obtained by exchanging only a variable region or only a constant region therebetween, and antibodies obtained by exchanging all or some of CDRs therebetween. The heavy chain variable region and light chain variable region of a chimerized antibody may be derived from different antibodies of the present invention. CDRH1 to CDRH3 and CDRL1 to CDRL3 in the variable region of a heavy chain and light chain of a humanized antibody may be derived from two or more antibodies of the present invention. CDRH1 to CDRH3 and CDRL1 to CDRL3 in the variable region of a heavy chain and light chain of a human antibody may be combination of CDRs included in two or more antibodies of the present invention. Such an antibody composed of moieties derived from a plurality of different antibodies may have one or two or more of activities described in (3-3) to (3-5).
The isotype of the monoclonal antibody of the present invention is not limited, and examples thereof include IgG such as IgG1, IgG2, IgG3, and IgG4; IgM; IgA such as IgA1 and IgA2; IgD; and IgE, and IgG and IgM are preferred, and IgG2 is more preferred. The isotype and subclass of the monoclonal antibody can be determined, for example, by using an Ouchterlony method, an ELISA method, or radio immunoassay (hereinafter, referred to as "RIA"). Commercially available identification kits (Mouse Typer Kit; manufactured by BioRad, MONOCLONAL ANTIBODY ISOTYPING TEST KIT; manufactured by serotec Co., Ltd.) can be utilized.

(3-2) Binding specificity of anti-ROBO4 antibody

The antibody of the present invention recognizes ROBO4 protein. In other words, the antibody of the present invention binds to ROBO4 protein. Such an antibody is referred to as "anti-ROBO4 antibody". A preferred antibody of the present invention specifically recognizes ROBO4 protein. In other words, the preferred antibody of the present invention specifically binds to ROBO4 protein (preferably, human ROBO4 protein (SEQ ID NO: 2)). Moreover, a more preferred antibody according the present invention specifically binds to an Ig-like domain included in ROBO4 protein. Examples of the Ig-like domain include Ig-like domain 1 and Ig-like domain 2, and the more preferred antibody of the present invention recognizes a region consisting of the amino acid sequence at amino acid positions 132 to 209 in SEQ ID NO: 2 in the sequence listing. The antibody of the present invention binds to human ROBO4 protein, monkey ROBO4 protein, preferably cynomolgus monkey ROBO4 protein, and rabbit ROBO4 protein, but does not bind to mouse and rat ROBO4 proteins.

In the present invention, the term "specific recognition", that is, "specific binding" refers to binding differing from nonspecific adsorption. Examples of criteria to determine whether binding is specific or not include dissociation constants (hereinafter, referred to as "KD"). The KD value of the preferred antibody of the present invention to ROBO4 protein is $1 \times 10^{-5}$ M or lower, $5 \times 10^{-6}$ M or lower, $2 \times 10^{-6}$ M or lower, or $1 \times 10^{-6}$ M or lower, more preferably $5 \times 10^{-7}$ M or lower, $2 \times 10^{-7}$ M or lower, or $1 \times 10^{-7}$ M or lower, even more preferably $5 \times 10^{-8}$ M or lower, $2 \times 10^{-8}$ M or lower, or $1 \times 10^{-8}$ M or lower, and still more preferably $5 \times 10^{-9}$ M or lower.

Binding between an antigen and an antibody in the present invention can be measured or determined, for example, by using an ELISA method, an RIA method, or a surface plasmon resonance analysis method. Binding between an antigen expressed on a cell surface and an antibody can be measured, for example, by using a flow cytometric method.

(3-3) *In vitro* angiogenesis-suppressing activity of anti-ROBO4 antibody

The antibody of the present invention has angiogenesis-suppressing activity *in vitro* in the absence of a crosslinking antibody. An antibody which does not exhibit pharmacological activity *in vitro* and exhibits it *in vivo* in the absence of a crosslinking antibody is known (Cancer Cell (2011), 19, p. 101-113). There exist leukocytes to express an Fcγ receptor having a function similar to that of a crosslinking antibody *in vivo* (Nature (2008), 8, p. 34-47), and hence crosslinking occurs in the presence of leukocytes even without a crosslinking antibody, which is inferred to allow the pharmacological activity to be exhibited. In actual living bodies, however, the number of leukocytes in a lesion differs among individuals (Cancer Res (2011), 71, 5670-5677), and for this reason an antibody which exhibits pharmacological activity depending on crosslinking by leukocytes is expected to exhibit effects differing among individuals. Because the antibody of the present invention exhibits superior angiogenesis-suppressing activity *in vitro* even in the absence of a crosslinking antibody, the antibody is inferred to have angiogenesis-suppressing action without depending on the number of leukocytes even *in vivo,* and thus is preferable as a medicament.

Angiogenesis-suppressing activity refers to activity to suppress, for example, proliferation of, migration of, and lumen formation by vascular endothelial cells. Angiogenesis-suppressing activity *in vitro* can be evaluated by using a vascular permeability test, cell migration test for vascular endothelial cells, or lumen formation test.

In the vascular permeability test, for example, normal human umbilical vein endothelial cells (HUVECs) are seeded in an upper layer of a Boyden Chamber with a pore size of 1 μm to allow the HUVECs to form a monolayer, and the permeation rate of FITC-labeled dextran or the like is measured for evaluation. The permeation rate can be measured, for example, by using an In Vitro Vascular Permeability Assay (Cat. ECM640, manufactured by Millipore Corporation) or the like. If an antibody added to a concentration of 10 μg/mL or less exhibits the action of suppressing the permeation rate of FITC-labeled dextran, the antibody can be determined to have vascular permeability-suppressing action and have angiogenesis-suppressing activity. The antibody of the present invention exhibits vascular permeability-suppressing action under the measurement conditions preferably in a concentration of 10 μg/mL or less, more preferably in a concentration of 1 μg/mL or less, particularly preferably in a concentration of 0.5 μg/mL or less.

In the cell migration test, HUVECs are seeded in an upper layer of a Boyden Chamber with a pore size of 3 to 8 μm, and a medium containing migration enhancement factor for endothelial cells such as VEGF is added to the lower layer, and cells which migrate to the lower layer are counted for evaluation. If the effect of reducing the number of migrating HUVECs is exhibited, it is determined that migration-suppressing effect for vascular endothelial cells is present and angiogenesis-suppressing activity is present. For example, measurement can be performed by using a cell migration assay system for vascular endothelial cells. The antibody of the present invention exhibits cell migration-suppressing activity under the measurement conditions preferably in a concentration of 10 μg/mL or less, more preferably in a concentration of 1 μg/mL or less, particularly preferably in a concentration of 0.5 μg/mL or less.

In the lumen formation test, HUVECs are seeded in a cell culture container coated with Matrigel, and the number of branching points, luminal length, and so forth of a luminal structure formed by the HUVECs on the Matrigel are measured for evaluation. If an action of reducing the number of branching points or luminal length of the luminal structure is exhibited, it is determined that lumen formation-suppressing effect is present and angiogenesis-suppressing activity is present. Measurement can be performed, for example, by using a lumen formation assay system for vascular endothelial cells (Cat. 354149, manufactured by BD Bioscience). The antibody of the present invention exhibits lumen formation-suppressing activity under the measurement conditions preferably in a concentration of 10 μg/mL or less, more preferably in a concentration of 1 μg/mL or less, particularly preferably in a concentration of 0.5 μg/mL or less.

However, measurement is not limited to the above-described tests, and any system capable of measuring angiogenesis induced by an angiogenesis-accelerating substance and suppression thereof can be used.

A crosslinking antibody refers to an antibody having the action of binding to the Fc region of the antibody of the present invention to crosslink two or more molecules of the antibody of the present invention. For example, in a where the Fc region of the antibody of the present invention is derived from a mouse, crosslinking antibody means an antibody that binds to the mouse Fc region and associates two or more molecule of the antibody of the present invention through binding of each two antibody molecules of the antibody of the present invention at two binding sites of the crosslinking antibody.

The phrase "having angiogenesis-suppressing activity in the absence of a crosslinking antibody" means that angiogenesis-suppressing effect is exhibited even without coexistence of a crosslinking antibody in an evaluation system for angiogenesis suppression, for example, in any of the above-described evaluation systems.

The phrase "having angiogenesis-suppressing activity in the presence of a crosslinking antibody" means that angiogenesis-suppressing activity is not exhibited in the absence of a crosslinking antibody and angiogenesis-suppressing activity is exhibited when one or more molecules, preferably two or more molecules of a crosslinking antibody coexist per molecule of the antibody of the present invention in an evaluation system for angiogenesis suppression, for example, in any of the above-described evaluation systems for angiogenesis-suppressing activity.

(3-4) *In vivo* angiogenesis-suppressing or -inhibiting activity of anti-ROBO4 antibody

The antibody of the present invention suppresses or inhibits angiogenesis *in vivo.* The *in vivo* angiogenesis-suppressing or -inhibiting activity can be evaluated by using animal disease models in accordance with a common method. For example, a laser-induced choroidal neovascularization model described in WO2013/160879 is widely used as a disease model of angiogenesis, and the amount of neovessels can be used as a score for evaluation. An oxygen-induced retinopathy model can be used as a model of ischaemic retinopathy such as diabetic retinopathy. For a patient, for example, the positions or sizes of choroidal neovessels can be measured through a fluorescence imaging test with fluorescein or indocyanine green before and after administration of the antibody of the present invention. Alternatively, the vascular density of intratumoral blood vessels in a tumor specimen taken from a tumor patient by biopsying can be measured through immunohistochemical analysis (IHC) to score the amount of neovessels before and after administration of the antibody of the present invention.

(3-5) Downstream signal activation by anti-ROBO4 antibody

The anti-ROBO4 antibody of the present invention may be subjected to an evaluation system with cell lines or primary cultured cells to which some response is induced by ROBO4 protein. Examples of such cell lines include mouse vascular endothelial cell lines (ATCC NO. CRL-2779) and examples of such primary cultured cells include mouse vascular endothelial cells and human vascular endothelial cells.

The antibody of the present invention is an agonistic antibody to ROBO4. Specifically, the antibody of the present invention binds to ROBO4 to activate downstream signals of ROBO4. Accordingly, the angiogenesis-suppressing effect of the antibody of the present invention can be evaluated by using activation of downstream signals of ROBO4 as an index. Examples of downstream signals of ROBO4 include IL-8 promoter activity. It has been reported that the promoter activity of IL-8 significantly increases in full-length human ROBO4-expressing cells as compared with cells not expressing human ROBO4, and is hardly found in human ROBO4-expressing cells with the intracellular domain deleted. Hence, the increase of IL-8 promoter activity detected indicates activation of a ROBO4 signal (WO2013/160879). The promoter activity of IL-8 can be evaluated, for example, in a manner such that an anti-ROBO4 antibody is added or an anti-ROBO4 antibody and a crosslinking antibody are added in combination to cells into which a reporter vector including an IL-8 promoter sequence inserted thereinto and a human ROBO4-expressing plasmid have been introduced, followed by measure for reporter activity.

(3-6) Monoclonal antibody

The present invention provides a monoclonal antibody. Examples of the monoclonal antibody include monoclonal antibodies derived from non-human animals such as a rat antibody, a mouse antibody, a rabbit antibody, a chicken antibody, and a fish antibody; chimeric antibodies; humanized antibodies; human antibodies; functional fragments of them; and modified forms of them. Examples of mouse monoclonal antibodies among them include MAb1 (WO2013/160879).

Preferably, the antibody mutant of the present invention has been provided with lowered sensitivity to decomposition or oxidation of protein, improved biological activity, improved antigen-binding ability, or physicochemical characteristics or functional characteristics imparted thereto. Examples of such antibody mutants include antibodies having an amino acid sequence obtained by subjecting the amino acid sequence of an antibody to conservative amino acid substitution. Conservative amino acid substitution is substitution that occurs in an amino acid group related to amino acid side chains (WO2013/160879).

Aspartic acid contained in protein is likely to be converted into isoaspartic acid through isomerization if the amino acid linking to the C-terminus of the aspartic acid has a small side chain, and, on the other hand, asparagine in the like case is likely to be converted into aspartic acid through deamidation, and may be further converted into isoaspartic acid through isomerization. Progression of such isomerization or deamidation may affect the stability of protein. To avoid such isomerization and deamidation, for example, aspartic acid in protein, asparagine, and amino acids adjacent thereto can be substituted with other amino acids. It is preferable that the antibody mutant having such amino acid substitution retain the antigen-biding activity of the original antibody.

The present invention includes antibody mutants having an amino acid sequence obtained by subjecting the amino acid sequence of the antibody of the present invention to conservative amino acid substitution, and mouse antibodies, rat antibodies, chimerized antibodies, humanized antibodies, and human antibodies including a CDRs having an amino acid sequence obtained by subjecting the amino acid sequence of any of CDRH1 to CDRH3 and CDRL1 to CDRL3 derived from MAb1 (WO2013/160879) to conservative amino acid mutation.

The constant region of the antibody of the present invention is not limited, and that of a human antibody is preferably used for the antibody of the present invention for treating or preventing human diseases. Examples of the heavy chain constant region of a human antibody include $C\gamma1$, $C\gamma2$, $C\gamma3$, $C\gamma4$, $C\mu$, $C\delta$, $C\alpha1$, $C\alpha2$, and $C\varepsilon$. Examples of the light chain constant region of a human antibody include $C\kappa$ and $C\lambda$.

Examples of the mouse-human IgG1 type chimeric antibody of the present invention include cMAb1-1, and examples of the mouse-human IgG2 type chimeric antibody of the present invention include cMAb1-2 (WO2013/160879).

(3-7) Functional fragment of anti-ROBO4 antibody

As one aspect of the present invention, the present invention provides a functional fragment of the anti-ROBO4 antibody of the present invention. A fragment of an antibody refers to a fragment retaining at least part of functions of the antibody or modified product thereof. Examples of such functions of an antibody include antigen-binding activity, activity to regulate the activity of an antigen, antibody-dependent cytotoxic activity, and complement-dependent cytotoxic activity. Examples of functions of the anti-ROBO4 antibody of the present invention include ROBO4 protein-binding activity, angiogenesis-suppressing activity, and the effect of activating ROBO4 downstream signals. More specifically, the functional fragment of the antibody of the present invention includes any functional fragment having all or some of the activities described in (3-3) to (3-5) above exhibited by the antibody to ROBO4 of the present invention.

The functional fragment of an antibody may be any fragment of the antibody retaining at least part of activities of the antibody, and examples thereof include, but are not limited to, Fab, F(ab')2, Fv, single chain Fv (scFv) obtained by linking Fvs of a heavy chain and light chain with an appropriate linker, diabodies, linear antibodies, multispecific antibodies formed from antibody fragments, and Fab', as a monovalent fragment of the variable region of an antibody, obtained by treating F(ab')2 under reducing conditions. Molecules including a portion other than a fragment of the antibody of the present invention, such as scFv including a linker portion, are also included in the functional fragment of the antibody of the present invention.

Molecules obtained by deleting one to several or more amino acids at an amino terminus and/or carboxy terminus of antibody protein and retaining at least part of functions of the antibody are also included in the functional fragment of the antibody of the present invention. However, such deletion and modification of a heavy chain sequence does not affect the antigen-binding ability and effector function (activation of complement, antibody-dependent cellular cytotoxicity, etc.) of an antibody. Such modified forms of a functional fragment of an antibody are also included in the antibody of the present invention or functional fragment thereof, or modified form of the antibody or functional fragment thereof (described later).

The antibody of the present invention or functional fragment thereof may be a multispecific antibody having specificity to at least two different antigens. The multispecific antibody is not limited to bispecific antibodies to bind to two different antigens, and antibodies having specificity to three or more different antigens are also included in the "multispecific antibody" of the present invention.

The multispecific antibody of the present invention may be a full-length antibody or a functional fragment thereof (e.g., F(ab')2 bispecific antibody). One aspect of the antibody of the present invention is a single chain antibody (hereinafter, referred to as "scFv"). In addition, the functional fragment of the antibody of the present invention includes Bis-cFv and Multi-scFv, each produced by binding two or more scFvs with a peptide linker, single chain immunoglobulin, single domain antibodies, and nanobodies.

(3-8) Humanized anti-ROBO4 antibody

In one aspect of the present invention, the present invention provides a humanized antibody or functional fragment thereof. Examples of the humanized antibody of the present invention include an antibody obtained by incorporating only complementarity determining regions (CDRs) of Mab1 (WO2013/160879) into a human-derived antibody (see Nature (1986)321, p. 522-525); and an antibody obtained by transplanting not only such CDR sequences but also amino acid residues of some frameworks into a human antibody with CDR grafting (International Publication No. WO90/07861). Any humanized antibody mutant with one to three amino acid residues in each CDR substituted with other amino acid residues is also included in the antibody of the present invention as long as the mutant has all or some of the activities described in (3-3) to (3-5) above.

Examples of the humanized anti-ROBO4 antibody of the present invention or functional fragment thereof include an antibody that comprises: a heavy chain comprising a variable region including CDRH1 consisting of the amino acid sequence shown in SEQ ID NO: 3 (Figure 6) in the sequence listing, CDRH2 consisting of the amino acid sequence shown in SEQ ID NO: 4 (Figure 7) in the sequence listing or the same amino acid with one amino acid substituted, and CDRH3 consisting of the amino acid sequence shown in SEQ ID NO: 6 (Figure 9) in the sequence listing; and a light chain comprising a variable region including CDRL1 consisting of the amino acid sequence shown in SEQ ID NO: 7 (Figure 10) in the sequence listing or the same amino acid sequence with one to three amino acids substituted, CDRL2 consisting of the amino acid sequence shown in SEQ ID NO: 9 (Figure 12) in the sequence listing, and CDRL3 consisting of the amino acid sequence shown in SEQ ID NO: 10 (Figure 13) in the sequence listing and recognizes the ROB04 protein of the present invention or a fragment of the antibody that retains the ROBO4 protein binding activity of the antibody thereof.

Examples of CDRH2 with amino acid substitution include CDRH2 with substitution of an amino acid at amino acid position 4 from the amino acid terminus in the amino acid sequence shown in SEQ ID NO: 4 in the sequence listing. Specifically, asparagine at amino acid position 4 from the amino acid terminus in the amino acid sequence shown in SEQ ID NO: 4 in the sequence listing can be substituted with glutamine (SEQ ID NO: 5 (Figure 8) in the sequence listing). Any amino acid substitution may be used as long as the resultant antibody has all or some of the activities described in (3-3) to (3-5) exhibited by the antibody to ROBO4 of the present invention.

Examples of CDRL1 with amino acid substitution include CDRL1 with substitution of one to three amino acids, preferably of three amino acids, at any or all of amino acid positions 9, 11, and 13 from the amino acid terminus in the amino acid sequence shown in SEQ ID NO: 7 in the sequence listing. As an example of amino acid substitution in CDRL1, CDRL1 with substitution of one to three amino acids, preferably of three amino acids at any or all of amino acid positions 9, 11, and 13 from the amino acid terminus in the amino acid sequence shown in SEQ ID NO: 7 in the sequence listing can be substituted. Specifically, serine (amino acid position 9), glycine (amino acid position 11), and threonine (amino acid position 13) in SEQ ID NO: 7 in the sequence listing can be substituted with amino acids selected from glutamic acid, lysine, and leucine, preferably with glutamic acid, lysine, and leucine, respectively (SEQ ID NO: 8 (Figure 11) in the sequence listing). Any amino acid substitution may be used as long as the resultant antibody has all or some of the activities described in (3-3) to (3-5) exhibited by the antibody to ROBO4 of the present invention.

Asparagine residues in peptide or protein are reported to be susceptible to amidation under certain conditions (Gerger et al: The Journal of Biological Chemistry Vol. 262, No. 2, 785-794, 1987), and hence amino acid substitution in CDRs as described above can enhance the stability of the humanized antibody of the present invention.

Examples of the heavy chain variable region of a more preferred humanized antibody having the CDRHs above include: the amino acid sequence at amino acid positions 20 to 137 in SEQ ID NO: 11 (Figure 14) in the sequence listing in which the CDRH1, the CDRH2, and the CDRH3 are at amino acid positions 50 to 54, 69 to 85, and 118 to 126, respectively, in SEQ ID NO: 11 (Figure 14) in the sequence listing; the amino acid sequence at amino acid positions 20 to 137 in SEQ ID NO: 12 (Figure 15) in the sequence listing in which the CDRH1, the CDRH2, and the CDRH3 are at amino acid positions 50 to 54, 69 to 85, and 118 to 126, respectively, in SEQ ID NO: 12 (Figure 15) in the sequence listing; the amino acid sequence at amino acid positions 20 to 137 in SEQ ID NO: 13 (Figure 16) in the sequence listing in which the CDRH1, the CDRH2, and the CDRH3 are at amino acid positions 50 to 54, 69 to 85, and 118 to 126, respectively, in SEQ ID NO: 13 (Figure 16) in the sequence listing; and the amino acid sequence at amino acid positions 20 to 137 in SEQ ID NO: 14 (Figure 17) in the sequence listing in which the CDRH1, the CDRH2, and the CDRH3 are at amino acid positions 50 to 54, 69 to 85, and 118 to 126, in SEQ ID NO: 14 (Figure 17) in the sequence listing; and examples of the light chain variable region of a more preferred humanized antibody having the CDRLs above include: the amino acid sequence at amino acid positions 21 to 134 in SEQ ID NO: 15 (Figure 18) in the sequence listing in which the CDRL1, the CDRL2, and the CDRL3 are at amino acid positions 44 to 59, 75 to 81, and 114 to 122, respectively, in SEQ ID NO: 15 (Figure 18) in the sequence listing; and the amino acid sequence at amino acid positions 21 to 134 in SEQ ID NO: 16 (Figure 19) in the sequence listing in which the CDRL1, the CDRL2, and the CDRL3 are at amino acid positions 44 to 59, 75 to 81, and 114 to 122, respectively, in SEQ ID NO: 16 (Figure 19) in the sequence listing.

Examples of more preferred humanized antibodies with more preferred combination of a heavy chain variable region and light chain variable region include: a humanized antibody comprising a heavy chain variable region consisting of the amino acid sequence at amino acid positions 20 to 137 in SEQ ID NO: 12 (Figure 15) in the sequence listing and a light chain variable region consisting of the amino acid sequence at amino acid positions 21 to 134 in SEQ ID NO: 15 (Figure 18) in the sequence listing; a humanized antibody comprising a heavy chain variable region consisting of the amino acid sequence at amino acid positions 20 to 137 in SEQ ID NO: 12 (Figure 15) in the sequence listing and a light chain variable region consisting of the amino acid sequence at amino acid positions 21 to 134 in SEQ ID NO: 16 (Figure 19) in the sequence listing; a humanized antibody comprising a heavy chain variable region consisting of the amino acid sequence at amino acid positions 20 to 137 in SEQ ID NO: 14 (Figure 17) in the sequence listing and a light chain variable region consisting of the amino acid sequence at amino acid positions 21 to 134 in SEQ ID NO: 16 (Figure 19) in the sequence listing; a humanized antibody comprising a heavy chain variable region consisting of the amino acid sequence at amino acid positions 20 to 137 in SEQ ID NO: 14 (Figure 17) in the sequence listing and a light chain variable region consisting of the amino acid sequence at amino acid positions 21 to 134 in SEQ ID NO: 15 (Figure 18) in the sequence listing; a humanized antibody comprising a heavy chain variable region consisting of the amino acid sequence at amino acid positions 20 to 137 in SEQ ID NO: 11 (Figure 14) in the sequence listing and a light chain variable region consisting of the amino acid sequence at amino acid positions 21 to 134 in SEQ ID NO: 15 (Figure 18) in the sequence listing; and a humanized antibody comprising a heavy chain variable region consisting of the amino acid sequence at amino acid positions 20 to 137 in SEQ ID NO: 13 (Figure 16) and a light chain variable region consisting of the amino acid sequence at amino acid positions 21 to 134 in SEQ ID NO: 15 (Figure 18) in the sequence listing.

Still more preferred examples of full-length humanized antibodies comprising the more preferred combination of a heavy chain variable region and light chain variable region include: a humanized antibody (H-1140) comprising a heavy chain consisting of the amino acid sequence at amino acid positions 20 to 463 in SEQ ID NO: 12 (Figure 15) in the sequence listing and a light chain consisting of the amino acid sequence at amino acid positions 21 to 239 in SEQ ID NO: 15 (Figure 18) in the sequence listing; a humanized antibody (H-1143) comprising a heavy chain consisting of the amino acid sequence at amino acid positions 20 to 463 in SEQ ID NO: 12 (Figure 15) in the sequence listing and a light chain consisting of the amino acid sequence at amino acid positions 21 to 239 in SEQ ID NO: 16 (Figure 19) in the sequence listing; a humanized antibody (H-2143) comprising a heavy chain consisting of the amino acid sequence at amino acid positions 20 to 463 in SEQ ID NO: 14 (Figure 17) in the sequence listing and a light chain consisting of the amino acid sequence at amino acid positions 21 to 239 in SEQ ID NO: 16 (Figure 19) in the sequence listing; a humanized antibody (H-2140) comprising a heavy chain consisting of the amino acid sequence at amino acid positions 20 to 463 in SEQ ID NO: 14 (Figure 17) in the sequence listing and a light chain consisting of the amino acid sequence at amino acid positions 21 to 239 in SEQ ID NO: 15 (Figure 18) in the sequence listing; a humanized antibody (H-1040) comprising a heavy chain consisting of the amino acid sequence at amino acid positions 20 to 463 in SEQ ID NO: 11 (Figure 14) in the sequence listing and a light chain consisting of the amino acid sequence at amino acid positions 21 to 239 in SEQ ID NO: 15 (Figure 18) in the sequence listing; and a humanized antibody (H-2040) comprising a heavy chain consisting of the amino acid sequence at amino acid positions 20 to 463 in SEQ ID NO: 13 (Figure 16) in the sequence listing and a light chain consisting of the amino acid sequence at amino acid positions 21 to 239 in SEQ ID NO: 15 (Figure 18) in the sequence listing.

The most preferred antibodies in the present invention are H-1140, H-1143, H-2140, and H-2143.

H-1140 has properties: 1) to specifically bind to human ROBO4, without binding to ROBO1, ROBO2, and ROB03; 2) to have a KD value of 3.9 nM to human ROBO4; 3) to maintain the affinity for human ROBO4 at 40°C for 4 weeks; 4) to inhibit the migration of HUVECs induced by one or more angiogenic factors selected from VEGF, bFGF, HGF, PDGF-BB, and SDF-1, in particular, VEGF or bFGF; 5) to inhibit angiogenesis *in vivo;* and 6) to exhibit low immunogenicity in ISPRI Web-based Immunogenicity Screening (EpiVax, Inc.).

H-1143 has properties: 1) to specifically bind to human ROBO4, without binding to ROBO1, ROBO2, and ROBO3; 2) to have a KD value of 3.5 nM to human ROBO4; 3) to maintain the affinity for human ROBO4 at 40°C for 4 weeks; 4) to inhibit the migration of HUVECs induced by one or more angiogenic factors selected from VEGF, bFGF, HGF, PDGF-BB, and SDF-1, in particular, VEGF and bFGF; 5) to inhibit angiogenesis *in vivo;* and 6) to exhibit low immunogenicity in an EpiScreen (trademark) immunogenicity test (Antitope Ltd.).

H-2143 has properties: 1) to specifically bind to human ROBO4, without binding to ROBO1, ROBO2, and ROBO3; 2) to have a KD value of 1.7 nM to human ROBO4; 3) to maintain the affinity for human ROBO4 at 40°C for 4 weeks; 4) to inhibit the migration of HUVECs induced by one or more angiogenic factors, for example, selected from VEGF, bFGF, HGF, PDGF-BB, and SDF-1, in particular, VEGF and bFGF; 5) to inhibit angiogenesis *in vivo;* 6) to exhibit low immunogenicity in an EpiScreen (trademark) immunogenicity test (Antitope Ltd.); and 7) to show no serious change in clinical signs, body weight, food intake, and results in a haematology test, blood chemical analysis, a pathological examination, and electroretinography after single intravitreal injection (2.75 mg/eye) to cynomolgus monkeys.

H-2140 has properties: 1) to specifically bind to human ROBO4, without binding to ROBO1, ROBO2, and ROBO3; 2) to have a KD value of 1.8 nM to human ROBO4; 3) to maintain the affinity for human ROBO4 at 40°C for 4 weeks; 4) to inhibit the migration of HUVECs induced by one or more angiogenic factors selected from VEGF, bFGF, HGF, PDGF-BB, and SDF-1, in particular, VEGF and bFGF; 5) to inhibit angiogenesis *in vivo;* and 6) to exhibit low immunogenicity in an EpiScreen (trademark) immunogenicity test (Antitope Ltd.).

As long as the antibody has all or some of the activities described in (3-3) to (3-5) above, any antibody including an amino acid sequence having an identity of 95% or more, preferably having 97% or more, even more preferably having 99% or more to amino acid sequence of antibodies including H-1140, H-1143, H-2143, H-2140, H-1040, and H-2040 is included in the antibody of the present invention. As long as the antibody has all or some of the activities described in (3-3) to (3-5) above, any antibody that has CDRs identical to amino acid sequence to the CDRs of the antibody comprising the above combination of a heavy chain variable region and a light chain variable region, or any antibody comprising the above combination of a heavy chain and a light chain , and has an amino acid sequence other than the CDR amino acid sequence having 95% or more, more preferably 97% or more, and even more preferably 99% or more identity thereto are included in the antibody of the present invention.

(3-9) Antibody which binds to same site

"Antibodies which bind to the same site" as the antibody provided by the present invention are also included in the antibody of the present invention. An "antibody which binds the same site" as a certain antibody refers to an antibody which binds to a site on an antigen molecule recognized by the certain antibody. If a second antibody binds to a partial peptide or partial three-dimensional structure on an antigen molecule to which a first antibody binds, it can be determined that the first antibody and the second antibody bind to the same site. In addition, by confirming that a second antibody competes with a first antibody for binding of the first antibody to an antigen, that is, that a second antibody interferes with binding of a first antibody to an antigen, it can be determined that the first antibody and the second antibody bind to the same site, even if the specific peptide sequence or three-dimensional structure of the binding site has not been determined. Furthermore, when a first antibody and a second antibody bind to the same site and the first antibody has an effect characteristic to a mode of the antibody of the present invention, such as angiogenesis-suppressing activity, the probability that the second antibody has the same activity is extremely high. Accordingly, if a second anti-ROBO4 antibody binds to a site to which a first anti-ROBO4 antibody binds, it can be determined that the first antibody and the second antibody bind to the same site on ROBO4 protein. In addition, if it is confirmed that a second anti-ROBO4 antibody competes with a first anti-ROBO4 antibody for binding of the first anti-ROBO4 antibody to ROBO4 protein, it can be determined that the first antibody and the second antibody bind to the same site on ROBO4 protein.

The present invention also includes antibodies which bind to a site on the ROBO4 protein invention recognized by MAb1 of the present invention (WO2013/160879).

A binding site to which an antibody binds can be determined by a method well known to a person skilled in the art, such as an immunoassay. For example, the amino acid sequence of an antigen is appropriately truncated from the C-terminus or N-terminus to produce a series of peptides, and the reactivity of an antibody to the peptides is studied, and recognition sites are roughly determined. Thereafter, further shorter peptides are synthesized, and the reactivity of the antibody to these peptides is then studied, so as to determine a binding site. Antigen fragment peptide can be prepared, for example, by using a technique of gene recombination or peptide synthesis.

If an antibody binds to or recognizes a partial higher-order structure of an antigen, the binding site to which the antibody binds can be determined by specifying the amino acid residues on the antigen adjacent to the antibody by X-ray structural analysis.

(3-10) Modified form of anti-ROBO4 antibody or functional fragment thereof

The present invention provides a Modified form of an antibody or functional fragment thereof. The Modified form of the antibody of the present invention or functional fragment thereof refers to the antibody of the present invention or functional fragment thereof, which is chemically or biologically modified. Examples of such a chemical Modified form include the binding of a chemical moiety to an amino acid skeleton, and the chemical Modified form of an N-linked or O-linked carbohydrate chain. Examples of such a biological Modified form include antibodies which have undergone a posttranslational modification (e.g., an N-linked or O-linked glycosylation, N-terminal or C-terminal processing, deamidation, isomerization of aspartic acid, and oxidation of methionine), and antibodies of which a methionine residue is added to the N-terminus by expression using prokaryote host cells. In addition, such a modified form also includes labeled forms for enabling detection or isolation of the antibody of the present invention or an antigen, such as an enzyme-labeled form, a fluorescent-labeled form, and an affinity-labeled form. Such a modified form of the antibody of the present invention or functional fragment thereof is useful for the improvement of the stability and retention in blood of the original antibody of the present invention or functional fragment thereof, a reduction in antigenicity, detection or isolation of such an antibody or antigen, etc.

Examples of chemical moieties included in such a chemically modified form include water-soluble polymers such as polyethylene glycol (PEG), ethylene glycol/propylene glycol copolymer, carboxymethyl cellulose, dextran, and polyvinyl alcohol.

Examples of such a biological modification include products modified with enzymatic treatment, cellular treatment, or the like, fusion form to which other peptide such as a tag has been added through gene recombination, and products prepared by using host cells expressing endogenous or exogenous glycosylation enzyme.

Such modification may be performed at an arbitrary position or an intended position in an antibody or functional fragment thereof, and the same modification or two or more different modifications may be performed at one or two or more positions.

The antibody of the present invention may further be a conjugate formed by such an antibody and another drug (Immunoconjugate). Such an antibody can be, for example, an antibody that binds to a radioactive substance or a compound having pharmacological action (WO2013/160879).

In the present invention, the term "a Modified form of an antibody fragment" includes "a fragment of a modification of an antibody".

It is known that the lysine residue at the carboxyl terminus of the heavy chain of an antibody produced in cultured mammalian cells is deleted (Journal of Chromatography A, 705: 129-134 (1995)), and also, it is known that the two amino acid residues at the heavy chain carboxyl terminus, glycine and lysine, are deleted, and that the proline residue positioned newly at the carboxyl terminus is amidated (Analytical Biochemistry, 360: 75-83 (2007)). However, such deletion and modification of a heavy chain sequence do not affect the antigen-binding ability and effector function (activation of complement, antibody-dependent cellular cytotoxicity, etc.) of an antibody. Accordingly, the present invention also includes an antibody or functional fragment thereof that has undergone the aforementioned modification, and specific examples of such an antibody include a deletion mutant comprising a deletion of one or two amino acids at the heavy chain carboxyl terminus, and a deletion mutant formed by amidating the aforementioned deletion mutant (e.g., a heavy chain in which the proline residue at the carboxyl terminal site is amidated). However, deletion mutants involving a deletion at the carboxyl terminus of the heavy chain of the antibody according to the present invention are not limited to the above described deletion mutants, as long as they retain antigen-binding ability and all or some of the activities described in (3-3) to (3-5). Two heavy chains constituting the antibody according to the present invention may be any one type of heavy chain selected from the group consisting of a full-length antibody and the above described deletion mutants, or a combination of any two types selected from the aforementioned group. The ratio of individual deletion mutants can be affected, for example, by the types of cultured mammalian cells that produce the antibody according to the present invention, and the culture conditions. The main ingredient of the antibody according to the present invention can be antibodies where one amino acid residue is deleted at each of the carboxyl termini of the two heavy chains. Such antibodies are all included in the antibody mutant of the present invention or functional fragment thereof, and the modified form of the antibody mutant or functional fragment thereof.

## 4. Method for producing antibody

### (4-1) Method using hybridoma

As one aspect of the present invention, the anti-ROBO4 antibody can be obtained, for example, in accordance with a method by Kohler and Milstein (Kohler and Milstein, Nature (1975)256, p. 495-497, Kennet, R. ed., Monoclonal Antibodies, p. 365-367, Plenum Press, N.Y. (1980)) (WO2013/160879).

### (4-2) Cell immunization method

The anti-ROBO4 antibody can be prepared in accordance with the hybridoma method, for example, by using cells expressing natural ROBO4 protein or cells expressing recombinant ROBO4 protein or a fragment thereof as immunogens (WO2013/160879).

### (4-3) DNA immunization method

The anti-ROBO4 antibody of the present invention can be also obtained by using a DNA immunization method. An antigen-expressing plasmid is transfected into an animal individual such as a mouse and a rat to allow the plasmid to express an antigen in the individual, thereby inducing immunity to the antigen. Existing examples of transfection techniques include a method of directly injecting a plasmid into the muscle, a method of intravenously injecting an introduction reagent such as liposomes and polyethyleneimine, a technique of using a virus vector, a technique of shooting a gold particle to which a plasmid has been attached with a Gene Gun, and a Hydrodynamic method to intravenously inject a huge amount of plasmid solution in a rapid fashion.

### (4-4) Gene recombination

The antibody of the present invention can be prepared in a manner such that a nucleotide including a nucleotide sequence encoding the amino acid sequence of the heavy chain (heavy chain nucleotide) and a nucleotide including a nucleotide sequence encoding the amino acid sequence of the light chain (light chain nucleotide), or a vector into which the heavy chain nucleotide has been inserted and a vector into which the light chain nucleotide has been inserted are introduced into host cells, and the cells are cultured and then the antibody of interest is collected from the culture, and the antibody obtained in this manner is also included in the present invention (WO2013/160879).

(4-5) Methods for designing and preparing humanized antibody

Examples of humanized antibodies include, but are not limited to, antibodies obtained by incorporating only CDRs of a non-human animal antibody into a human-derived antibody (see Nature (1986)321, p. 522-525); antibodies obtained by transplanting not only such CDR sequences but also amino acid residues of some frameworks into a human antibody with CDR grafting (see WO90/07861, US6972323); and such antibodies in which one or two or more amino acids of a non-human animal antibody have been substituted with human-type amino acids in any of these humanized antibodies (WO2013/160879).

(4-6) Method for preparing human antibody

Another example of the antibody of the present invention can be a human antibody. A human anti-ROBO4 antibody refers to an anti-ROBO4 antibody consisting of the amino acid sequence of a human-derived antibody. Such a human anti-ROBO4 antibody can be obtained by using a known method such as a method using a human antibody-producing mouse having a genome DNA fragment comprising the heavy chain and light chain genes of a human antibody, and a method of obtaining a phage display-derived human antibody that has been selected from a human antibody library (WO2013/160879).

(4-7) Method for preparing functional fragment of antibody

Methods for producing a single chain antibody are known (e.g., see U.S. Patent Nos. 4,946,778, 5,260,203, 5,091,513, 5,455,030).

Functional fragments of other antibodies can be obtained similarly in accordance with a common method, in which a gene encoding such a functional fragment is obtained and introduced into cells, and the functional fragment is collected from the culture of the cells.

The antibody of the present invention may be an antibody that has been multimerized to enhance affinity for an antigen. The antibody to be multimerized may be either a single type of antibody, or multiple antibodies recognizing multiple epitopes of a single antigen.

(4-8) Purification of antibody

The obtained antibody can be purified to a homogenous state. For separation and purification of the antibody, separation and purification methods used for ordinary proteins may be used.

For example, column chromatography, filtration, ultrafiltration, salting-out, dialysis, preparative polyacrylamide gel electrophoresis, isoelectric focusing, etc. are appropriately selected and combined with one another, so that the antibody can be separated and purified, though the separation and purification methods are not limited thereto.

(4-9) Recombinant antibody

The present invention includes an antibody or function fragment thereof, or a modified form of the antibody or function fragment thereof obtained by using a method for producing an antibody or function fragment thereof, or a modified form of the antibody or function fragment thereof, the method comprising a step of culturing cells into which a nucleotide (antibody gene) or vector encoding the antibody of the present invention or functional fragment thereof or a modified form of the antibody or functional fragment thereof has been introduced, and collecting the antibody or functional fragment thereof or a modified form of the antibody or functional fragment thereof from the culture.

5. Pharmaceutical composition

The present invention provides a pharmaceutical composition comprising an anti-ROBO4 antibody or functional fragment thereof, or a modified form of the anti-ROBO4 antibody or functional fragment thereof.

The pharmaceutical composition of the present invention is useful for treatment or prevention of diseases such that shows angiogenesis as one of pathological findings in the course of onset, progression, and/or exacerbation, and suppression of such angiogenesis and/or vascular permeability improves the pathological condition (hereinafter, referred to as "angiogenetic diseases" for convenience), preferably of ophthalmic angiogenic diseases (ocular angiogenic diseases). Examples of angiogenic diseases include exudative age-related macular degeneration, diabetic retinopathy, macular edema (e.g., diabetic macular edema), central retinal vein occlusion, benign or malignant tumor, atherosclerosis, retrolental fibroplasia, haemangioma, chronic inflammation , intraocular neovascular disease, proliferative retinopathy, neovascular glaucoma, immune rejection of transplanted corneal tissue or other tissue, rheumatoid arthritis, psoriasis, acute inflammation, sepsis, and adiposity, and preferred examples are exudative age-related macular degeneration, diabetic retinopathy, macular edema (e.g., diabetic macular edema), central retinal vein occlusion, intraocular neovascular disease, proliferative retinopathy, neovascular glaucoma, and immune rejection of transplanted corneal tissue.

In the present invention, treatment and/or therapeutic prevention of a disease include(s), but are not limited to, prevention of the onset of such a disease, preferably of such a disease in an individual with expression of ROBO4 protein, or suppression or inhibition of the exacerbation or progression thereof; mitigation of one or more symptoms exhibited by an individual affected with such a disease, or suppression or remission of the exacerbation or progression thereof; and treatment or prevention of secondary diseases.

The pharmaceutical composition of the present invention can comprise a therapeutically or prophylactically effective amount of an anti-ROBO4 antibody or functional fragment of the antibody, and a pharmaceutically acceptable diluent, carrier, solubilizer, emulsifier, preservative, and/or adjuvant.

The phrase "therapeutically or prophylactically effective amount" is used to mean an amount such that therapeutic or prophylactic effect is exerted with a specific mode of administration and administration route for a specific disease.

The pharmaceutical composition of the present invention can comprise a substance (hereinafter, referred to as "pharmaceutical substance") for altering, maintaining, or retaining pH, osmotic pressure, viscosity, transparency, color, isotonicity, sterility, the stability of the composition or an antibody comprised therein, solubility, sustained releasability, absorbability, permeability, dosage form, strength, character, shape, etc. The pharmaceutical substance may be any pharmacologically acceptable substance. For example, being non-toxic or having low toxicity is a property preferably possessed by the pharmaceutical substance.

Examples of the pharmaceutical substance can include the following substances, but are not limited thereto: amino acids such as glycine, alanine, glutamine, asparagine, histidine, arginine or lysine; antibacterial agents; antioxidants such as ascorbic acid, sodium sulfate or sodium hydrogen sulfite; buffers such as a phosphate, citrate or borate buffer, sodium hydrogen carbonate, or a Tris-HCl solution; fillers such as mannitol or glycine; chelating agents such as ethylenediaminetetraacetic acid (EDTA); complexing agents such as caffeine, polyvinylpyrrolidine, β-cyclodextrin or hydroxypropyl-β-cyclodextrin; bulking agents such as glucose, mannose or dextrin; other carbohydrates such as monosaccharides, disaccharides, glucose, mannose, and dextrin; a coloring agent; a flavor agent; a diluent; an emulsifier; hydrophilic polymers such as polyvinylpyrrolidine; a low-molecular-weight polypeptide; salt-forming counterions; antiseptics such as benzalkonium chloride, benzoic acid, salicylic acid, thimerosal, phenethyl alcohol, methylparaben, propylparaben, chlorhexidine, sorbic acid or hydrogen peroxide; solvents such as glycerin, propylene glycol or polyethylene glycol; sugar alcohols such as mannitol or sorbitol; suspending agent; PEG; sorbitan esters; polysorbates such as polysorbate 20 or polysorbate 80; surfactants such as Triton, tromethamine, lecithin or cholesterol; stability enhancers such as sucrose or sorbitol; elasticity enhancers such as sodium chloride, potassium chloride, mannitol or sorbitol; transporting agents; diluents; excipients; and/or pharmaceutical adjuvants.

The amount of such a pharmaceutical substance to be added is 0.001 to 1000 times, preferably 0.01 to 100 times, more preferably 0.1 to 10 times higher than the weight of the anti-ROBO4 antibody or functional fragment thereof or a modified form of the anti-ROBO4 antibody or functional fragment thereof.

The pharmaceutical composition of the present invention also includes a pharmaceutical composition comprising an immunoliposome comprising the anti-ROBO4 antibody or functional fragment thereof or a modified form of the anti-ROBO4 antibody or functional fragment thereof in a liposome, or an modified form of the antibody in which the antibody and a liposome are binding together (e.g., U.S. Patent No. 6214388).

An excipient or a carrier is typically a liquid or a solid, and may be any of water for injection, normal saline, an artificial cerebrospinal fluid, or other substances commonly used in formulations for oral administration or parenteral administration. Examples of normal saline include neutral normal saline and normal saline comprising serum albumin.

Examples of buffers include a Tris buffer prepared to provide the pharmaceutical composition with a final pH of 7.0 to 8.5, an acetate buffer prepared for a final pH of 4.0 to 5.5, a citrate buffer prepared for a final pH of 5.0 to 8.0, and a histidine buffer prepared for a final pH of 5.0 to 8.0.

The pharmaceutical composition of the present invention is a solid, a liquid, a suspension, or the like. A freeze-dried formulation is an example. To form a freeze-dried formulation, an excipient such as sucrose can be used.

The administration route of the pharmaceutical composition of the present invention may be any of enteral administration, topical administration, and parenteral administration, and a preferred administration route depending on a disease of interest can be selected. Specific examples thereof include intravenous administration, intraarterial administration, intramuscular administration, intradermal administration, subcutaneous administration, intraperitoneal administration, transdermal administration, intraosseous administration, and intraarticular administration.

In addition, intraocular or intravitreal administration can be preferably used for ocular angiogenic diseases including exudative age-related macular degeneration, diabetic retinopathy, macular edema (e.g., diabetic macular edema), central retinal vein occlusion, retrolental fibroplasia, intraocular neovascular disease, proliferative retinopathy, neovascular glaucoma, and immune rejection of transplanted corneal tissue.

The composition of the pharmaceutical composition can be determined depending on the administration method, the binding affinity of the antibody for ROBO4 protein, etc. As the anti-ROBO4 antibody of the present invention or functional fragment thereof or a modified form of the anti-ROBO4 antibody or functional fragment thereof has higher affinity for ROBO4 protein (i.e., the KD value is lower), the pharmaceutical composition can exhibit medicinal effects even with a smaller dose.

The dose of the anti-ROBO4 antibody of the present invention can be appropriately determined depending on the species of an individual, the type of a disease, symptoms, sex, age, chronic illness, the binding affinity of the antibody for ROBO4 protein or the biological activity of the antibody, and other factors, and typically 0.01 to 1000 mg/kg, preferably 0.1 to 100 mg/kg of the anti-ROBO4 antibody of the present invention can be administered once every 1 to 180 days, or twice or three times or more a day.

Examples of the form of the pharmaceutical composition include an injection (including a freeze-dried formulation, a drip infusion), a suppository, a transnasal absorption formulation, a transdermal absorption formulation, a sublingual agent, a capsule, a tablet, an ointment, a granule, an aerosol, a pill, a powder, a suspension, an emulsion, an ophthalmic preparation, and a formulation for biological implantation.

The anti-ROBO4 antibody or functional fragment thereof or a modified form of the anti-ROBO4 antibody or functional fragment thereof (hereinafter, referred to as "the anti-ROBO4 antibody or the like") can be used in combination with an additional agent. The anti-ROBO4 antibody or the like, or a pharmaceutical composition comprising the anti-ROBO4 antibody or the like as an active ingredient can be administered simultaneously with or separately from a pharmaceutical composition comprising an additional agent, namely, a drug other than the anti-ROBO4 antibody or the like, as an active ingredient. For example, a pharmaceutical composition comprising the anti-ROBO4 antibody or the like as an active ingredient may be administered after an additional agent is administered; an additional agent may be administered after a pharmaceutical composition comprising the anti-ROBO4 antibody or the like as an active ingredient is administered; and a pharmaceutical composition comprising the anti-ROBO4 antibody or the like as an active ingredient and an additional agent may be simultaneously administered. In the present invention, both the case where the anti-ROBO4 antibody or the like and an additional agent are both comprised as active ingredients in a single pharmaceutical composition and the case where the active ingredients are comprised separately in a plurality of pharmaceutical compositions are referred to as a "pharmaceutical composition comprising the anti-ROBO4 antibody or the like and an additional agent". In the present invention, such a "pharmaceutical composition" has the same meaning as a "pharmaceutical composition such that the anti-ROBO4 antibody or the like and an additional agent are administered in combination".

In the present invention, the situation that the anti-ROBO4 antibody or the like and an additional agent are "administered in combination" means that the anti-ROBO4 antibody or the like and an additional agent are incorporated in the body of a subject by administration in a certain period of time. A single formulation containing the anti-ROBO4 antibody or the like and an additional agent may be administered, and they may be separately formulated and separately administered. In a case where the anti-ROBO4 antibody or the like and an additional agent are separately formulated, the timing of administration is not limited, and the formulations may be administered simultaneously, or separately at different times with a certain interval of time, or on different days. In a case where the anti-ROBO4 antibody or the like and an additional agent are administered separately at different times or on different days, the order of administration is not limited. Since different formulations are typically administered in different administration methods, the number of administrations is identical between the formulations in some cases and different therebetween in other cases. In a case where the anti-ROBO4 antibody or the like and an additional agent are separately formulated, the administration methods (administration routes) for the formulations may be identical, and the formulations may be administered in different administration methods (administration routes). The anti-ROBO4 antibody or the like and an additional agent are not required to be simultaneously present in the body, and only required to be incorporated in the body for a certain period of time (e.g., for 1 month, preferably for 1 week, more preferably for several days, even more preferably for 1 day), and one of the active ingredients may have disappeared from the body when the other is administered.

Examples of the mode of administration of the "pharmaceutical composition such that the anti-ROBO4 antibody or the like and an additional agent are administered in combination" include: 1) administration of a single formulation containing the anti-ROBO4 antibody or the like and an additional agent; 2) simultaneous administration of two formulations obtained by separately formulating the anti-ROBO4 antibody or the like and an additional agent through the same administration route; 3) administration of two formulations obtained by separately formulating the anti-ROBO4 antibody or the like and an additional agent through the same administration route with an interval of time; 4) simultaneous administration of two formulations obtained by separately formulating the anti-ROBO4 antibody or the like and an additional agent through different administration routes; and 5) administration of two formulations obtained by separately formulating the anti-ROBO4 antibody or the like and an additional agent through different administration routes with an interval of time. The dose, interval of administration, mode of administration, formulation, etc., of the "pharmaceutical composition such that the anti-ROBO4 antibody or the like and an additional agent are administered in combination" are according to those for the pharmaceutical composition comprising the anti-ROBO4 antibody, though they are not limited thereto.

In a case where the pharmaceutical composition is formulated into two different formulations, a kit comprising the formulations may be applied.

In the present invention, "combination" of the anti-ROBO4 antibody and an additional agent means that the anti-ROBO4 antibody and an additional agent are "administered in combination".

In the present invention, the "additional agent" may be any angiogenesis suppressor, anti-inflammatory drug, or the like, and is preferably VEGF antagonist, PDGF antagonist, or a steroid, and more preferably VEGF antagonist.

Examples of VEGF antagonist include molecules capable of neutralizing, blocking, inhibiting, preventing, reducing, or interfering with VEGF activities including binding to VEGF or one or more VEGF receptors or nucleic acids encoding them.

Examples of preferred VEGF antagonist include an antibody or antigen-binding fragment thereof, fusion protein, immunoadhesin, a nucleic acid, an oligonucleotide, an aptamer, a polypeptide, and a low-molecular-weight compound. Examples of preferred VEGF antagonist include aflibercept (VEGF-trap), ranibizumab, bevacizumab, brolucizumab, LMG324, RG7716, pegaptanib sodium, abicipar pegol, DE-120, and OPT-302. Examples of more preferred VEGF antagonist include aflibercept, ranibizumab, and bevacizumab. VEGF antagonist includes biosimilars (follow-on biologics) and generic medicaments of the antagonist. However, the "additional agent" comprised in the combination or pharmaceutical composition of the present invention is not limited thereto.

A further additional medicament can be used for the combination or pharmaceutical composition of the present invention. Examples of the further additional medicament include various therapies including photodynamic therapy (PDT) and drugs for PDT.

The present invention also provides a method for treating or preventing angiogenic diseases, preferably ROBO4-associated diseases such as ocular angiogenic diseases, use of the antibody of the present invention for preparing a pharmaceutical composition for treating or preventing the disease, and use of the antibody of the present invention for treating or preventing the disease. A kit comprising the antibody of the present invention for treatment or prevention is also included in the present invention.

## Examples

Hereinafter, the present invention will be described in more detail with reference to Examples; however, the present invention is not limited thereto.

Unless otherwise specified, individual operations regarding the following Examples have been carried out by using methods described in experimental manuals used by persons skilled in the art, or when commercially available reagents or kits have been used, the examples have been carried out in accordance with the instructions included in the commercially available products.

### Example 1. Effect of humanized anti-ROBO4 antibody (H-1140) in combination with ranibizumab on HUVEC migration

#### 1)-1 Preparation of human retinal pigment epithelial (H-RPE) cell culture supernatant

H-RPE cells (Lonza Walkersville, Inc) were cultured in 2% Fetal bovine serum (FBS)-containing RtEGM medium (Lonza Walkersville, Inc) at 37°C in a humidified atmosphere containing 5% $CO_2$. H-RPE cells were detached from the dish, and subsequently collected. After centrifugation, the H-RPE cells were resuspended in 2% FCS-containing RtEGM medium at a density of $2 \times 10^5$ cells/mL. To a lower well of a 12 mm Transwell with 0.4 μm Pore Polyester Membrane Insert, Sterile (12-well transwell plate, Corning Incorporated), 2% FCS-containing RtEGM medium was added at 1.5 mL/well, and the cell suspension of $2 \times 10^5$ cells/mL was then added to an upper chamber at 0.5 mL/well. On the next day, the media in the upper chamber and lower well of the 12-well transwell plate were replaced with FCS-free RtEGM medium at 0.5 mL/well and 1.5 mL/well, respectively. Thereafter, the media in the upper chamber and lower chamber were replaced every 2 to 3 days. After culturing for approximately 1 month, the H-RPE cells were washed with HEPES buffer (Kurabo Industries Ltd.), and 0.1% BSA-containing HuMedia-EB2 medium (Kurabo Industries Ltd.) was added to the upper chamber and lower well of the 12-well transwell plate at 0.5 mL/well and 1 mL/well, respectively. After about 25 h of cultivation at 37°C in a humidified atmosphere containing 5% $CO_2$, the medium in the lower well was collected for use as H-RPE cell culture supernatant. The H-RPE cell culture supernatant was stored in liquid nitrogen before use in this study.

1)-2 HUVEC migration assay

HUVECs (Kurabo Industries Ltd.) were cultured in 0.1% BSA-containing HuMedia-EB2 at 37°C in a humidified atmosphere containing 5% $CO_2$ for 19 hours, and then resuspended in 0.1% BSA-containing HuMedia-EB2 at a density of 4 × $10^5$ cells/mL. To an upper chamber of a Corning FluoroBlok HTS 96 Well Multiwell Permeable Support System with 3.0 μm High Density PET Membrane (FluoroBlok HTS 96 Well Multiwell Support System, Corning Incorporated) coated with gelatin, the HUVEC suspension of 4 × $10^5$ cells/mL was added at 50 μL/well, and the following samples were then added to a lower well at 210 μL/well (n = 3).

1): 0.1% BSA-containing medium supplemented with 10 nM IgG2, Kappa from human myeloma plasma (hIgG2, Sigma-Aldrich Co. LLC.) and 10 nM ChromoPure Human IgG, Fab Fragment (hFab, Jakson ImmunoResearch Laboratories, Inc.)
2): 0.1% BSA-containing medium supplemented with 5 nM H-1140 (WO2013/160879, e.g., Example 9, 10), 5 nM hIgG2, and 10 nM hFab
3): 0.1% BSA-containing medium supplemented with 10 nM H-1140 and 10 nM hFab
4): 0.1% BSA-containing medium supplemented with 10 nM hIgG2, 5 nM ranibizumab (Novartis Pharma K.K.), and 5 nM hFab
5): 0.1% BSA-containing medium supplemented with 10 nM hIgG2 and 10 nM ranibizumab
6): 0.1% BSA-containing medium supplemented with 10 nM H-1140 and 10 nM ranibizumab
7): 0.1% BSA-containing medium supplemented with 10 nM hIgG2, 10 nM hFab, and 8.2-fold diluted H-RPE cell culture supernatant
8): 0.1% BSA-containing medium supplemented with 5 nM H-1140, 5 nM hIgG2, 10 nM hFab, and 8.2-fold diluted H-RPE cell culture supernatant
9): 0.1% BSA-containing medium supplemented with 10 nM H-1140, 10 nM hFab, and 8.2-fold diluted H-RPE cell culture supernatant
10): 0.1% BSA-containing medium supplemented with 10 nM hIgG2, 5 nM ranibizumab, 5 nM hFab, and 8.2-fold diluted H-RPE cell culture supernatant
11): 0.1% BSA-containing medium supplemented with 10 nM hIgG2, 10 nM ranibizumab, and 8.2-fold diluted H-RPE cell culture supernatant
12): 0.1% BSA-containing medium supplemented with 10 nM H-1140, 10 nM ranibizumab, and 8.2-fold diluted H-RPE cell culture supernatant

To an upper chamber without HUVECs, 50 μL of 0.1% BSA-containing HuMedia-EB2 was added, and 210 μL of 0.1% BSA-containing HuMedia-EB2 was added to a lower well (blank, n = 3).

The FluoroBlok HTS 96 Well Multiwell Support System to which the cells and the samples had been added was incubated at 37°C in a humidified atmosphere containing 5%$CO_2$. After 3 hours of incubation, HUVECs migrating to the underside of the chamber in the upper plate were washed once with PBS, and labeled by incubating for 15 min at 37°C in a humidified atmosphere containing 5% $CO_2$ with HuMedia-EB2 supplemented with 4 μg/mL of Calcein-AM (Thermo Fisher Scientific, Inc.). Thereafter, fluorescence intensity (excitation wavelength/fluorescence wavelength: 485 nm/538 nm) was measured for each well by using a plate reader (SpectraMAX M3, Molecular Devices, LLC.), and cell migration in each well was calculated according to the following formula.

$$\text{Cell migration} = \text{mean of fluorescence intensity for wells with HUVECs } (n = 3) - \text{mean of fluorescence intensity for wells without HUVECs } (n = 3)$$

The results are shown in Figure 1. Under nonstimulated conditions by H-RPE cell culture supernatant, H-1140 suppressed the migration of HUVECs compared to the control but ranibizumab did not alter it (Figure 1A). H-1140 and ranibizumab in combination also reduced HUVEC migration, but to the same extent as H-1140 alone (Figure 1A). Under stimulated conditions by H-RPE cell culture supernatant, H-1140 and ranibizumab independently reduced HUVEC migration to the same extent (Figure 1B). Cotreatment of H-1140 and ranibizumab further reduced HUVEC migration compared to the individual agents alone (Figure 1B). These results suggest that H-1140 in combination with ranibizumab has the potential to enhance the inhibitory effect on HUVEC migration stimulated by H-RPE cell culture supernatant.

Example 2. Effect of H-1140 in combination with bevacizumab on HUVEC migration

2)-1 Human retinal pigment epithelial (H-RPE) cell culture supernatant

The culture supernatant prepared in Example 1 was used.

2)-2 HUVEC migration assay

A HUVEC migration assay was performed in the manner shown in 1)-2. HUVECs cultured in 0.1% BSA-containing HuMedia-EB2 at 37°C in a humidified atmosphere containing 5% $CO_2$ for approximately 22 hours were used for the migration assay, and the following samples were added to a lower layer of a chamber.

1): 0.1% BSA-containing medium supplemented with 10 nM hIgG2 and 10 nM IgG1, Kappa from human myeloma plasma (hIgG1, Sigma-Aldrich Co. LLC.)
2): 0.1% BSA-containing medium supplemented with 5 nM H-1140, 5 nM hIgG2, and 10 nM hIgG1
3): 0.1% BSA-containing medium supplemented with 10 nM H-1140 and 10 nM hIgG1
4): 0.1% BSA-containing medium supplemented with 10 nM hIgG2, 5 nM bevacizumab (Genentech, Inc.), and 5 nM hIgG1
5): 0.1% BSA-containing medium supplemented with 10 nM hIgG2 and 10 nM bevacizumab
6): 0.1% BSA-containing medium supplemented with 10 nM H-1140 and 10 nM bevacizumab
7): 0.1% BSA-containing medium supplemented with 10 nM hIgG2, 10 nM hIgG1, and 8.2-fold diluted H-RPE cell culture supernatant
8): 0.1% BSA-containing medium supplemented with 5 nM H-1140, 5 nM hIgG2, 10 nM hIgG1, and 8.2-fold diluted H-RPE cell culture supernatant
9): 0.1% BSA-containing medium supplemented with 10 nM H-1140, 10 nM hIgG1, and 8.2-fold diluted H-RPE cell culture supernatant
10): 0.1% BSA-containing medium supplemented with 10 nM hIgG2, 5 nM bevacizumab, 5 nM hIgG1, and 8.2-fold diluted H-RPE cell culture supernatant
11): 0.1% BSA-containing medium supplemented with 10 nM hIgG2, 10 nM bevacizumab, and 8.2-fold diluted H-RPE cell culture supernatant
12): 0.1% BSA-containing medium supplemented with 10 nM H-1140, 10 nM bevacizumab, and 8.2-fold diluted H-RPE cell culture supernatant

The results are shown in Figure 2. Under nonstimulated conditions by H-RPE cell culture supernatant, H-1140 suppressed the migration of HUVECs compared to the control but bevacizumab did not alter it (Figure 2A). H-1140 and bevacizumab in combination also reduced HUVEC migration, but to the same extent as H-1140 alone (Figure 2A). Under stimulated conditions by H-RPE cell culture supernatant, H-1140 and bevacizumab independently reduced HUVEC migration to the same extent (Figure 2B). Cotreatment of H-1140 and bevacizumab further reduced HUVEC migration compared to the individual agents alone (Figure 2B). These results suggest that H-1140 in combination with bevacizumab has the potential to enhance the inhibitory effect on HUVEC migration stimulated by H-RPE cell culture supernatant.

Example 3. Effect of H-1140 in combination with aflibercept on HUVEC migration

3)-1 Human retinal pigment epithelial (H-RPE) cell culture supernatant

The culture supernatant prepared in Example 1 was used.

3)-2 HUVEC migration assay

A HUVEC migration test was performed in the manner shown in 1)-2. HUVECs cultured in 0.1% BSA-containing HuMedia-EB2 at 37°C in a humidified atmosphere containing 5% $CO_2$ for 18 hours were used for the migration assay, and the following samples were added to a lower layer of a chamber.

1): 0.1% BSA-containing medium supplemented with 10 nM hIgG2 and 10 nM Recombinant Human IgG1 Fc, CF (hIgG1 Fc (R&D Systems, Inc.)
2): 0.1% BSA-containing medium supplemented with 5 nM H-1140, 5 nM hIgG2, and 10 nM hIgG1 Fc
3): 0.1% BSA-containing medium supplemented with 10 nM H-1140 and 10 nM hIgG1
4): 0.1% BSA-containing medium supplemented with 10 nM hIgG2, 5 nM aflibercept (Santen Pharmaceutical Co.,

Ltd.), and 5 nM hIgG1 Fc
5): 0.1% BSA-containing medium supplemented with 10 nM hIgG2 and 10 nM aflibercept
6): 0.1% BSA-containing medium supplemented with 10 nM H-1140 and 10 nM aflibercept
7): 0.1% BSA-containing medium supplemented with 10 nM hIgG2, 10 nM hIgG1 Fc, and 8.2-fold diluted H-RPE cell culture supernatant
8): 0.1% BSA-containing medium supplemented with 5 nM H-1140, 5 nM hIgG2, 10 nM hIgG1 Fc, and 8.2-fold diluted H-RPE cell culture supernatant
9): 0.1% BSA-containing medium supplemented with 10 nM H-1140, 10 nM hIgG1 Fc, and 8.2-fold diluted H-RPE cell culture supernatant
10): 0.1% BSA-containing medium supplemented with 10 nM hIgG2, 5 nM aflibercept, 5 nM hIgG1 Fc, and 8.2-fold diluted H-RPE cell culture supernatant
11): 0.1% BSA-containing medium supplemented with 10 nM hIgG2, 10 nM aflibercept, and 8.2-fold diluted H-RPE cell culture supernatant
12): 0.1% BSA-containing medium supplemented with 10 nM H-1140, 10 nM aflibercept, and 8.2-fold diluted H-RPE cell culture supernatant

The results are shown in Figure 3. Under nonstimulated conditions by H-RPE cell culture supernatant, H-1140 suppressed the migration of HUVECs compared to the control but aflibercept did not alter it (Figure 3A). H-1140 and aflibercept in combination also reduced HUVEC migration, but to the same extent as H-1140 alone (Figure 3A). H-1140 and aflibercept in combination also reduced HUVEC migration, but to the same extent as H-1140 alone (Figure 3A). Under stimulated conditions by H-RPE cell culture supernatant, H-1140 and aflibercept independently reduced HUVEC migration to the same extent (Figure 3B). Cotreatment of H-1140 and aflibercept further reduced HUVEC migration compared to the individual agents alone (Figure 3B). These results suggest that H-1140 in combination with aflibercept has the potential to enhance the inhibitory effect on HUVEC migration stimulated by H-RPE cell culture supernatant.

Industrial Applicability

The combination of an antibody and an additional agent provided by the present invention enables treatment or prevention of various ocular angiogenic diseases.

Sequence Listing Free Text

SEQ ID NO: 1: Human ROBO4 full-length cDNA (Figure 4)
SEQ ID NO: 2: Full-length amino acid sequence of human ROBO4 (Figure 5)
SEQ ID NO: 3: Amino acid sequence of CDRH1 of hMAb1-H1 to -H4 type heavy chains (Figure 6)
SEQ ID NO: 4: Amino acid sequence of CDRH2 of an hMAb1-H1 or -H3 type heavy chain (Figure 7)
SEQ ID NO: 5: Amino acid sequence of CDRH2 of an hMAb1-H2 or -H4 type heavy chain (Figure 8)
SEQ ID NO: 6: Amino acid sequence of CDRH3 of hMAb1-H1 to -H4 type heavy chains (Figure 9)
SEQ ID NO: 7: Amino acid sequence of CDRL1 of an hMAb1-L1 type light chain (Figure 10)
SEQ ID NO: 8: Amino acid sequence of CDRL1 of an hMAb1-L2 type light chain (Figure 11)
SEQ ID NO: 9: Amino acid sequence of CDRL2 of hMAb1-L1 and -L2 type light chains (Figure 12)
SEQ ID NO: 10: Amino acid sequence of CDRL3 of hMAb1-L1 and -L2 type light chains (Figure 13)
SEQ ID NO: 11: Amino acid sequence of an hMAb1-H1 type heavy chain (Figure 14)
SEQ ID NO: 12: Amino acid sequence of an hMAb1-H2 type heavy chain (Figure 15)
SEQ ID NO: 13: Amino acid sequence of an hMAb1-H3 type heavy chain (Figure 16)
SEQ ID NO: 14: Amino acid sequence of an hMAb1-H4 type heavy chain (Figure 17)
SEQ ID NO: 15: Amino acid sequence of an hMAb1-L1 type light chain (Figure 18)
SEQ ID NO: 16: Amino acid sequence of an hMAb1-L2 type light chain (Figure 19)
SEQ ID NO: 17: Nucleotide sequence of cDNA encoding an hMAb1-H1 type heavy chain
SEQ ID NO: 18: Nucleotide sequence of cDNA encoding an hMAb1-H2 type heavy chain
SEQ ID NO: 19: Nucleotide sequence of cDNA encoding an hMAb1-H3 type heavy chain
SEQ ID NO: 20: Nucleotide sequence of cDNA encoding an hMAb1-H4 type heavy chain
SEQ ID NO: 21: Nucleotide sequence of cDNA encoding an hMAb1-L1 type light chain
SEQ ID NO: 22: Nucleotide sequence of cDNA encoding an hMAb1-L2 type light chain

## Claims

**1.** A combination of a monoclonal antibody or antigen-binding fragment thereof having features (i) to (v) below and a VEGF antagonist; or a pharmaceutical composition comprising the antibody or antigen-binding fragment thereof and a VEGF antagonist:

(i) binding to ROBO4 protein with a Kd value of $1 \times 10^{-8}$ M or less;
(ii) specifically binding to human ROBO4 protein shown in SEQ ID NO: 2 in the sequence listing;
(iii) suppressing or inhibiting migration of vascular endothelial cells *in vitro* in the absence of a crosslinking antibody;
(iv) suppressing or inhibiting angiogenesis *in vivo;* or
(v) being a chimeric antibody or a humanized antibody.

**2.** The combination or pharmaceutical composition according to claim 1, wherein the antibody comprises: a heavy chain comprising CDRH1 consisting of the amino acid sequence shown in SEQ ID NO: 3 (Figure 6) in the sequence listing, CDRH2 consisting of the amino acid sequence shown in SEQ ID NO: 4 (Figure 7) in the sequence listing or the amino acid sequence shown in SEQ ID NO: 5 (Figure 8) in the sequence listing, and CDRH3 consisting of the amino acid sequence shown in SEQ ID NO: 6 (Figure 9) in the sequence listing; and a light chain comprising CDRL1 consisting of the amino acid sequence shown in SEQ ID NO: 7 (Figure 10) in the sequence listing or the amino acid sequence shown in SEQ ID NO: 8 (Figure 11) in the sequence listing, CDRL2 consisting of the amino acid sequence shown in SEQ ID NO: 9 (Figure 12) in the sequence listing, and CDRL3 consisting of the amino acid sequence shown in SEQ ID NO: 10 (Figure 13) in the sequence listing.

**3.** The combination or pharmaceutical composition according to claim 1 or 2, wherein the antibody comprises any one of the following combinations (i) to (vi) of a heavy chain variable region and a light chain variable region:

(i) a heavy chain variable region consisting of the amino acid sequence at amino acid positions 20 to 137 in SEQ ID NO: 12 (Figure 15) in the sequence listing, and a light chain variable region consisting of the amino acid sequence at amino acid positions 21 to 134 in SEQ ID NO: 15 (Figure 18) in the sequence listing;
(ii) a heavy chain variable region consisting of the amino acid sequence at amino acid positions 20 to 137 in SEQ ID NO: 12 (Figure 15) in the sequence listing, and a light chain variable region consisting of the amino acid sequence at amino acid positions 21 to 134 in SEQ ID NO: 16 (Figure 19) in the sequence listing;
(iii) a heavy chain variable region consisting of the amino acid sequence at amino acid positions 20 to 137 in SEQ ID NO: 14 (Figure 17) in the sequence listing, and a light chain variable region consisting of the amino acid sequence at amino acid positions 21 to 134 in SEQ ID NO: 16 (Figure 19) in the sequence listing;
(iv) a heavy chain variable region consisting of the amino acid sequence at amino acid positions 20 to 137 in SEQ ID NO: 14 (Figure 17) in the sequence listing, and a light chain variable region consisting of the amino acid sequence at amino acid positions 21 to 134 in SEQ ID NO: 15 (Figure 18) in the sequence listing;
(v) a heavy chain variable region consisting of the amino acid sequence at amino acid positions 20 to 137 in SEQ ID NO: 11 (Figure 14) in the sequence listing, and a light chain variable region consisting of the amino acid sequence at amino acid positions 21 to 134 in SEQ ID NO: 15 (Figure 18) in the sequence listing; and
(vi) a heavy chain variable region consisting of the amino acid sequence at amino acid positions 20 to 137 in SEQ ID NO: 13 (Figure 16) in the sequence listing, and a light chain variable region consisting of the amino acid sequence at amino acid positions 21 to 134 in SEQ ID NO: 15 (Figure 18) in the sequence listing.

**4.** The combination or pharmaceutical composition according to any one of claims 1 to 3, wherein the antibody comprises any one of the following combinations (i) to (vi) of a heavy chain and a light chain:

(i) a heavy chain consisting of the amino acid sequence at amino acid positions 20 to 463 in SEQ ID NO: 12 (Figure 15) in the sequence listing, and a light chain consisting of the amino acid sequence at amino acid positions 21 to 239 in SEQ ID NO: 15 (Figure 18) in the sequence listing (H-1140);
(ii) a heavy chain consisting of the amino acid sequence at amino acid positions 20 to 463 in SEQ ID NO: 12 (Figure 15) in the sequence listing, and a light chain consisting of the amino acid sequence at amino acid positions 21 to 239 in SEQ ID NO: 16 (Figure 19) in the sequence listing (H-1143);
(iii) a heavy chain consisting of the amino acid sequence at amino acid positions 20 to 463 in SEQ ID NO: 14 (Figure 17) in the sequence listing, and a light chain consisting of the amino acid sequence at amino acid positions 21 to 239 in SEQ ID NO: 16 (Figure 19) in the sequence listing (H-2143);
(iv) a heavy chain consisting of the amino acid sequence at amino acid positions 20 to 463 in SEQ ID NO: 14 (Figure 17) in the sequence listing, and a light chain consisting of the amino acid sequence at amino acid positions 21 to 239 in SEQ ID NO: 15 (Figure 18) in the sequence listing (H-2140);
(v) a heavy chain consisting of the amino acid sequence at amino acid positions 20 to 463 in SEQ ID NO: 11 (Figure 14) in the sequence listing, and a light chain consisting of the amino acid sequence at amino acid positions 21 to 239 in SEQ ID NO: 15 (Figure 18) in the sequence listing (H-1040); and
(vi) a heavy chain consisting of the amino acid sequence at amino acid positions 20 to 463 in SEQ ID NO: 13 (Figure 16) in the sequence listing, and a light chain consisting of the amino acid sequence at amino acid positions 21 to 239 in SEQ ID NO: 15 (Figure 18) in the sequence listing (H-2040).

**5.** The combination or pharmaceutical composition according to claim 1, comprising a heavy chain and a light chain having an identity of 95% or more to the amino acid sequences of a heavy chain and light chain, respectively, of the antibody according to any one described in claim 4, and binds to human ROBO4.

**6.** The combination or pharmaceutical composition according to claim 1, wherein the antibody or antigen-binding fragment thereof binds to a site on an antigen recognized by the antibody according to any one of claims 2 to 4.

**7.** The combination or pharmaceutical composition according to claim 1, wherein the antibody or antigen-binding fragment thereof competes with the antibody according to any one of claims 2 to 4 for binding to hROBO4.

**8.** A combination of an antibody or antigen-binding fragment thereof obtained by a method comprising steps (i) and (ii) below and a VEGF antagonist; or a pharmaceutical composition comprising the antibody or antigen-binding fragment thereof and a VEGF antagonist:

(i) a step of culturing cells according to (A) and (B) below; and

(A) recombinant cells into which a recombinant vector comprising an insert of nucleotide according to any one of (a) to (c) below or the nucleotide has been introduced; or
and the nucleotide is any one of the following nucleotides (a) to (c):

(a) a nucleotide comprising a nucleotide sequence encoding a partial and/or whole amino acid sequence of a heavy chain or light chain of the antibody according to any one of claims 1 to 7;
(b) a nucleotide consisting of a nucleotide sequence comprising a nucleotide sequence encoding a partial and/or whole amino acid sequence of a heavy chain or light chain of the antibody according to any one of claims 1 to 7; and
(c) a nucleotide consisting of a nucleotide sequence encoding a partial and/or whole amino acid sequence of a heavy chain or light chain of the antibody according to any one of claims 1 to 7;

(B) cells producing the antibody or antigen-binding fragment thereof according to any one of claims 1 to 7,

(ii) a step of collecting the antibody or antigen-binding fragment thereof according to any one of claims 1 to 7 from a culture obtained by the step (i).

**9.** The combination or pharmaceutical composition according to any one of claims 1 to 8, wherein one to several amino acids are deleted at the carboxyl terminus of a heavy chain or light chain of the antibody.

**10.** A combination of a modified form of the antibody or antigen-binding fragment thereof comprised in the combination or composition according to any one of claims 1 to 9 and an additional agent; or a pharmaceutical composition comprising the modified form and an additional agent.

**11.** The combination or pharmaceutical composition according to any one of claims 1 to 10, which is for use in treating or preventing an angiogenic disease.

**12.** The combination or pharmaceutical composition according to claim 11, wherein the disease is an ocular angiogenic disease.

**13.** The combination or pharmaceutical composition according to claim 12, wherein the ocular angiogenic disease is exudative age-related macular degeneration, diabetic retinopathy, macular edema, intraocular neovascular disease, central retinal vein occlusion, retrolental fibroplasia, proliferative retinopathy, neovascular glaucoma, or immune rejection of transplanted corneal tissue.

**14.** A pharmaceutical composition for use in combination with a VEGF antagonist, the pharmaceutical composition comprising the antibody or antigen-binding fragment thereof comprised in the combination or composition according to any one of claims 1 to 9 or the modified form comprised in the combination or composition according to claim 10.

**15.** A pharmaceutical composition comprising a VEGF antagonist, wherein when used in combination with the antibody or antigen-binding fragment thereof comprised in the combination or composition according to any one of claims 1 to 9 or the modification comprised in the combination or composition according to claim 10, the pharmaceutical composition increases effect of the antibody or antigen-binding fragment thereof or the modified form.

**16.** A pharmaceutical composition comprising the antibody or antigen-binding fragment thereof comprised in the combination or composition according to any one of claims 1 to 9 or the modified form comprised in the combination or composition according to claim 10, wherein when used in combination with a VEGF antagonist, the pharmaceutical composition increases effect of the VEGF antagonist.

**17.** A pharmaceutical composition, wherein the antibody or antigen-binding fragment thereof comprised in the combination or composition according to any one of claims 1 to 9 or the modified form comprised in the combination or composition according to claim 10 are administered in combination with a VEGF antagonist.

**18.** The pharmaceutical composition according to claim 17, wherein the antibody or antigen-binding fragment thereof comprised in the combination or composition according to any one of claims 1 to 9 or the modified form comprised in the combination or composition according to claim 10 and the VEGF antagonist are separately contained in different formulations each as an active ingredient, and administered simultaneously or at different times.

**19.** The pharmaceutical composition according to claim 17, wherein the antibody or antigen-binding fragment thereof comprised in the combination or composition according to any one of claims 1 to 9 or the modified form comprised in the combination or composition according to claim 10 and the VEGF antagonist are contained in a single formulation as active ingredients.

**20.** The pharmaceutical composition according to any one of claims 1 to 19, wherein the VEGF antagonist is one or two or more selected from the group consisting of an antibody or antigen-binding fragment, a fusion protein, an aptamer, and a low-molecular-weight compound.

**21.** The pharmaceutical composition according to any one of claims 1 to 20, wherein the VEGF antagonist is one or two or more selected from the group consisting of aflibercept, ranibizumab, bevacizumab, brolucizumab, LMG324, RG7716, pegaptanib sodium, abicipar pegol, DE-120, and OPT-302.

**22.** The pharmaceutical composition according to claim 21, wherein the VEGF antagonist is aflibercept.

**23.** The pharmaceutical composition according to claim 21, wherein the VEGF antagonist is ranibizumab.

**24.** The pharmaceutical composition according to claim 21, wherein the VEGF antagonist is bevacizumab.

**25.** A method for treating an ocular angiogenic disease, wherein the antibody or antigen-binding fragment thereof comprised in the combination or composition according to any one of claims 1 to 9 or the modified form comprised in the combination or composition according to claim 10 are administered in combination wifh a VEGF antagonist.

**26.** The method according to claim 22, wherein the ocular angiogenic disease is exudative age-related macular degeneration, diabetic retinopathy, macular edema, intraocular neovascular disease, central retinal vein occlusion, retrolental fibroplasia, proliferative retinopathy, neovascular glaucoma, or immune rejection of transplanted corneal tissue.

**27.** The method according to claim 22 or 23, wherein the VEGF antagonist is one or two or more selected from the group consisting of an antibody or antigen-binding fragment, a fusion protein, an aptamer, and a low-molecular-weight compound.

**28.** The method according to any one of claims 22 to 24, wherein the VEGF antagonist is one or two or more selected from the group consisting of aflibercept, ranibizumab, bevacizumab, brolucizumab, LMG324, RG7716, pegaptanib sodium, abicipar pegol, DE-120, and OPT-302.

**29.** The method according to claim 28, wherein the VEGF antagonist is aflibercept.

**30.** The method according to claim 28, wherein the VEGF antagonist is ranibizumab.

**31.** The method according to claim 28, wherein the VEGF antagonist is bevacizumab.

Figure 1

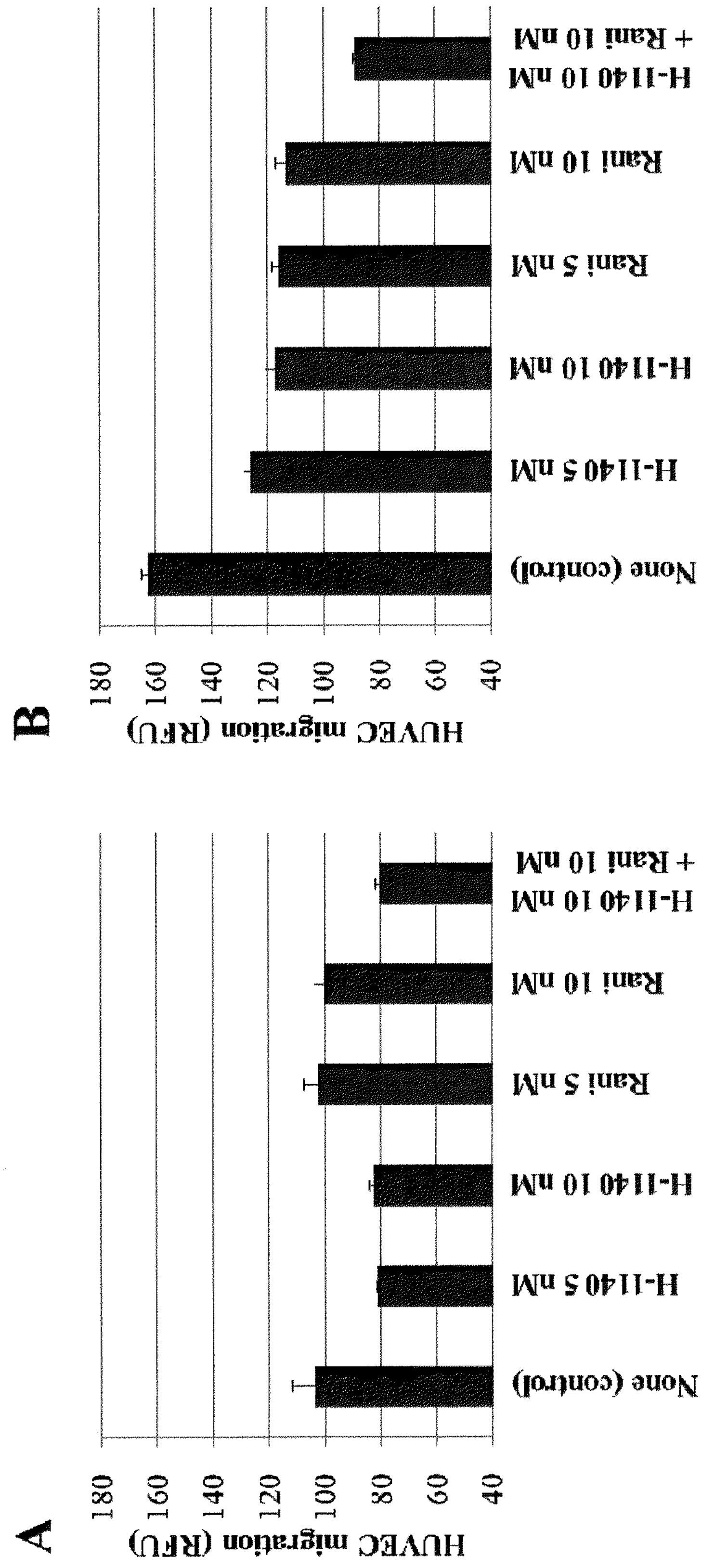
A
B
HUVEC migration (RFU)
180
160
140
120
100
80
60
40
None (control)
H-1140 5 nM
H-1140 10 nM
Rani 5 nM
Rani 10 nM
H-1140 10 nM
+ Rani 10 nM

Figure 2

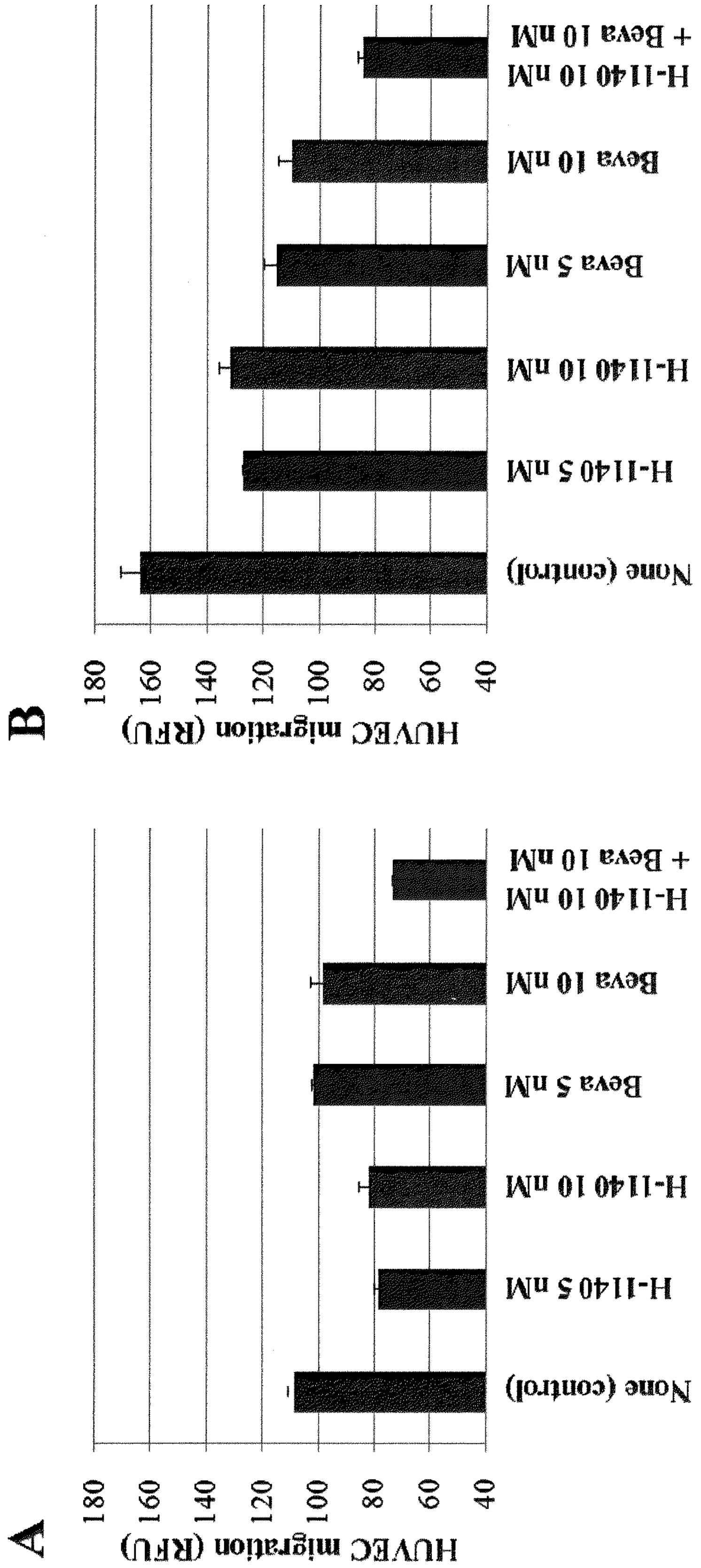
A
B
HUVEC migration (RFU)
180
160
140
120
100
80
60
40
None (control)
H-1140 5 nM
H-1140 10 nM
Beva 5 nM
Beva 10 nM
H-1140 10 nM + Beva 10 nM

Figure 3

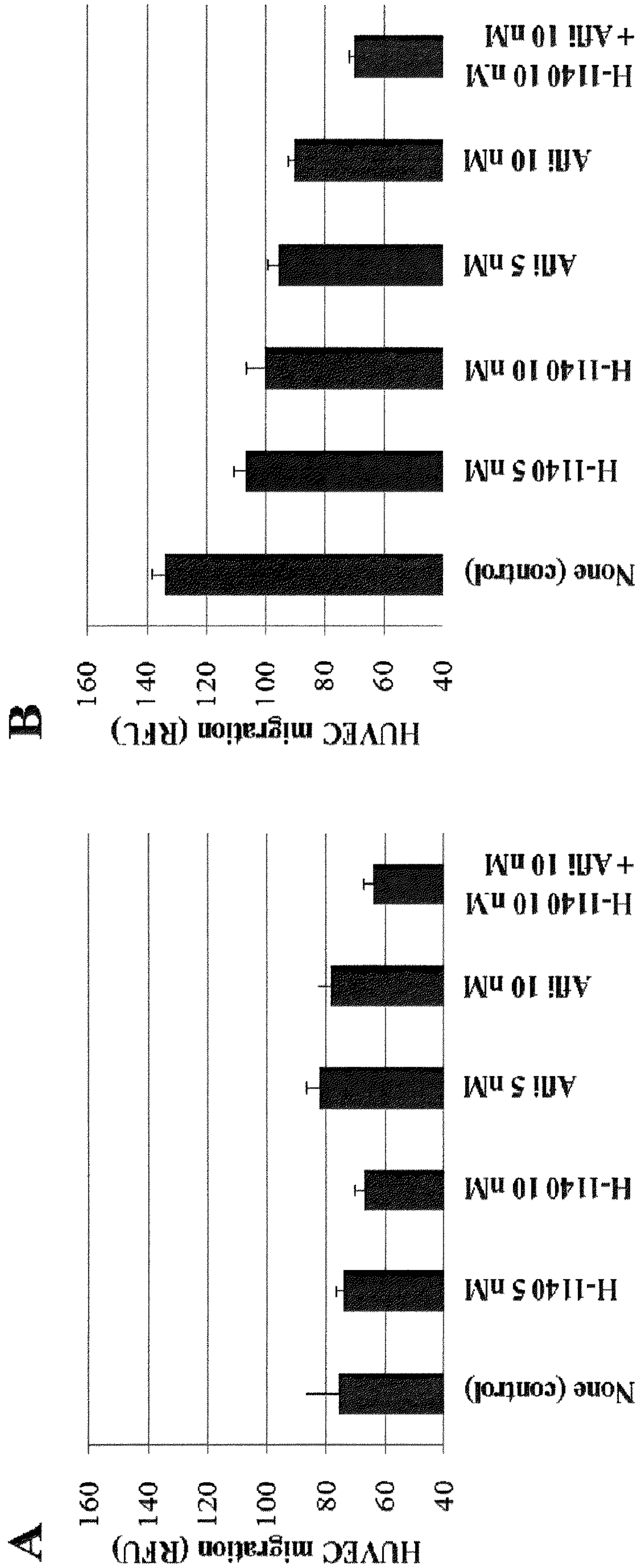
A
B
HUVEC migration (RFU)
40
60
80
100
120
140
160
None (control)
H-1140 5 nM
H-1140 10 nM
Afli 5 nM
Afli 10 nM
H-1140 10 nM
+Afli 10 nM

Figure 4

Human ROBO4 full-length cDNA (SEQ ID NO: 1)

atgggctctggaggagacagcctcctgggggcaggggttccctgcctctgctgctc
ctgctcatcatgggaggcatggctcaggactccccgccccagatcctagtccacccc
caggaccagctgttccagggccctggccctgccaggatgagctgccaagcctcaggc
cagccacctcccaccatccgctggttgctgaatgggcagcccctgagcatggtgccc
ccagacccacaccacctcctgcctgatgggacccttctgctgctacagccccctgcc
cggggacatgcccacgatggccaggccctgtccacagacctgggtgtctacacatgt
gaggccagcaaccggcttggcacggcagtcagcagaggcgctcggctgtctgtggct
gtcctccgggaggatttccagatccagcctcgggacatggtggctgtggtgggtgag
cagtttactctggaatgtgggccgccctggggccacccagagcccacagtctcatgg
tggaaagatgggaaacccctggccctccagcccggaaggcacacagtgtccgggggg
tccctgctgatggcaagagcagagaagagtgacgaagggacctacatgtgtgtggcc
accaacagcgcaggacatagggagagccgcgcagcccgggtttccatccaggagccc
caggactacacggagcctgtggagcttctggctgtgcgaattcagctggaaaatgtg
acactgctgaacccggatcctgcagagggccccaagcctagaccggcggtgtggctc
agctggaaggtcagtggccctgctgcgcctgcccaatcttacacggccttgttcagg
acccagactgccccgggaggccagggagctccgtgggcagaggagctgctggccggc
tggcagagcgcagagcttggaggcctccactggggccaagactacgagttcaaagtg
agaccatcctctggccgggctcgaggccctgacagcaacgtgctgctcctgaggctg
ccggaaaaagtgcccagtgccccacctcaggaagtgactctaaagcctggcaatggc
actgtctttgtgagctgggtcccaccacctgctgaaaaccacaatggcatcatccgt
ggctaccaggtctggagcctgggcaacacatcactgccaccagccaactggactgta
gttggtgagcagacccagctggaaatcgccacccatatgccaggctcctactgcgtg
caagtggctgcagtcactggtgctggagctggggagcccagtagacctgtctgcctc
cttttagagcaggccatggagcgagccacccaagaacccagtgagcatggtccctgg
accctggagcagctgagggctaccttgaagcggcctgaggtcattgccacctgcggt
gttgcactctggctgctgcttctgggcaccgccgtgtgtatccaccgccggcgccga
gctagggtgcacctgggcccaggtctgtacagatataccagtgaggatgccatccta
aaacacaggatggatcacagtgactcccagtggttggcagacacttggcgttccacc
tctggctctcgggacctgagcagcagcagcagcctcagcagtcggctgggggcggat
gcccgggacccactagactgtcgtcgctccttgctctcctgggactcccgaagcccc
ggcgtgcccctgcttccagacaccagcacttttatggctccctcatcgctgagctg
ccctccagtaccccagccaggccaagtccccaggtcccagctgtcaggcgcctccca
ccccagctggcccagctctccagcccctgttccagctcagacagcctctgcagccgc

Figure 4 (cont.)

aggggactctcttctccccgcttgtctctggcccctgcagaggcttggaaggccaaa
aagaagcaggagctgcagcatgccaacagttccccactgctccggggcagccactcc
ttggagctccgggcctgtgagttaggaaatagaggttccaagaacctttcccaaagc
ccaggagctgtgccccaagctctggttgcctggcgggccctgggaccgaaactcctc
agctcctcaaatgagctggttactcgtcatctccctccagcacccctctttcctcat
gaaactcccccaactcagagtcaacagacccagcctccggtggcaccacaggctccc
tcctccatcctgctgccagcagcccccatccccatccttagcccctgcagtcccccct
agcccccaggcctcttccctctctggccccagcccagcttccagtcgcctgtccagc
tcctcactgtcatccctgggggaggatcaagacagcgtgctgaccccctgaggaggta
gccctgtgcttggaactcagtgagggtgaggagactcccaggaacagcgtctctccc
atgccaagggctccttcaccccccaccacctatgggtacatcagcgtcccaacagcc
tcagagttcacggacatgggcaggactggaggaggggtggggcccaagggggggagtc
ttgctgtgcccacctcggccctgcctcacccccacccccagcgagggctccttagcc
aatggttggggctcagcctctgaggacaatgccgccagcgccagagccagccttgtc
agctcctccgatggctccttcctcgctgatgctcactttgcccgggccctggcagtg
gctgtggatagctttggtttcggtctagagcccagggaggcagactgcgtcttcata
gatgcctcatcacctccctccccacgggatgagatcttcctgacccccaacctctcc
ctgcccctgtgggagtggaggccagactggttggaagacatggaggtcagccacacc
cagcggctgggaaggggggatgcctccctggccccctgactctcagatctcttcccag
agaagtcagctccactgtcgtatgcccaaggctggtgcttctcctgtagattactcc
tga
stop codon

Figure 5

Full-length amino acid sequence of human ROBO4 (SEQ ID NO: 2)

MGSGGDSLLGGRGSLPLLLLLIMGGMAQDSPPQILVHPQDQLFQGPGPARMSCQASG
QPPPTIRWLLNGQPLSMVPPDPHHLLPDGTLLLLQPPARGHAHDGQALSTDLGVYTC
EASNRLGTAVSRGARLSVAVLREDFQIQPRDMVAVVGEQFTLECGPPWGHPEPTVSW
WKDGKPLALQPGRHTVSGGSLLMARAEKSDEGTYMCVATNSAGHRESRAARVSIQEP
QDYTEPVELLAVRIQLENVTLLNPDPAEGPKPRPAVWLSWKVSGPAAPAQSYTALFR
TQTAPGGQGAPWAEELLAGWQSAELGGLHWGQDYEFKVRPSSGRARGPDSNVLLLRL
PEKVPSAPPQEVTLKPGNGTVFVSWVPPPAENHNGIIRGYQVWSLGNTSLPPANWTV
VGEQTQLEIATHMPGSYCVQVAAVTGAGAGEPSRPVCLLLEQAMERATQEPSEHGPW
TLEQLRATLKRPEVIATCGVALWLLLLGTAVCIHRRRRARVHLGPGLYRYTSEDAIL
KHRMDHSDSQWLADTWRSTSGSRDLSSSSSLSSRLGADARDPLDCRRSLLSWDSRSP
GVPLLPDTSTFYGSLIAELPSSTPARPSPQVPAVRRLPPQLAQLSSPCSSSDSLCSR
RGLSSPRLSLAPAEAWKAKKKQELQHANSSPLLRGSHSLELRACELGNRGSKNLSQS
PGAVPQALVAWRALGPKLLSSSNELVTRHLPPAPLFPHETPPTQSQQTQPPVAPQAP
SSILLPAAPIPILSPCSPPSPQASSLSGPSPASSRLSSSSLSSLGEDQDSVLTPEEV
ALCLELSEGEETPRNSVSPMPRAPSPPTTYGYISVPTASEFTDMGRTGGGVGPKGGV
LLCPPRPCLTPTPSEGSLANGWGSASEDNAASARASLVSSSDGSFLADAHFARALAV
AVDSFGFGLEPREADCVFIDASSPPSPRDEIFLTPNLSLPLWEWRPDWLEDMEVSHT
QRLGRGMPPWPPDSQISSQRSQLHCRMPKAGASPVDYS

Figure 6

Amino acid sequence of CDRH1 of hMAb1-H1 to -H4 type heavy chains (SEQ ID NO: 3)

TYAMS

Figure 7

Amino acid sequence of CDRH2 of hMAb1-H1 and -H3 type heavy chains (SEQ ID NO: 4)

TISNGGTYTYYPDSVKG

Figure 8

Amino acid sequence of CDRH2 of hMAb1-H2 and -H4 type heavy chains (SEQ ID NO: 5)

TISQGGTYTYYPDSVKG

Figure 9

Amino acid sequence of CDRH3 of hMAb1-H1 to -H4 type heavy chains (SEQ ID NO: 6)

LIYYDYLDY

Figure 10

Amino acid sequence of CDRL1 of hMAb1-L1 type light chain (SEQ ID NO: 7)

RSSQSLENSNGNTYLN

Figure 11

Amino acid sequence of CDRL1 of hMAb1-L2 type light chain (SEQ ID NO: 8)

RSSQSLENENKNLYLN

Figure 12

Amino acid sequence of CDRL2 of hMAb1-L1 and -L2 type light chains (SEQ ID NO: 9)

RVSNRFS

Figure 13

Amino acid sequence of CDRL3 of hMAb1-L1 and -L2 type light chains (SEQ ID NO: 10)

LQVTHVPWT

Figure 14

Amino acid sequence of hMAb1-H1 type heavy chain (SEQ ID NO: 11)

MKHLWFFLLLVAAPRWVLSEVQLVESGGGLVQPGGSLRLSCAASGFTFSTYAMSWVR
QAPGKGLEWVATISNGGTYTYYPDSVKGRFTISRDNAKNTLYLQMNSLRAEDTAVYY
CARLIYYDYLDYWGQGTLVTVSSASTKGPSVFPLAPCSRSTSESTAALGCLVKDYFP
EPVTVSWNSGALTSGVHTFPAVLQSSGLYSLSSVVTVPSSNFGTQTYTCNVDHKPSN
TKVDKTVERKCCVECPPCPAPPVAGPSVFLFPPKPKDTLMISRTPEVTCVVVDVSHE
DPEVQFNWYVDGVEVHNAKTKPREEQFNSTFRVVSVLTVVHQDWLNGKEYKCKVSNK
GLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKS
LSLSPGK

signal sequence (1-19), variable region (20-137), constant region (138-463)

Figure 15

Amino acid sequence of hMAb1-H2 type heavy chain (SEQ ID NO: 12)

MKHLWFFLLLVAAPRWVLSEVQLVESGGGLVQPGGSLRLSCAASGFTFSTYAMSWVR
QAPGKGLEWVATISQGGTYTYYPDSVKGRFTISRDNAKNTLYLQMNSLRAEDTAVYY
CARLIYYDYLDYWGQGTLVTVSSASTKGPSVFPLAPCSRSTSESTAALGCLVKDYFP
EPVTVSWNSGALTSGVHTFPAVLQSSGLYSLSSVVTVPSSNFGTQTYTCNVDHKPSN
TKVDKTVERKCCVECPPCPAPPVAGPSVFLFPPKPKDTLMISRTPEVTCVVVDVSHE
DPEVQFNWYVDGVEVHNAKTKPREEQFNSTFRVVSVLTVVHQDWLNGKEYKCKVSNK
GLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKS
LSLSPGK

signal sequence (1-19), variable region (20-137), constant region (138-463)

Figure 16

Amino acid sequence of hMAb1-H3 type heavy chain (SEQ ID NO: 13)

MKHLWFFLLLVAAPRWVLSEVQLVESGGGLVQPGGSLRLSCAASGFTFSTYAMSWVR
QAPGKRLEWVATISNGGTYTYYPDSVKGRFTISRDNAKNTLYLQMNSLRAEDTAVYY
CARLIYYDYLDYWGQGTLVTVSSASTKGPSVFPLAPCSRSTSESTAALGCLVKDYFP
EPVTVSWNSGALTSGVHTFPAVLQSSGLYSLSSVVTVPSSNFGTQTYTCNVDHKPSN
TKVDKTVERKCCVECPPCPAPPVAGPSVFLFPPKPKDTLMISRTPEVTCVVVDVSHE
DPEVQFNWYVDGVEVHNAKTKPREEQFNSTFRVVSVLTVVHQDWLNGKEYKCKVSNK
GLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKS
LSLSPGK

signal sequence (1-19), variable region (20-137), constant region (138-463)

Figure 17

Amino acid sequence of hMAb1-H4 type heavy chain (SEQ ID NO: 14)

MKHLWFFLLLVAAPRWVLSEVQLVESGGGLVQPGGSLRLSCAASGFTFSTYAMSWVR
QAPGKRLEWVATISQGGTYTYYPDSVKGRFTISRDNAKNTLYLQMNSLRAEDTAVYY
CARLIYYDYLDYWGQGTLVTVSSASTKGPSVFPLAPCSRSTSESTAALGCLVKDYFP
EPVTVSWNSGALTSGVHTFPAVLQSSGLYSLSSVVTVPSSNFGTQTYTCNVDHKPSN
TKVDKTVERKCCVECPPCPAPPVAGPSVFLFPPKPKDTLMISRTPEVTCVVVDVSHE
DPEVQFNWYVDGVEVHNAKTKPREEQFNSTFRVVSVLTVVHQDWLNGKEYKCKVSNK
GLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKS
LSLSPGK

signal sequence (1-19), variable region (20-137), constant region (138-463)

Figure 18

Amino acid sequence of hMAb1-L1 type light chain (SEQ ID NO: 15)

MVLQTQVFISLLLWISGAYGDIVMTQSPLSLPVTLGEPASISCRSSQSLENSNGNTY
LNWYLQKPGQSPQLLIYRVSNRFSGVPDRFSGSGSGTDFTLKISRVEAEDVGVYYCL
QVTHVPWTFGPGTKVDIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSS
PVTKSFNRGEC

signal sequence (1-20), variable region (21-134), constant region (135-239)

Figure 19

Amino acid sequence of hMAb1-L2 type light chain (SEQ ID NO: 16)

MVLQTQVFISLLLWISGAYGDIVMTQSPLSLPVTLGEPASISCRSSQSLENENKNLY
LNWYLQKPGQSPQLLIYRVSNRFSGVPDRFSGSGSGTDFTLKISRVEAEDVGVYYCL
QVTHVPWTFGPGTKVDIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSS
PVTKSFNRGEC

signal sequence (1-20), variable region (21-134), constant region (135-239)

## INTERNATIONAL SEARCH REPORT

International application No.
PCT/JP2017/036721

A. CLASSIFICATION OF SUBJECT MATTER
Int.Cl. A61K39/395(2006.01)i, A61K31/7088(2006.01)i, A61K38/02(2006.01)i, A61K45/00(2006.01)i, A61P27/02(2006.01)i, A61P27/06(2006.01)i, A61P37/06(2006.01)i, A61P43/00(2006.01)i, C07K16/18(2006.01)n, C12N15/09(2006.01)n

According to International Patent Classification (IPC) or to both national classification and IPC

B. FIELDS SEARCHED

Minimum documentation searched (classification system followed by classification symbols)
Int.Cl. A61K39/395, A61K31/7088, A61K38/02, A61K45/00, A61P27/02, A61P27/06, A61P37/06, A61P43/00, C07K16/18, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model specifications of Japan | 1994-2017 |

Electronic data base consulted during the international search (name of data base and, where practicable, search terms used)
CAplus/MEDLINE/EMBASE/BIOSIS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2015-515490 A (DAIICHI SANKYO CO., LTD.) 28 May 2015, entire text, paragraph [0185] & US 2015/0098945 A1, entire text, paragraph [0272] & WO 2013/160879 A1 & EP 2841457 A1 & KR 10-2015-0008074 A & CN 104768973 A | 1-31 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 November 2017 (28.11.2017) | 12 December 2017 (12.12.2017) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/036721

C (Continuation). DOCUMENTS CONSIDERED TO BE RELEVANT

| Category* | Citation of document, with indication, where appropriate, of the relevant passages | Relevant to claim No. |
|---|---|---|
| Y | 古泉 英貴, 8. 加齢黄斑変性の治療 2)抗 VEGF 療法, Progress in Medicine, 2013, vol. 33, no. 10, pp. 2107-2111 page 2107, left column, fig. 4, page 2109, right column to page 2111, left column, non-official translation (KOIZUMI, Hideki, 8. Treatment of Age-related Macular Degeneration 2) Anti-VEGF Theraphy) | 1-31 |
| Y | WYGLEDOWSKA-PROMIENSKA, Dorota, et al., Combination of Aflibercept and Bromfenac Therapy in Age-Related Macular Degeneration: A Pilot Study Aflibercept and Bromfenac in AMD, Medical Science Monitor, 2015, vol. 21, pp. 3906-3912 entire text | 1-31 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9845331 A
- WO 00075319 A
- WO 2008073441 A
- WO 2013160879 A
- WO 9007861 A
- US 6972323 B
- US 4946778 A
- US 5260203 A
- US 5091513 A
- US 5455030 A
- US 6214388 B


### Non-patent literature cited in the description


- *Cancer Res,* 1997, vol. 57 (20), 4593-4599
- *Genomics,* 2002, vol. 79 (4), 547-552
- *Nature Medicine,* 2008, vol. 14, 448-453
- *Molecular Vision,* 2009, vol. 15, 1057-1069
- *Cancer Cell,* 2011, vol. 19, 101-113
- *Nature,* 2008, vol. 8, 34-47
- *Cancer Res,* 2011, vol. 71, 5670-5677
- *Nature,* 1986, vol. 321, 522-525
- **GERGER et al.** *The Journal of Biological Chemistry,* 1987, vol. 262 (2), 785-794
- *Journal of Chromatography A,* 1995, vol. 705, 129-134
- *Analytical Biochemistry,* 2007, vol. 360, 75-83
- **KOHLER ; MILSTEIN.** *Nature,* 1975, vol. 256, 495-497
- Monoclonal Antibodies. Plenum Press, 1980, 365-367